# EUROPEAN PATENT SPECIFICATION

(11) **EP 4 288 422 B1**
(45) Date of publication and mention of the grant of the patent: **29.10.2025**
(21) Application number: 22706022.5
(22) Date of filing: 08.02.2022
(51) Int. Cl.: C07D 311/30, C07D 405/12, C07D 413/12, C07D 493/14, A61K 31/352, A61K 31/405, A61K 31/4433, A61K 45/06, A61P 35/00, A61P 35/02, A61K 31/4025

(54) **HETEROAROMATIC SPERMIDINE ANALOGUES AND THEIR ANTICANCER ACTIVITY**
HETEROAROMATISCHE SPERMIDINANALOGA UND IHRE ANTIKREBSWIRKUNG
ANALOGUES DE LA SPERMIDINE HÉTÉROAROMATIQUES ET LEUR ACTIVITÉ ANTICANCÉREUSE

(30) Priority: 08.02.2021 GB 202101728
(43) Date of publication of application: 13.12.2023
(73) Proprietor: Floratek Pharma SA, 1170 Aubonne (CH)
(72) Inventor: STOICESCU, Dan Florin, 1170 Aubonne (CH)
(74) Representative: Russell, Tim
(86) International application number: PCT/EP2022/053045
(87) International publication number: WO 2022/167698

(56) References cited:
- WO-A1-2014/018741
- WO-A1-2015/112898
- WO-A1-2020/154434
- WO-A1-2020/228513
- WO-A2-02/074036
- WO-A2-2010/011836
- WO-A2-2011/156479
- WO-A2-2018/022668
- US-A1- 2020 390 755
- WANG JUBO ET AL: "Design of wogonin-inspired selective cyclin-dependent kinase 9 (CDK9) inhibitors with potent in vitro and in vivo antitumor activity", EUROPEAN JOURNAL OF MEDICINAL CHEMISTRY, ELSEVIER, AMSTERDAM, NL, vol. 178, 15 June 2019 (2019-06-15), pages 782 - 801, XP085751639, ISSN: 0223-5234, [retrieved on 20190615], DOI: 10.1016/J.EJMECH.2019.06.024
- MANIVANNAN ELANGOVAN ET AL: "QSAR Analysis and Pharmacophore Mapping of Catecholic Flavonoids for Telomerase Inhibitory Activity", ACTA FARMACEUTICA BONAERENSE; LATIN AMERICAN JOURNAL OF PHARMACY, COLEGIO DE FARMACEUTICOS DE LA PROVINCIA DE BUENOS AIRES, AR, vol. 32, no. 6, 1 January 2013 (2013-01-01), pages 802 - 808, XP009535258, ISSN: 0326-2383
- DAS SREEPARNA ET AL: "Design, synthesis and exploring the quantitative structure-activity relationship of some antioxidant flavonoid analo", BIOORGANIC & MEDICINAL CHEMISTRY LETTERS, ELSEVIER, AMSTERDAM, NL, vol. 24, no. 21, 17 September 2014 (2014-09-17), pages 5050 - 5054, XP029077486, ISSN: 0960-894X, DOI: 10.1016/J.BMCL.2014.09.028
- LIU XIA ET AL: "O-Methylated Metabolite of 7,8-Dihydroxyflavone Activates TrkB Receptor and Displays Antidepressant Activity", PHARMACOLOGY, vol. 91, no. 3-4, 1 January 2013 (2013-01-01), CH, pages 185 - 200, XP055916173, ISSN: 0031-7012, Retrieved from the Internet <URL:https://www.karger.com/Article/Pdf/346920> DOI: 10.1159/000346920

## Description

### FIELD OF THE INVENTION

The invention relates to compounds, pharmaceutical compositions comprising the same, and the same for use in medicine. In particular, the compounds are useful for the treatment or prevention of cancer.

### BACKGROUND

Dual-targeting or multi-targeting of malignant pathways by a single drug molecule represents an efficient, logical and alternative approach to drug combinations. A new generation of dual or multi-targeting drugs is emerging, where a single chemical entity can act on multiple molecular targets [Raghavendra NM, et al. Dual or multi-targeting inhibitors: The next generation anticancer agents. Eur J Med Chem. 2018 Jan 1;143:1277-1300]. The present invention uses a rational, bioinformatics and poly-pharmacological approach to design multi-target anticancer agents by combining flavonoid-like structures with a variety of polyamine chains from the spermidine class. Both broad molecule structures have been shown to target multiple cellular pathways leading to cancer inhibition [Kikuchi H, Yuan B, Hu X, Okazaki M. Chemopreventive and anticancer activity of flavonoids and its possibility for clinical use by combining with conventional chemotherapeutic agents. Am J Cancer Res. 2019;9(8):1517-1535; Carl W. Porter, Raymond J. Bergeron and Neal J. Stolowich. Biological Properties of N4-Spermidine Derivatives and Their Potential in Anticancer Chemotherapy. Cancer Res. 1982 (42) (10) 4072-4078].

The role of flavonoids as potential cancer therapies includes the inhibition of activation of pro-carcinogens, inhibition of proliferation of cancer cells, selective death of cancer cells by apoptosis, inhibition of metastasis and angiogenesis, activation of immune response against cancer cells, modulation of the inflammatory cascade and the modulation of drug resistance [Kikuchi H, Yuan B, Hu X, Okazaki M. Chemopreventive and anticancer activity of flavonoids and its possibility for clinical use by combining with conventional chemotherapeutic agents. Am J Cancer Res. 2019;9(8):1517-1535]. In spite of their promising potential in controlling the malignant process, natural flavonoids present major limitations to their clinical use due to low bioavailability and their perceived lack of specificity. Such versatile biological activity implies a great underlying complexity in the true mechanisms of action of different flavonoids, often dependent on a fine balance between pro- and anti-oxidant properties or between other beneficial and detrimental effects [Martinez Perez, et al. 2014. Novel flavonoids as anticancer agents: mechanisms of action and promise for their potential application in breast cancer. Biochemical Society Transactions, vol. 42, no. 4, pp. 1017 - 1023.]. As such, a systematic study of the structure activity relationship of the flavonoid structures is necessary and can be addressed by the rationale design of a series of molecules.

The present invention addresses some of the limitations of flavonoid derivatives by employing a novel approach of combining such moieties with a synthetic polyamine chain with multiple potential advantages: (i) multi-targeting cancer pathways (ii) inhibition of the natural polyamine pathway which is often dysregulated in cancer and (iii) facilitating transport through the cell membrane and targeting to specific intracellular structures.

It is well-known that polyamines interact with aspartate, glutamate, and aromatic residues of a given receptor and/or enzyme mainly through the formation of ion bonds, since at physiological pH, protonation of amino groups is nearly complete. From this, the hypothesis arises that a polyamine may be a universal template able to recognize different receptor systems. This hypothesis suggests that both affinity and selectivity may be fine-tuned by inserting appropriate substituents onto the amine functions and by varying the methylene chain lengths between them on the polyamine backbone [Minarini, A., Milelli, A., Tumiatti, V. et al. Synthetic polyamines: an overview of their multiple biological activities. Amino Acids 38, 383-392 (2010).]

Polyamine metabolism is often dysregulated in cancers. In addition, the polyamine pathway is a downstream target for many oncogenes [Shantz LM, Levin VA. Regulation of ornithine decarboxylase during oncogenic transformation: mechanisms and therapeutic potential. Amino Acids. 2007;33(2):213-23]. Polyamine biosynthesis is activated in tumors, and these metabolites are important for developmental and compensatory growth in response to systemic stimuli like hormones (growth hormones, corticosteroids, androgens, and estrogens). As a result, various strategies targeting polyamine biosynthetic enzymes have been brought to the preclinical and clinical arena [Arruabarrena-Aristorena A, et al. Oil for the cancer engine: The crosstalk between oncogenic signaling and polyamine metabolism. Sci Adv. 2018;4(1):1-11]. The most successful and widely used inhibitor of polyamine biosynthesis is 2-difluoromethylornithine (DFMO). DFMO was specifically designed to be an enzyme-activated irreversible inhibitor of ODC [Metcalf BW, et al. Catalytic irreversible inhibition of mammalian ornithine decarboxylase (E.C4.1.1.17) by substrate and product analogues. J. Am. Chem. Soc. 1978;100:2551-2553.]

These encouraging results in selective cancers, both *in vitro* and *in vivo* led to clinical trials with DFMO as a single agent. Although DFMO was exceedingly well tolerated, there were no significant clinical responses observed in the early trials. More recently, a resurgence of interest in DFMO as a single agent has occurred in the treatment of neuroblastoma [Bassiri H, et al. Translational development of difluoromethylornithine (DFMO) for the treatment of neuroblastoma. Transl. Pediatr. 2015;4:226-238] and as a chemoprevention agent, alone or in various combinations [Gerner EW, Meyskens FL Jr. Polyamines and cancer: old molecules, new understanding. Nat Rev Cancer. 2004 Oct;4(10):781-92].

Another rational for linking polyamines to bioactive moieties is not only to use the polyamine transport system to enter the cell, but also to direct the agent to its intracellular molecular target, which can be mitochondria or other anionic structures [Murray-Stewart TR, et al. Targeting polyamine metabolism for cancer therapy and prevention. Biochem J. 2016;473(19):2937-2953].

Wang Jubo et al. "Design of wogonin-inspired selective cyclin- dependent kinase 9 (CDK9) inhibitors with potent in vitro and in vivo antitumor activity", WO 2020/228513 A1, and WO 2015/112898 A1 disclose compounds which exhibit oxygen derived substituent at the position of R₁, R₂ and R₄.

WO 2020/154434 A1, WO 2018/022668 A2, Manivannan Elangovan et al.:"QSAR Analysis and Pharmacophore Mapping of Catecholic Flavonoids for Telomerase Inhibitory Activity", WO 02/074036 A2, WO 2011/156479 A2, WO 2014/018741 A1, Das Sreeparna et al.: "Design, synthesis and exploring the quantitative structure-activity relationship of some antioxidant flavonoid analogues", Liu Xia et al.: "O-Methylated Metabolite of 7,8-Dihydroxyflavone Activates TrkB Receptor and Displays Antidepressant Activity", US 2020/390755 A1, and WO 2010/011836 A2 disclose compounds which exhibit oxygen derived substituents at the position of R₁ and R₂._

### SUMMARY OF THE INVENTION

The present invention is defined by the claims.

A first aspect of the invention provides a compound of formula (1), or a pharmaceutically acceptable salt, multi-salt or solvate thereof: 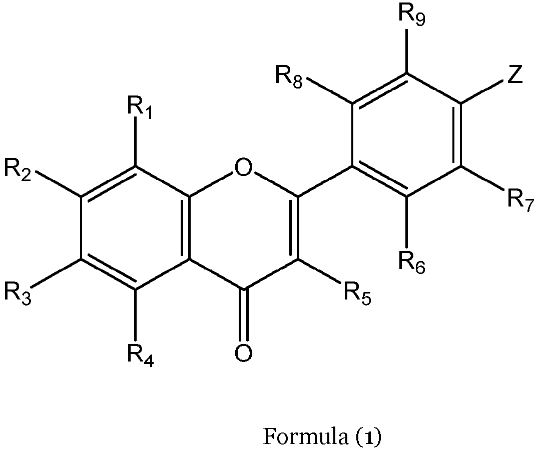 wherein:
Z is selected from:
   -NR¹¹R¹²;
   -N(R¹⁰)-(CH₂)ₚ-NR¹¹R¹²;
   -N(R¹⁰)-(CH₂)_{q}-N(R¹⁰)-(CH₂)_{q}-NR¹¹R¹²; and
   -N(R¹⁰)-(CH₂)ᵣ-N(R¹⁰)-(CH₂)ᵣ-N(R¹⁰)-(CH₂)ᵣ-NR¹¹R¹²;
R¹, R², and R⁵,independently, are selected from -OH, -O-C₁₋₄ alkyl, - OC(O)R₁₃, -OC(O)NHR₁₃, -OC(O)N(R¹³)₂; or R¹ and R² together form -O-CH₂-O-;
R³, R⁴, R⁶, R⁷, R⁸, and R⁹, independently, are selected from H; halo; -CN; -NO₂; and -NH₂;
each -R^{β} is independently selected from a C₁-C₆ alkyl, C₂-C₆ alkenyl, C₂-C₆ alkynyl , -O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, - CHO, -CON(CH₃)₂ or oxo (=O) groups;
each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β};
R¹¹ and R¹² are independently selected from H, C₁₋₆-alkyl, C₂-C₆ alkenyl, C₂₋₆ alkynyl, C₃₋₁₀ cycloalkyl, and benzyl, wherein each R¹¹ and R¹², when is not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl or benzyl;
each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴;
each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅;
each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl;
each p is independently an integer selected from 1 to 4;
each q is independently an integer selected from 1 to 4; and
each r is independently an integer selected from 1 to 4.

In one embodiment, R¹, R², and R⁵, independently, are selected from -OH, and -O-C₁₋₄ alkyl.

In one embodiment, R¹, R², and R⁵ are independently selected from -OH and -OCH₃.

In one embodiment, R³, R⁴, R⁶, R⁷, R⁸, and R⁹, are H.

In one embodiment, Z is -N(R¹⁰)-(CH₂)ₚ-NR¹¹R¹².

In one embodiment, p is selected from 3 and 4.

In one embodiment, R¹¹ and R¹² together form a 5- or 6-membered heterocycle optionally substituted with 1 or 2 C₁₋₄ alkyl or benzyl.

In one embodiment, the 5- or 6-membered heterocycle is morpholine, piperidine, piperazine, or pyrrolidine optionally substituted with 1 or 2 C₁₋₄ alkyl or benzyl.

In one embodiment, R¹¹ and R¹² are independently selected from H and C₁₋₆ alkyl (e.g., methyl or ethyl), C₃₋₁₀ cycloalkyl (e.g., adamantyl), and benzyl; wherein each R¹¹ and R¹², when not H, are independently optionally substituted with 1 or 2 -R^{β}.

In one embodiment, R¹⁰ is H or -CH₃.

In one embodiment, the compound may be a compound of Formula (1A): 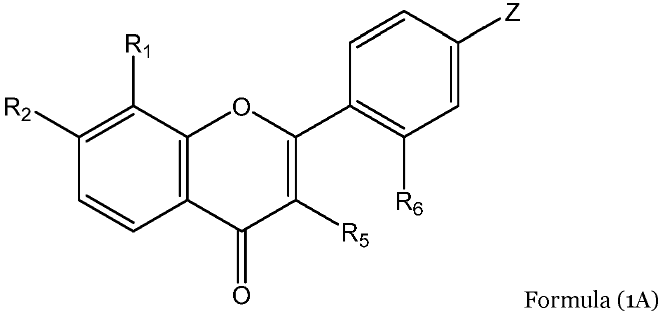 wherein R¹, R², R⁵, R⁶, and Z are as defined herein.

A second aspect of the invention provides a compound selected from:

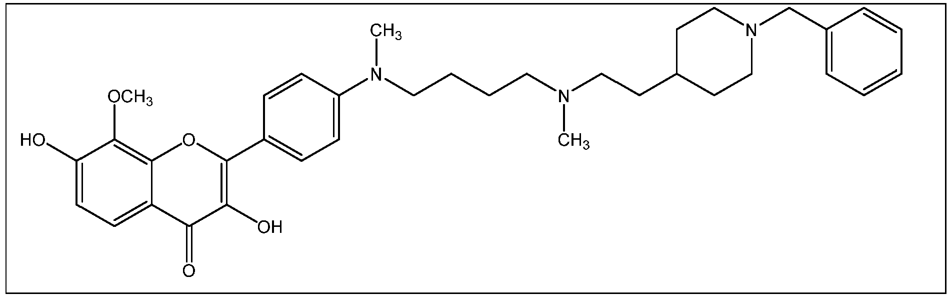

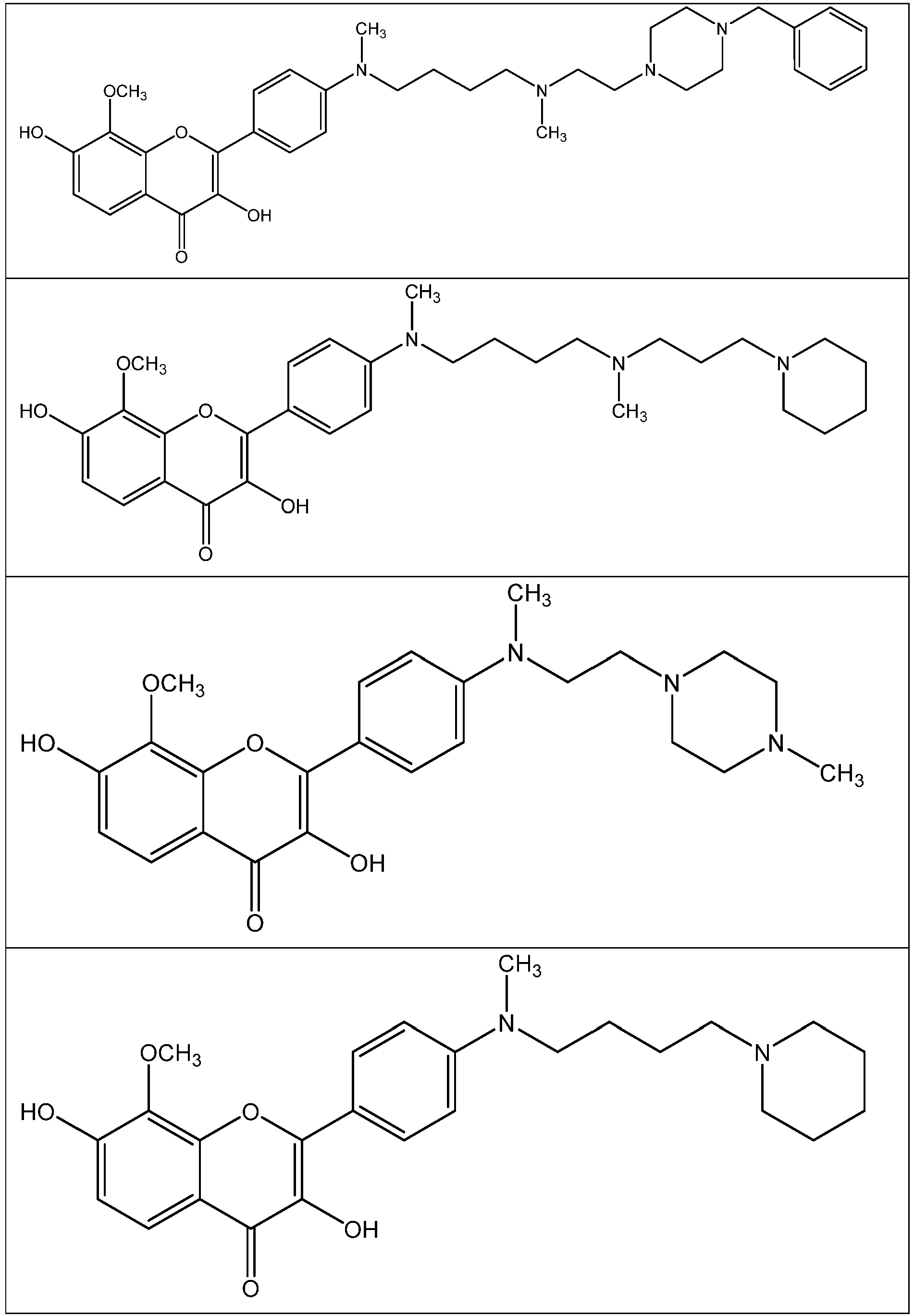

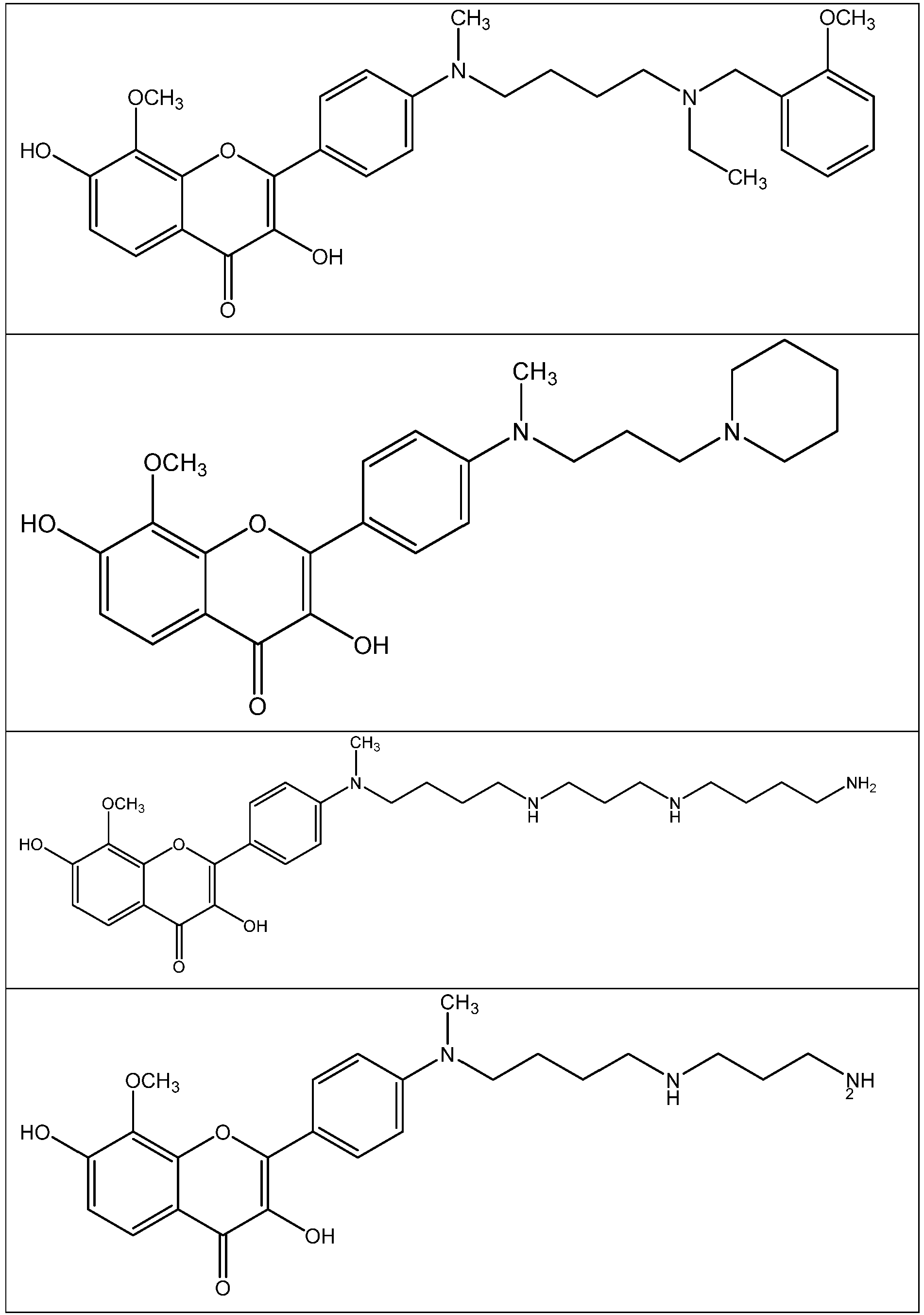

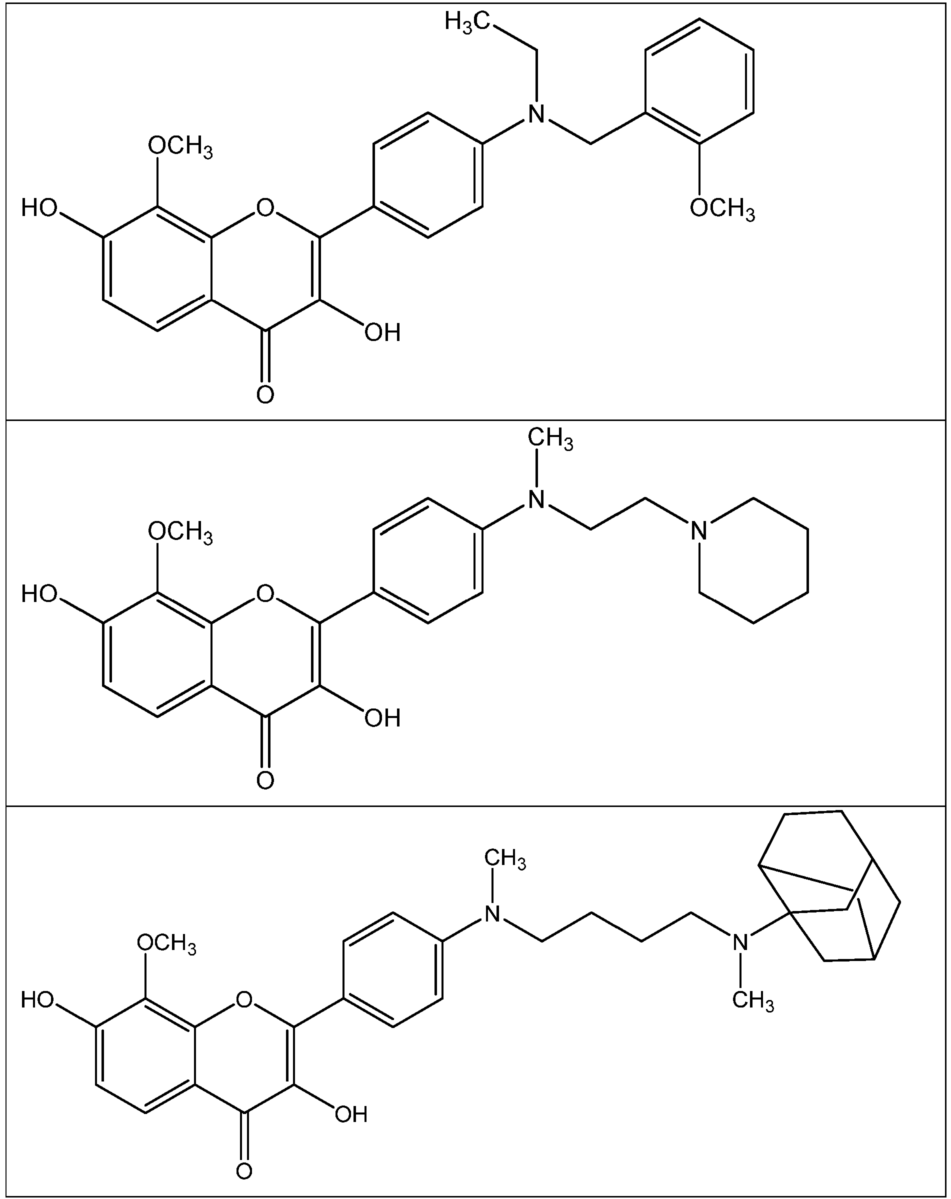

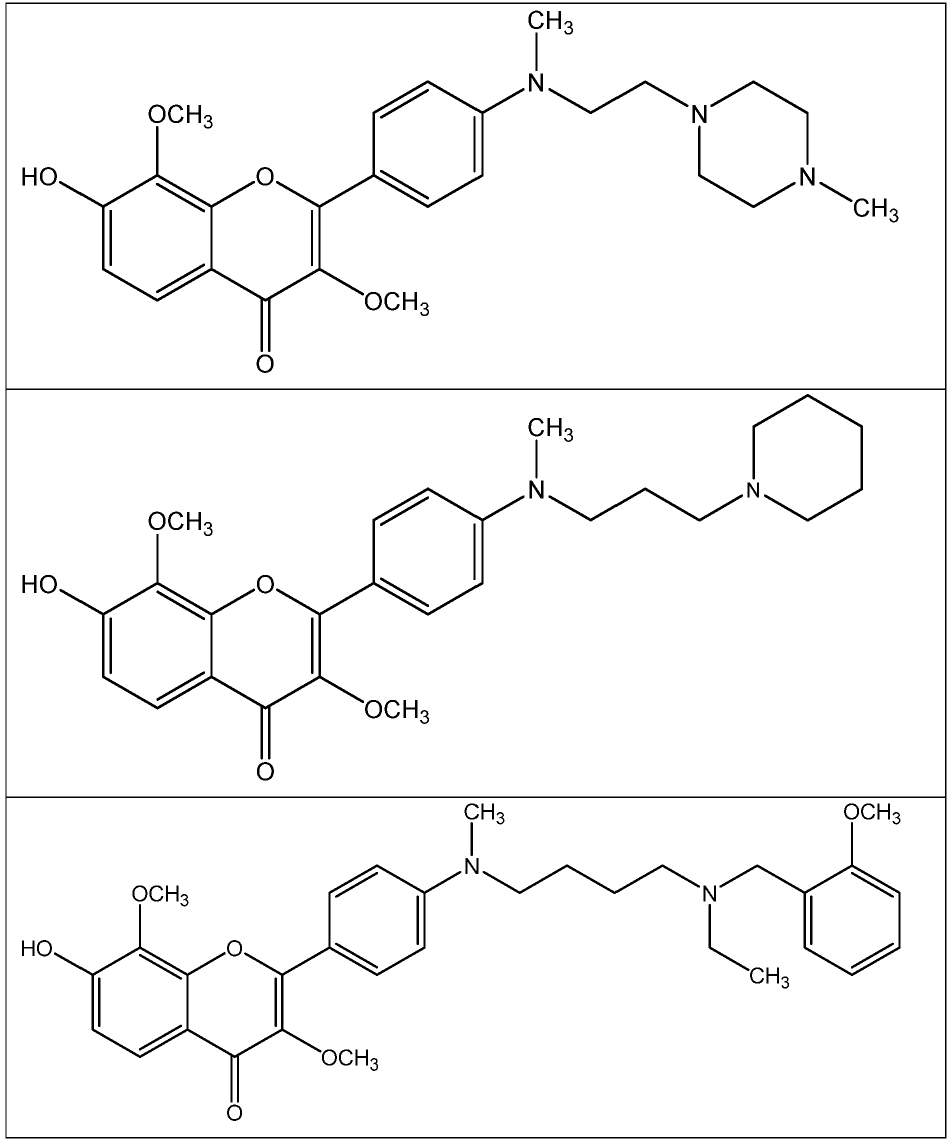

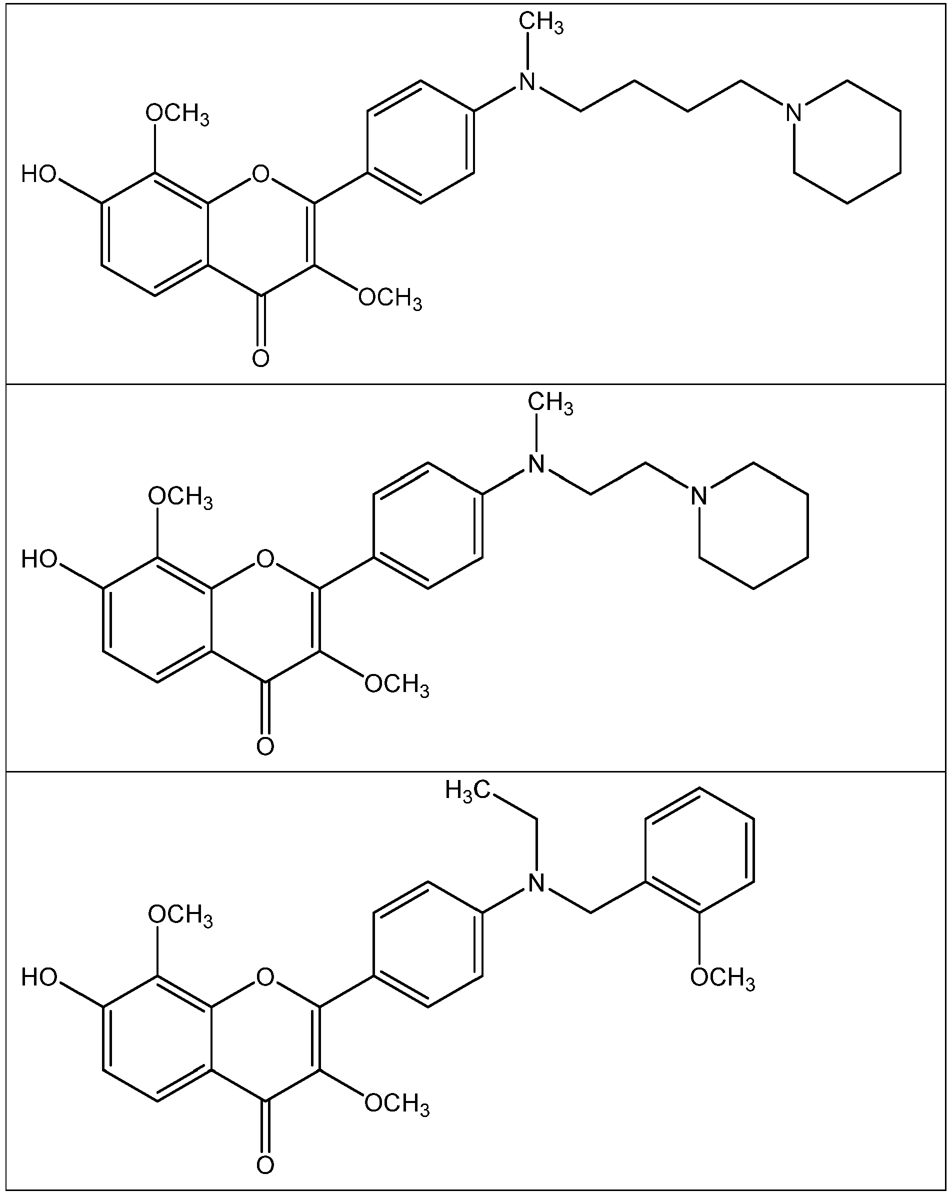

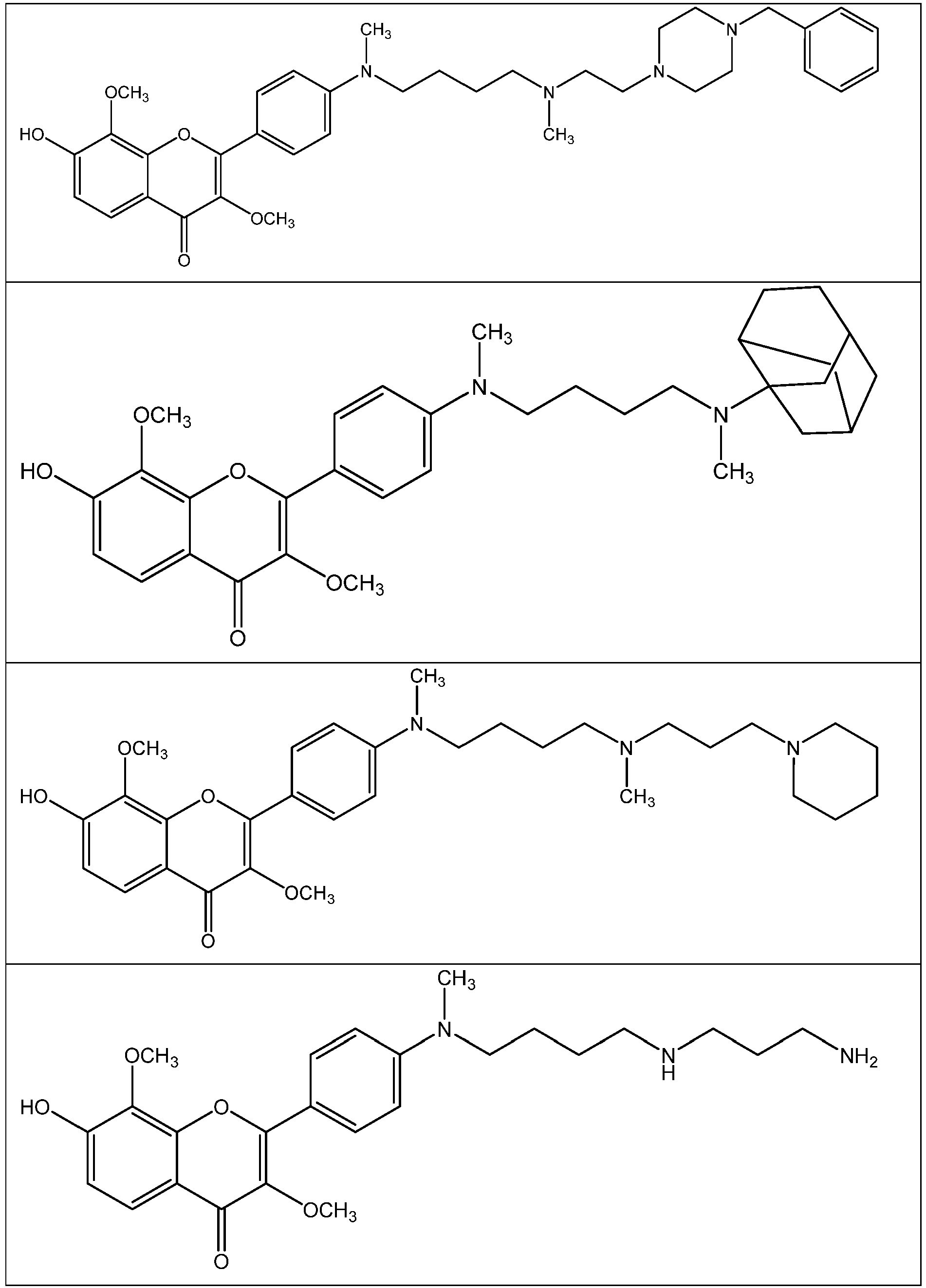

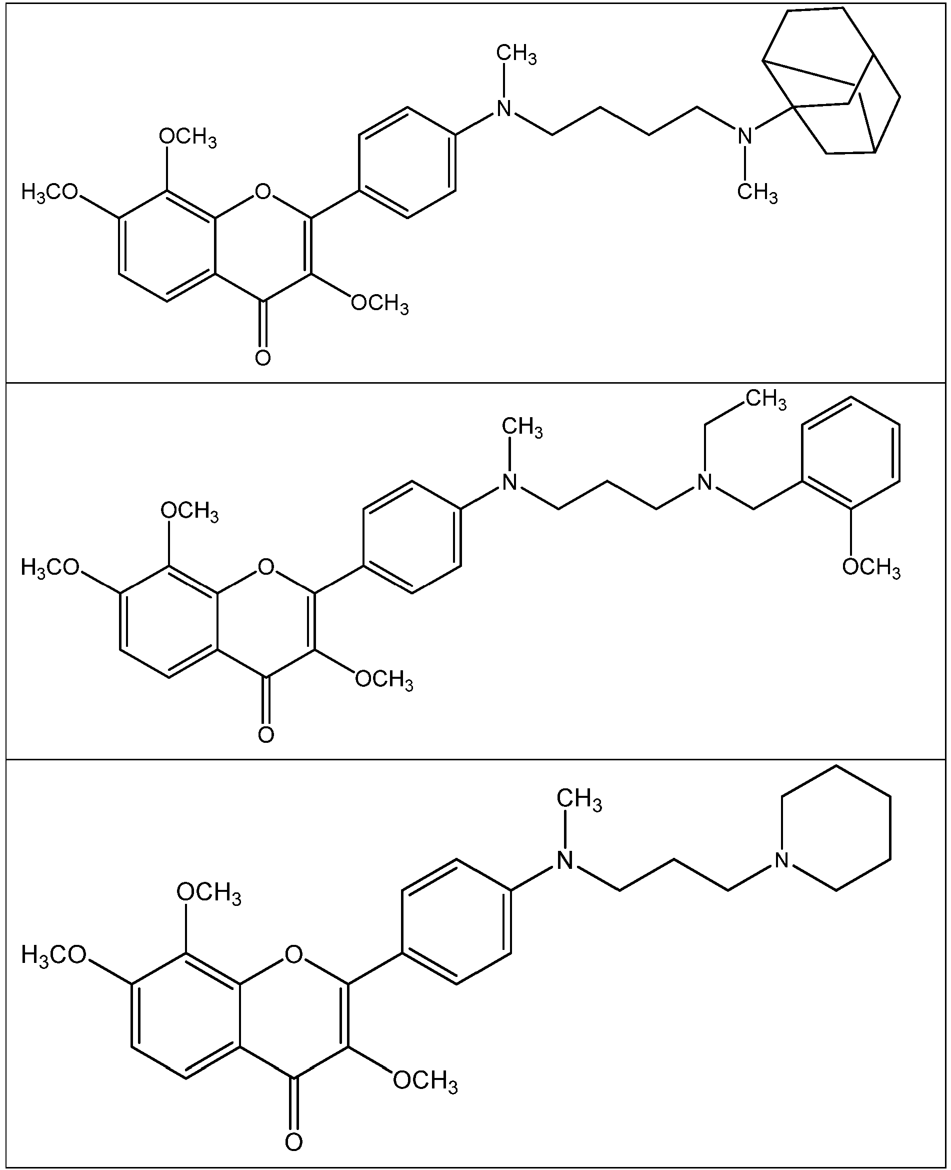

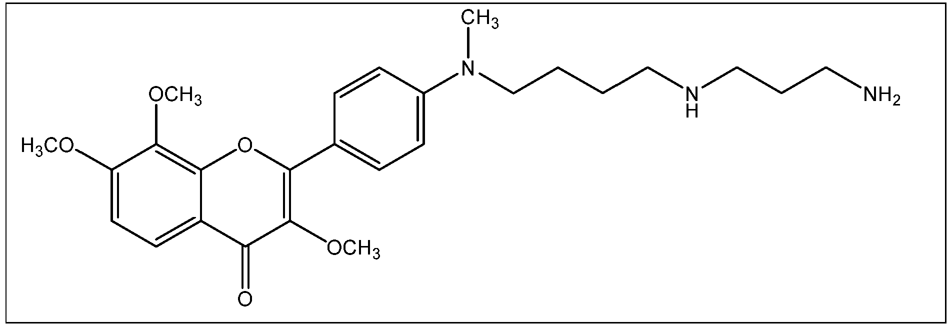

A third aspect of the invention provides a pharmaceutical composition comprising a compound, or a pharmaceutically acceptable salt, multi-salt or solvate of the first or second aspect of the invention, and a pharmaceutically acceptable excipient.

A fourth aspect of the invention provides a compound, or a pharmaceutically acceptable salt, multi-salt or solvate of the first or second aspect of the invention, or a pharmaceutical composition of the third aspect of the invention, for use in medicine, and/or for use in the treatment or prevention of a disease, disorder or condition. In one embodiment, the disease, disorder or condition is cancer.

### Definitions

In the context of the present specification, a "hydrocarbyl" substituent group or a hydrocarbyl moiety in a substituent group only includes carbon and hydrogen atoms but, unless stated otherwise, does not include any heteroatoms, such as N, O or S, in its carbon skeleton. A hydrocarbyl group/moiety may be saturated or unsaturated (including aromatic), and may be straight-chained or branched, or be or include cyclic groups wherein, unless stated otherwise, the cyclic group does not include any heteroatoms, such as N, O or S, in its carbon skeleton. Examples of hydrocarbyl groups include alkyl, alkenyl, alkynyl, cycloalkyl, cycloalkenyl and aryl groups/moieties and combinations of all of these groups/moieties. Typically a hydrocarbyl group is a C₁-C₁₂ hydrocarbyl group. More typically a hydrocarbyl group is a C₁-C₁₀ hydrocarbyl group. A "hydrocarbylene" group is similarly defined as a divalent hydrocarbyl group.

An "alkyl" substituent group or an alkyl moiety in a substituent group may be linear or branched. Examples of alkyl groups/moieties include methyl, ethyl, *n*-propyl, *i*-propyl, *n*-butyl, *i*-butyl, *t*-butyl and *n*-pentyl groups/moieties. Unless stated otherwise, the term "alkyl" does not include "cycloalkyl". Typically an alkyl group is a C₁-C₁₂ alkyl group. More typically an alkyl group is a C₁-C₆ alkyl group. An "alkylene" group is similarly defined as a divalent alkyl group.

An "alkenyl" substituent group or an alkenyl moiety in a substituent group refers to an unsaturated alkyl group or moiety having one or more carbon-carbon double bonds. Examples of alkenyl groups/moieties include ethenyl, propenyl, 1-butenyl, 2-butenyl, 1-pentenyl, 1-hexenyl, 1,3-butadienyl, 1,3-pentadienyl, 1,4-pentadienyl and 1,4-hexadienyl groups/moieties. Unless stated otherwise, the term "alkenyl" does not include "cycloalkenyl". Typically an alkenyl group is a C₂-C₁₂ alkenyl group. More typically an alkenyl group is a C₂-C₆ alkenyl group. An "alkenylene" group is similarly defined as a divalent alkenyl group.

An "alkynyl" substituent group or an alkynyl moiety in a substituent group refers to an unsaturated alkyl group or moiety having one or more carbon-carbon triple bonds. Examples of alkynyl groups/moieties include ethynyl, propargyl, but-1-ynyl and but-2-ynyl. Typically an alkynyl group is a C₂-C₁₂ alkynyl group. More typically an alkynyl group is a C₂-C₆ alkynyl group. An "alkynylene" group is similarly defined as a divalent alkynyl group.

A "haloalkyl" substituent group or haloalkyl group in a substituent group refers to an alkyl, alkenyl, or alkynyl substituent group or moiety including one or more carbon atoms and one or more halo atoms, e.g. Cl, Br, I, or F. Each halo atom replaces a hydrogen of the alkyl, alkenyl, or alkynyl substituent group or moiety. Examples include -CH₂F -CHF₂, -CHI₂, -CHBr₂,-CHCl₂,-CF₃, -CH₂CF₃ and CF₂CH₃.

An "alkoxy" substituent group or alkoxy group in a substituent group refers to an alkyl, alkenyl, or alkynyl substituent group or moiety including one or more carbon atoms and one or more oxygen atoms. Each oxygen atom replaces a carbon atom (for example the terminal or bonding carbon) of the alkyl, alkenyl, or alkynyl substituent group or moiety. Examples include -OCH₃, -OCH₂CH₃, -OCH₂CH₂CH₃, and -OCH(CH₃)(CH₃).

An "alkylthio" substituent group or alkylthio group in a substituent group refers to an alkyl, alkenyl, or alkynyl substituent group or moiety including one or more carbon atoms and one or more sulphur atoms. Each sulphur atom replaces a carbon atom (for example the terminal or bonding carbon) of the alkyl, alkenyl, or alkynyl substituent group or moiety. Examples include -SCH₃, -SCH₂CH₃, -SCH₂CH₂CH₃, and - SCH(CH₃)(CH₃).

An "alkylsulfinyl" substituent group or alkylsulfinyl group in a substituent group refers to an alkyl, alkenyl, or alkynyl substituent group or moiety including one or more carbon atoms and one or more sulfinyl groups (-S(=O)-). Each sulfinyl group replaces a carbon atom (for example the terminal or bonding carbon) of the alkyl, alkenyl, or alkynyl substituent group or moiety. Examples include - S(=O)CH₃, - S(=O)CH₂CH₃, - S(=O)CH₂CH₂CH₃, and - S(=O)CH(CH₃)(CH₃).

An "alkylsulfonyl" substituent group or alkylsulfonyl group in a substituent group refers to an alkyl, alkenyl, or alkynyl substituent group or moiety including one or more carbon atoms and one or more sulfonyl groups (-SO₂-). Each sulfonyl group replaces a carbon atom (for example the terminal or bonding carbon) of the alkyl, alkenyl, or alkynyl substituent group or moiety. Examples include - SO₂(CH₃), - SO₂(CH₂CH₃), - SO₂(CH₂CH₂CH₃), and - SO₂(CH(CH₃)(CH₃)).

An "arylsulfonyl" substituent group or arylsulfonyl group in a substituent group refers to an aryl substituent group or moiety including one or more carbon atoms and one or more sulfonyl groups (-SO₂-). Each sulfonyl group replaces a carbon atom (for example the terminal or bonding carbon) of the alkyl, alkenyl, or alkynyl substituent group or moiety. Examples include - SO₂(CH₃), - SO₂(CH₂CH₃), - SO₂(CH₂CH₂CH₃), and - SO₂(CH(CH₃)(CH₃)).

A "cyclic" substituent group or a cyclic moiety in a substituent group refers to any hydrocarbyl ring, wherein the hydrocarbyl ring may be saturated or unsaturated and may include one or more heteroatoms, e.g. N, O or S, in its carbon skeleton. Examples of cyclic groups include aliphatic cyclic, cycloalkyl, cycloalkenyl, heterocyclic, aryl and heteroaryl groups as discussed below. A cyclic group may be monocyclic, bicyclic (e.g. bridged, fused or spiro), or polycyclic. Typically, a cyclic group is a 3- to 12-membered cyclic group, which means it contains from 3 to 12 ring atoms. More typically, a cyclic group is a 3- to 7-membered monocyclic group, which means it contains from 3 to 7 ring atoms.

A "heterocyclic" substituent group or a heterocyclic moiety in a substituent group refers to a cyclic group or moiety including one or more carbon atoms and one or more heteroatoms, e.g. N, O or S, in the ring structure. Examples of heterocyclic groups include heteroaryl groups as discussed below and non-aromatic heterocyclic groups such as azetidinyl, azetinyl, tetrahydrofuranyl, pyrrolidinyl, tetrahydrothiophenyl, tetrahydropyranyl, piperidinyl, piperazinyl, morpholinyl and thiomorpholinyl groups.

An "aliphatic cyclic" substituent group or aliphatic cyclic moiety in a substituent group refers to a hydrocarbyl cyclic group or moiety that is not aromatic. The aliphatic cyclic group may be saturated or unsaturated and may include one or more heteroatoms, e.g. N, O or S, in its carbon skeleton. Examples include cyclopropyl, cyclohexyl and morpholinyl. Unless stated otherwise, an aliphatic cyclic substituent group or moiety may include monocyclic, bicyclic or polycyclic hydrocarbyl rings.

A "cycloalkyl" substituent group or a cycloalkyl moiety in a substituent group refers to a saturated hydrocarbyl ring containing, for example, from 3 to 7 carbon atoms, examples of which include cyclopropyl, cyclobutyl, cyclopentyl and cyclohexyl. Unless stated otherwise, a cycloalkyl substituent group or moiety may include monocyclic, bicyclic or polycyclic hydrocarbyl rings.

A "cycloalkenyl" substituent group or a cycloalkenyl moiety in a substituent group refers to a non-aromatic unsaturated hydrocarbyl ring having one or more carbon-carbon double bonds and containing, for example, from 3 to 7 carbon atoms, examples of which include cyclopent-1-en-1-yl, cyclohex-1-en-1-yl and cyclohex-1,3-dien-1-yl. Unless stated otherwise, a cycloalkenyl substituent group or moiety may include monocyclic, bicyclic or polycyclic hydrocarbyl rings.

An "aryl" substituent group or an aryl moiety in a substituent group refers to an aromatic hydrocarbyl ring. The term "aryl" includes monocyclic aromatic hydrocarbons and polycyclic fused ring aromatic hydrocarbons wherein all of the fused ring systems (excluding any ring systems which are part of or formed by optional substituents) are aromatic. Examples of aryl groups/moieties include phenyl, naphthyl, anthracenyl and phenanthrenyl. Unless stated otherwise, the term "aryl" does not include "heteroaryl".

A "heteroaryl" substituent group or a heteroaryl moiety in a substituent group refers to an aromatic heterocyclic group or moiety. The term "heteroaryl" includes monocyclic aromatic heterocycles and polycyclic fused ring aromatic heterocycles wherein all of the fused ring systems (excluding any ring systems which are part of or formed by optional substituents) are aromatic. Examples of heteroaryl groups/moieties include the following: 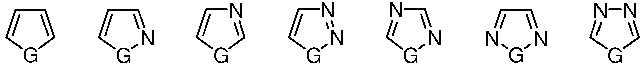 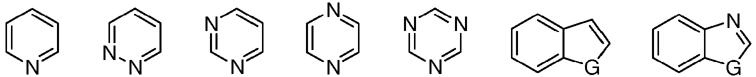 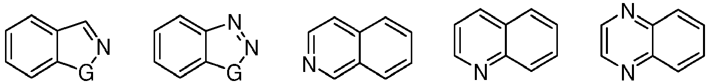 wherein G = O, S or NH.

For the purposes of the present specification, where a combination of moieties is referred to as one group, for example, arylalkyl, arylalkenyl, arylalkynyl, alkylaryl, alkenylaryl or alkynylaryl, the last mentioned moiety contains the atom by which the group is attached to the rest of the molecule. An example of an arylalkyl group is benzyl.

Typically a substituted group comprises 1, 2, 3 or 4 substituents, more typically 1, 2 or 3 substituents, more typically 1 or 2 substituents, and even more typically 1 substituent.

Unless stated otherwise, any divalent bridging substituent (e.g. -O-, -S-, -NH-, -N(R^{β})- or -R^{α}-) of an optionally substituted group or moiety must only be attached to the specified group or moiety and may not be attached to a second group or moiety, even if the second group or moiety can itself be optionally substituted.

The term "halo" includes fluoro, chloro, bromo and iodo.

Where reference is made to a carbon atom of a group being replaced by an N, O or S atom, what is intended is that: 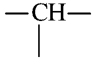 is replaced by 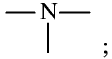
-CH₂- is replaced by -NH-, -O- or -S-;
-CH₃ is replaced by -NH₂, -OH, or -SH;
-CH= is replaced by -N=;
CH₂= is replaced by NH=, O= or S=; or
CH≡ is replaced by N≡.

In the context of the present specification, unless otherwise stated, a Cₓ-C_{y} group is defined as a group containing from x to y carbon atoms. For example, a C₁-C₄ alkyl group is defined as an alkyl group containing from 1 to 4 carbon atoms. Optional substituents and moieties are not taken into account when calculating the total number of carbon atoms in the parent group substituted with the optional substituents and/or containing the optional moieties. For the avoidance of doubt, replacement heteroatoms, e.g. N, O or S, are counted as carbon atoms when calculating the number of carbon atoms in a Cₓ-C_{y} group. For example, a morpholinyl group is to be considered a C₆ heterocyclic group, not a C₄ heterocyclic group.

A "protecting group" refers to a grouping of atoms that when attached to a reactive functional group (e.g. OH) in a compound masks, reduces or prevents reactivity of the functional group.

In the context of the present specification, = is a double bond; ≡ is a triple bond.

The protection and deprotection of functional groups is described in 'Protective Groups in Organic Synthesis', 2nd edition, T.W. Greene and P.G.M Wuts, Wiley-Interscience.

### DETAILED DESCRIPTION OF THE INVENTION

The disclosure provides a compound of formula (1): 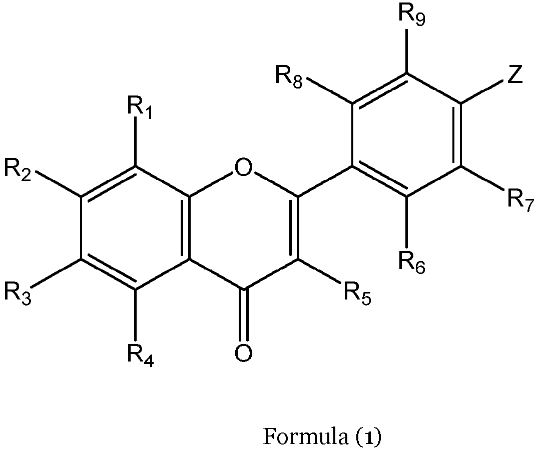 wherein:
Z is selected from:
   -NR¹¹R¹²;
   -N(R¹⁰)-(CH₂)ₚ-NR¹¹R¹²;
   -N(R¹⁰)-(CH₂)_{q}-N(R¹⁰)-(CH₂)_{q}-NR¹¹R¹²; and
   -N(R¹⁰)-(CH₂)ᵣ-N(R¹⁰)-(CH₂)ᵣ-N(R¹⁰)-(CH₂)ᵣ-NR¹¹R¹²;
R¹, R², and R⁴ independently, are selected from -OH, -O-C₁₋₄ alkyl, - OC(O)R₁₃, -OC(O)NHR₁₃, -OC(O)N(R¹³)₂; or R¹ and R² together form -O-CH₂-O-;
R³, R⁴, R⁶, R⁷, R⁸, and R⁹, independently, are selected from H; halo; -CN; -NO₂; -R^{β}; - OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; -NHR^{β}; -N(R^{β})₂; -CHO; -COR^{β}; -COOH; -COOR^{β}; -OCOR^{β}; and benzyl optionally substituted with 1-3 -R^{β};
each -R^{β} is independently selected from a C₁-C₆ alkyl, C₂-C₆ alkenyl, C₂-C₆ alkynyl , -O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C=CH, - CHO, -CON(CH₃)₂ or oxo (=O) groups;
each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β};
R¹¹ and R¹² are independently selected from H, C₁₋₆-alkyl, C₂-C₆ alkenyl, C₂₋₆ alkynyl, C₃₋₁₀ cycloalkyl, and benzyl, wherein each R¹¹ and R¹², when is not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl or benzyl;
each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴;
each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅;
each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl;
each p is independently an integer selected from 1 to 4;
each q is independently an integer selected from 1 to 4; and
each r is independently an integer selected from 1 to 4.

For example, R¹, R², and R⁵, independently, are selected from -OH, -O-C₁₋₄ alkyl, - OC(O)R₁₃, -OC(O)NHR₁₃, -OC(O)N(R¹³)₂. For example, R¹, R², and R⁵, independently, are selected from -OH, and -O-C₁₋₄ alkyl. For example, R¹, R², and R⁵, independently, are selected from -OH and -OCH₃.

For example, R¹ and R² together form -O-CH₂-O-.
R³, R⁴, R⁶, R⁷, R⁸, and R⁹, independently, are selected from H; halo; -CN; -NO₂; -R^{β}; - OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -OH; -OR^{β}; -NH₂; -NHR^{β}; -N(R^{β})₂; -CHO; -COR^{β}; -COOH; -COO R^{β}; and -OCOR^{β}. For example, R³, R⁴, R⁶, R⁷, R⁸, and R⁹, independently, are selected from H; halo; -CN; -NO₂; -R^{β}; -NH₂; -NHR^{β}; -N(R^{β})₂; -CHO; -COR^{β}; -COOH; -COOR^{β}; and -OCOR^{β}. For example, R³, R⁴, R⁶, R⁷, R⁸, and R⁹, independently, are selected from H; halo; -CN; -NO₂; -R^{β}; -NH₂; -NHR^{β}; -N(R^{β})₂; -CHO; -COR^{β}; -COOH; and -COOR^{β}. For example, R³, R⁴, R⁶, R⁷, R⁸, and R⁹, independently, are selected from H;
halo; -CN; -NO₂; and -NH₂. For example, R³, R⁴, R⁶, R⁷, R⁸, and R⁹, are H.
For example, R¹, R², and R⁵, independently, are selected from -OH, -O-C₁₋₄ alkyl, - OC(O)R₁₃, -OC(O)NHR₁₃, -OC(O)N(R¹³)₂; and R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; -OCOR^{β}; and benzyl optionally substituted with 1-3 -R^{β}.
For example, R¹, R², and R⁵, independently, are selected from -OH, and -O-C₁₋₄ alkyl. For example, R³, R⁴, R⁶, R⁷, R⁸, and R⁹, are independently selected from H;
halo; -CN; -NO₂; -R^{β}; -OH; -OR^{β}; -NH₂; -NHR^{β}; -N(R^{β})₂; -CHO; -COR^{β}; -COOH; -COO R^{β}; and -OCOR^{β}.
For example, R¹, R², and R⁵, independently, are selected from -OH, and -O-C₁₋₄ alkyl. For example, R³, R⁴, R⁶, R⁷, R⁸, and R⁹, are independently selected from H;
halo; -CN; -NO₂; -R^{β}; -NH₂; -NHR^{β}; -N(R^{β})₂; -CHO; -COR^{β}; -COOH; -COOR^{β};
and -OCOR^{β}.

For example, R¹, R², and R⁵, independently, are selected from -OH, and -OCH₃. For example, R³, R⁴, R⁶, R⁷, R⁸, and R⁹, are independently selected from H;
halo; -CN; -NO₂; -R^{β}; -NH₂; -NHR^{β}; -N(R^{β})₂; -CHO; -COR^{β}; -COOH; -COOR^{β};
and -OCOR^{β}.

For example, R¹, R², and R⁵, independently, are selected from -OH, and -OCH₃. For example, R³, R⁴, R⁶, R⁷, R⁸, and R⁹, are independently selected from H;
halo; -CN; -NO₂; and -NH₂.

For example, R¹, R², and R⁵, independently, are selected from -OH, and -OCH₃. For example, R³, R⁴, R⁶, R⁷, R⁸, and R⁹, are H.

For example, R¹, R² and R⁵, independently, are selected from -OH, -O-C₁₋₄ alkyl, - OC(O)R₁₃, -OC(O)NHR₁₃, -OC(O)N(R¹³)₂; and R³, R⁴, R⁶, R⁷, R⁸, and R⁹, independently, are selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R³, R⁴, R⁶, R⁷, R⁸, and R⁹, independently, are selected from H; halo; -CN; -NO₂; -SH; -SO₂H; -NH₂; -CHO; -COOH. For example, R³, R⁴, R⁶, R⁷, R⁸, and R⁹ are H.

For example, R¹, R² and R⁵ are independently selected from -OH and -O-C₁₋₄ alkyl, e.g. -OH and -OCH₃; and R³, R⁴, R⁶, R⁷, R⁸, and R⁹, independently, are selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R³, R⁴, R⁶, R⁷, R⁸, and R⁹, independently, are selected from H; halo; -CN; -NO₂; -SH; -SO₂H; -NH₂; -CHO; -COOH. For example, R³, R⁴, R⁶, R⁷, R⁸, and R⁹ are H.

For example, R¹, R² and R⁵, independently, are selected from -OH and -O-C₁₋₄ alkyl; and R³, R⁴, R⁶, R⁷, R⁸, and R⁹, independently, are selected from H;
halo; -CN; -NO₂; -SH; -SO₂H; and -NH₂. For example, R³, R⁴, R⁶, R⁷, R⁸, and R⁹ are H.

For example, R¹, R² and R⁵, independently, are selected from -OH and -OCH₃; and R³, R⁴, R⁶, R⁷, R⁸, and R⁹, independently, are selected from H;
halo; -CN; -NO₂; -SH; -SO₂H; and -NH₂. For example, R³, R⁴, R⁶, R⁷, R⁸, and R⁹ are H.

For example, R¹ is -O-C₁₋₄ alkyl, e.g. -O-Me; R² is -OH; R⁵ is -OH ; and R³, R⁴, R⁶, R⁷, R⁸, and R⁹, independently, are selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R³, R⁴, R⁶, R⁷, R⁸, and R⁹, independently, are selected from H; halo; -CN; -NO₂; -SH; -SO₂H; -NH₂; -CHO; -COOH. For example, R³, R⁴, R⁶, R⁷, R⁸, and R⁹ are H.

For example, R¹, R², and R⁵ are -OH ; and R³, R⁴, R⁶, R⁷, R⁸, and R⁹, independently, are selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R³, R⁴, R⁶, R⁷, R⁸, and R⁹, independently, are selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R³, R⁴, R⁶, R⁷, R⁸, and R⁹, independently, are selected from H; halo; -CN; -NO₂; -SH; -SO₂H; -NH₂; -CHO; -COOH. For example, R³, R⁴, R⁶, R⁷, R⁸, and R⁹ are H.

For example, Z is -NR¹¹R¹².

For example, Z is -N(R¹⁰)-(CH₂)ₚ-NR¹¹R¹².

For example, Z is -N(R¹⁰)-(CH₂)_{q}-N(R¹⁰)-(CH₂)_{q}-NR¹¹R¹².

For example, Z is -N(R¹⁰)-(CH₂)_{q}-N(R¹⁰)-(CH₂)_{q}-N(R¹⁰)-(CH₂)_{q}-NR¹¹R¹².

Each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β}.

For example, each R¹⁰ may independently be selected from H, C₁₋₆ alkyl, and C₂-C₄ alkenyl.

For example, each R¹⁰ may independently be selected from H, C₁₋₃ alkyl, and C₂-C₄ alkenyl.

For example, each R¹⁰ is independently selected from H and C₁₋₆ alkyl.

For example, each R¹⁰ may independently be selected from H and C₁₋₃ alkyl.

For example R¹⁰ may independently be selected from H and -CH₃.

R¹¹ and R¹² are independently selected from H, C₁₋₆-alkyl (e.g. methyl or ethyl), C₂-C₆ alkenyl, C₂₋₆ alkynyl, C₃₋₁₀ cycloalkyl (e.g. adamantyl), and benzyl, wherein each R¹¹ and R¹², when not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl (e.g. methyl) or benzyl.

For example, R¹¹ and R¹² are independently selected from H and C₁₋₆ alkyl (e.g. methyl or ethyl), C₃₋₁₀ cycloalkyl (e.g. adamantyl), and benzyl ; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl (e.g. methyl) or benzyl.

For example, R¹¹ and R¹² are independently selected from H and C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, R¹¹ and R¹² are independently selected from H and C₁₋₆ alkyl (e.g. methyl or ethyl), C₃₋₁₀ cycloalkyl (e.g. adamantyl), and benzyl; wherein each R¹¹ and R¹², when not H, are independently optionally substituted with 1 or 2 -R^{β}.

For example, R¹¹ is H, and R¹² is selected from H and C₁₋₆ alkyl (methyl or ethyl), C₃₋₁₀ cycloalkyl (e.g. adamantyl), and benzyl; wherein R¹², when not H, is optionally substituted with 1 -R^{β}.

For example, R¹¹ and R¹² are both H.

For example, R¹¹ is H, and R¹² is benzyl, optionally substituted with 1 -R^{β}. For example, R¹¹ is H, and R¹² is benzyl substituted with methoxy, e.g. R¹² is ortho-methoxy-benzyl. When R¹¹ and R¹² together form a 5- or 6-membered heterocycle as described above, it may be a 5- or 6-membered heterocycle optionally having one additional heteroatom selected from N and O; wherein the 5- or 6-membered heterocycle is optionally substituted with 1 or 2 C₁₋₄ alkyl. In this respect, the 5- or 6-membered heterocycle may be morpholine, piperidine, piperazine, or pyrrolidine optionally substituted with 1 or 2 C₁₋₄ alkyl. For example, the 5- or 6-membered heterocycle may be piperazine, 4-methyl piperazine, or pyrrolidine.

Each -R^{β} is independently selected from a C₁-C₆ alkyl, C₂-C₆ alkenyl, C₂-C₆ alkynyl , - O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C=CH, -CHO, - CON(CH₃)₂ or oxo (=O) groups.

For example, each -R^{β} is independently selected from a C₁-C₃ alkyl and -O(C₁-C₂ alkyl), and any -R^{β} may optionally be substituted with one or more
halo, -OH, -NH₂, -CN, -NO₂, -C=CH, -CHO, -CON(CH₃)₂ or oxo (=O) groups.

For example, each -R^{β} is independently selected from a C₁-C₃ alkyl and -O(C₁-C₂ alkyl), and any -R^{β} may optionally be substituted with one or more
halo, -OH, -NH₂, -CN, -NO₂, -C=CH, -CHO, -CON(CH₃)₂ or oxo (=O) groups.

For example, each -R^{β} is independently selected from C₁-C₂ alkyl and -O(C₁-C₂ alkyl), and any -R^{β} may optionally be substituted with one or more halo, -OH, -NH₂, -CN,
or -NO₂ groups.

Each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴.

For example, each R¹³ is independently selected from a H and C₁-C₃ alkyl, wherein
any -R¹³ may optionally be substituted with one or more -R¹⁴.

For example, each R¹³ is independently selected from a H and C₁-C₂ alkyl.

Each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅.

For example, each R¹⁴ is independently selected from a C₁-C₆ alkyl, halo, -NO₂, -CN, - OH, -NH₂, mercapto, formyl, carboxy, carbamoyl, and C₁₋₆ alkoxy, wherein any -R₁₄ may optionally be substituted with one or more -R₁₅.

Each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl.

For example, each -R¹⁵ is independently selected from halogen, nitro, cyano, hydroxy, trifluoromethoxy, trifluoromethyl, amino, formyl, and carboxy.

Each p is independently an integer selected from 1 to 4.

For example, each p is independently an integer selected from 2 to 4.

For example, each p is independently selected from 3 and 4.

Each q is independently an integer selected from 1 to 4.

For example, each q is independently an integer selected from 2 to 4.

For example, each q is independently selected from 3 and 4.

For example, Z may be -N(R¹⁰)-(CH₂)₃-N(R¹⁰)-(CH₂)₄-NR¹¹R¹².

For example, Z may be -N(R¹⁰)-(CH₂)₄-N(R¹⁰)-(CH₂)₃-NR¹¹R¹².

Each r is independently an integer selected from 1 to 4.

For example, each r is independently an integer selected from 2 to 4.

For example, each r is independently selected from 3 and 4.

For example, Z may be -N(R¹⁰)-(CH₂)ᵣ-N(R¹⁰)-(CH₂)ᵣ-N(R¹⁰)-(CH₂)ᵣ-NR¹¹R¹².

For example, Z is -NR¹¹R¹². For example, Z is -NR¹¹R¹²; R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, Z is -N(R¹⁰)-(CH₂)ₚ-NR¹¹R¹². For example, Z is -N(R¹⁰)-(CH₂)ₚ-NR¹¹R¹²; R¹⁰ is H or C₁₋₆ alkyl; and R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, Z is -N(R¹⁰)-(CH₂)ₚ-NR¹¹R¹²; and p is 1-4. For example, p is 2-4, for example 2 or 3.

For example, Z is -N(R¹⁰)-(CH₂)_{q}-N(R¹⁰)-(CH₂)_{q}-NR¹¹R¹²; and q is independently selected from 1-4. For example, q may be 2, 3 or 4. For example, Z is -N(R¹⁰)-(CH₂)_{q}-N(R¹⁰)-(CH₂)_{q}-NR¹¹R¹²; each R¹⁰ is independently selected from H and C₁₋₆ alkyl; and and R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, R³, R⁴, R⁷, R⁸ and R⁹ are H, for example as represented by Formula 1A below.

For example, the compound may be a compound of Formula 1A: 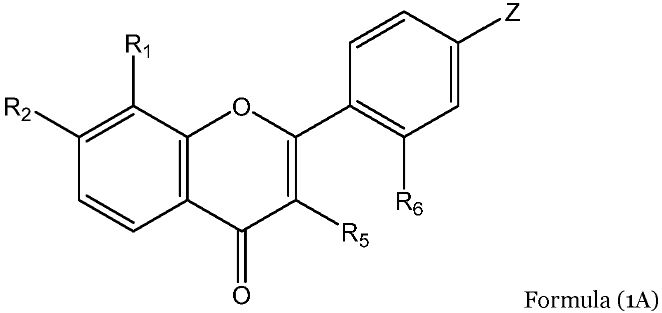 wherein R¹, R², R⁵, R⁶ and Z are as defined herein.

For example, the compound may be a compound of Formula (1A) wherein:
Z is selected from:
   -NR¹¹R¹²;
   -N(R¹⁰)-(CH₂)ₚ-NR¹¹R¹²;
   -N(R¹⁰)-(CH₂)_{q}-N(R¹⁰)-(CH₂)_{q}-NR¹¹R¹²; and
   -N(R¹⁰)-(CH₂)ᵣ-N(R¹⁰)-(CH₂)ᵣ-N(R¹⁰)-(CH₂)ᵣ-NR¹¹R¹²;
R¹, R², and R⁵ independently, are selected from -OH, -O-C₁₋₄ alkyl, - OC(O)R₁₃, -OC(O)NHR¹³, -OC(O)N(R¹³)₂; or R¹ and R² together form -O-CH₂-O-;
R³, R⁴, R⁶, R7, R⁸, and R⁹, independently, are selected from H; halo; -CN; -NO₂; -R^{β}; -
   OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β};
each -R^{β} is independently selected from a C₁-C₆ alkyl, C₂-C₆ alkenyl, C₂-C₆ alkynyl , -O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C=CH, - CHO, -CON(CH₃)₂ or oxo (=O) groups;
each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β};
R¹¹ and R¹² are independently selected from H, C₁₋₆-alkyl, C₂-C₆ alkenyl, C₂₋₆ alkynyl, C₃₋₁₀ cycloalkyl, and benzyl, wherein each R¹¹ and R¹², when is not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl or benzyl;
each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴;
each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅;
each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl;
each p is independently an integer selected from 1 to 4; and
each q is independently an integer selected from 1 to 4.

R¹, R², and R⁵, independently, are selected from -OH, -O-C₁₋₄ alkyl, -
OC(O)R₁₃, -OC(O)NHR¹³, -OC(O)N(R¹³)₂. For example, R¹, R², and R⁵, independently, are selected from -OH, and -O-C₁₋₄ alkyl. For example, R¹, R², and R⁵, independently, are selected from -OH and -OCH₃.

For example, R¹ and R² together form -O-CH₂-O-.

R⁶ is selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOR^{β}; and benzyl optionally substituted with 1-3 -R^{β}. For example, R⁶ is selected from H;
halo; -CN; -NO₂; -R^{β}; -OH; -OR^{β}; -NH₂; -NHR^{β}; -N(R^{β})₂; -CHO; -COR^{β}; -COOH; -COO R^{β}; and -OCOR^{β}. For example, R⁶ is selected from H;
halo; -CN; -NO₂; -R^{β}; -NH₂; -NHR^{β}; -N(R^{β})₂; -CHO; -COR^{β}; -COOH; -COOR^{β};
and -OCOR^{β}. For example, R⁶ is selected from H;
halo; -CN; -NO₂; -R^{β}; -NH₂; -NHR^{β}; -N(R^{β})₂; -CHO; -COR^{β}; -COOH; and -COOR^{β}. For example, R⁶ is selected from H; halo; -CN; -NO₂; and -NH₂. For example, R⁶ is H.

For example, R¹, R², and R⁵, independently, are selected from -OH, -O-C₁₋₄ alkyl, - OC(O)R₁₃, -OC(O)NHR¹³, -OC(O)N(R¹³)₂; and R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; -OCOR^{β}; and benzyl optionally substituted with 1-3 -R^{β}.

For example, R¹, R², and R⁵, independently, are selected from -OH, and -O-C₁₋₄ alkyl. For example, R⁶ is selected from H;
halo; -CN; -NO₂; -R^{β}; -OH; -OR^{β}; -NH₂; -NHR^{β}; -N(R^{β})₂; -CHO; -COR^{β}; -COOH; -COO R^{β}; and -OCOR^{β}.

For example, R¹, R², and R⁵, independently, are selected from -OH, and -O-C₁₋₄ alkyl. For example, R⁶ is selected from H;
halo; -CN; -NO₂; -R^{β}; -NH₂; -NHR^{β}; -N(R^{β})₂; -CHO; -COR^{β}; -COOH; -COOR^{β};
and -OCOR^{β}.

For example, R¹, R², and R⁵, independently, are selected from -OH, and -OCH₃. For example, R⁶ is selected from H;
halo; -CN; -NO₂; -R^{β}; -NH₂; -NHR^{β}; -N(R^{β})₂; -CHO; -COR^{β}; -COOH; -COOR^{β};
and -OCOR^{β}.

For example, R¹, R², and R⁵, independently, are selected from -OH, and -OCH₃. For example, R⁶ is selected from H; halo; -CN; -NO₂; and -NH₂.

For example, R¹, R², and R⁵, independently, are selected from -OH, and -OCH₃. For example, R⁶ is H.

For example, R¹, R² and R⁵, independently, are selected from -OH, -O-C₁₋₄ alkyl, - OC(O)R₁₃, -OC(O)NHR¹³, -OC(O)N(R¹³)₂; and R⁶ is selected from H;
halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R⁶ is selected from H;
halo; -CN; -NO₂; -SH; -SO₂H; -NH₂; -CHO; -COOH. For example, R⁶ is H.

For example, R¹, R² and R⁵ are independently selected from -OH and -O-C₁₋₄ alkyl, e.g. -OH and -OCH₃; and R⁶ is selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R⁶ is selected from H;
halo; -CN; -NO₂; -SH; -SO₂H; -NH₂; -CHO; -COOH. For example, R⁶ is H.

For example, R¹, R² and R⁵, independently, are selected from -OH and -O-C₁₋₄ alkyl; and R⁶ is selected from H; halo; -CN; -NO₂; -SH; -SO₂H; and -NH₂. For example, R⁶ is H.

For example, R¹, R² and R⁵, independently, are selected from -OH and -OCH₃; and R⁶ is selected from H; halo; -CN; -NO₂; -SH; -SO₂H; and -NH₂. For example, R⁶ is H.

For example, R¹ is -O-C₁₋₄ alkyl, e.g. -O-Me; R² is -OH; R⁵ is -OH ; and R⁶ is selected from H; halo; -CN; -NO₂; -R^{β}; -
OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R⁶ is selected from H;
halo; -CN; -NO₂; -SH; -SO₂H; -NH₂; -CHO; -COOH. For example, R⁶ is H.

For example, R¹, R², and R⁵ are -OH ; and R⁶ is selected from H; halo; -CN; -NO₂; -R^{β}; - OH, -OR^{β}; -SH; -SR^{β}; -SOR^{β}; -SO₂H; -SO₂R^{β}; -SO₂NH₂; -SO₂NHR^{β}; -SO₂N(R^{β})₂; -NH₂; - NHR^{β}; -N(R^{β})₂; -CHO; -COR^{β}; -COOH; -COOR^{β}; -OCOR^{β}; and benzyl optionally substituted with 1-3 -R^{β}. For example, R⁶ is selected from H;
halo; -CN; -NO₂; -R^{β}; -SH; -SR^{β}; -SOR^{β}; -SO₂H; -SO₂R^{β}; -SO₂NH₂; -SO₂NHR^{β}; -SO₂N(R ^{β})₂; -NH₂; -NHR^{β}; -N(R^{β})₂; -CHO; -COR^{β}; -COOH; -COOR^{β}; and benzyl optionally substituted with 1-3 -R^{β}. For example, R⁶ is selected from H;
halo; -CN; -NO₂; -SH; -SO₂H; -NH₂; -CHO; -COOH. For example, R⁶ is H.

For example, Z is -NR¹¹R¹².

For example, Z is -N(R¹⁰)-(CH₂)ₚ-NR¹¹R¹².

For example, Z is -N(R¹⁰)-(CH₂)_{q}-N(R¹⁰)-(CH₂)_{q}-NR¹¹R¹².

For example, Z is -N(R¹⁰)-(CH₂)_{q}-N(R¹⁰)-(CH₂)_{q}-N(R¹⁰)-(CH₂)_{q}-NR¹¹R¹².

Each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β}.

For example, each R¹⁰ may independently be selected from H, C₁₋₆ alkyl, and C₂-C₄ alkenyl.

For example, each R¹⁰ may independently be selected from H, C₁₋₃ alkyl, and C₂-C₄ alkenyl.

For example, each R¹⁰ is independently selected from H and C₁₋₆ alkyl.

For example, each R¹⁰ may independently be selected from H and C₁₋₃ alkyl.

For example, each R¹⁰ may independently be selected from H and -CH₃.

R¹¹ and R¹² are independently selected from H, C₁₋₆-alkyl (e.g. methyl or ethyl), C₂-C₆ alkenyl, C₂₋₆ alkynyl, C₃₋₁₀ cycloalkyl (e.g. adamantyl), and benzyl, wherein each R¹¹ and R¹², when not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl (e.g. methyl) or benzyl.

For example, R¹¹ and R¹² are independently selected from H and C₁₋₆ alkyl (e.g. methyl or ethyl), C₃₋₁₀ cycloalkyl (e.g. adamantyl), and benzyl ; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl (e.g. methyl) or benzyl.

For example, R¹¹ and R¹² are independently selected from H and C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, R¹¹ and R¹² are independently selected from H and C₁₋₆ alkyl (e.g. methyl or ethyl), C₃₋₁₀ cycloalkyl (e.g. adamantyl), and benzyl; wherein each R¹¹ and R¹², when not H, are independently optionally substituted with 1 or 2 -R^{β}.

For example, R¹¹ is H, and R¹² is selected from H and C₁₋₆ alkyl (methyl or ethyl), C₃₋₁₀ cycloalkyl (e.g. adamantyl), and benzyl; wherein R¹², when not H, is optionally substituted with 1 -R^{β}.

For example, R¹¹ and R¹² are both H.

For example, R¹¹ is H, and R¹² is benzyl, optionally substituted with 1 -R^{β}. For example, R¹¹ is H, and R¹² is benzyl substituted with methoxy, e.g. R¹² is ortho-methoxy-benzyl. When R¹¹ and R¹² together form a 5- or 6-membered heterocycle as described above, it may be a 5- or 6-membered heterocycle optionally having one additional heteroatom selected from N and O; wherein the 5- or 6-membered heterocycle is optionally substituted with 1 or 2 C₁₋₄ alkyl. In this respect, the 5- or 6-membered heterocycle may be morpholine, piperidine, piperazine, or pyrrolidine optionally substituted with 1 or 2 C₁₋₄ alkyl. For example, the 5- or 6-membered heterocycle may be piperazine, 4-methyl piperazine, or pyrrolidine.

Each -R^{β} is independently selected from a C₁-C₆ alkyl, C₂-C₆ alkenyl, C₂-C₆ alkynyl , - O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C=CH, -CHO, - CON(CH₃)₂ or oxo (=O) groups.

For example, each -R^{β} is independently selected from a C₁-C₃ alkyl and -O(C₁-C₂ alkyl), and any -R^{β} may optionally be substituted with one or more
halo, -OH, -NH₂, -CN, -NO₂, -C=CH, -CHO, -CON(CH₃)₂ or oxo (=O) groups.

For example, each -R^{β} is independently selected from a C₁-C₃ alkyl and -O(C₁-C₂ alkyl), and any -R^{β} may optionally be substituted with one or more
halo, -OH, -NH₂, -CN, -NO₂, -C=CH, -CHO, -CON(CH₃)₂ or oxo (=O) groups.

For example, each -R^{β} is independently selected from C₁-C₂ alkyl and -O(C₁-C₂ alkyl), and any -R^{β} may optionally be substituted with one or more halo, -OH, -NH₂, -CN,
or -NO₂ groups.

Each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴.

For example, each R¹³ is independently selected from a H and C₁-C₃ alkyl, wherein
any -R¹³ may optionally be substituted with one or more -R¹⁴.

For example, each R¹³ is independently selected from a H and C₁-C₂ alkyl.

Each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅.

For example, each R¹⁴ is independently selected from a C₁-C₆ alkyl, halo, -NO₂, -CN, - OH, -NH₂, mercapto, formyl, carboxy, carbamoyl, and C₁₋₆ alkoxy, wherein any -R₁₄ may optionally be substituted with one or more -R₁₅.

Each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl.

For example, each -R¹⁵ is independently selected from halogen, nitro, cyano, hydroxy, trifluoromethoxy, trifluoromethyl, amino, formyl, and carboxy.

Each p is independently an integer selected from 1 to 4.

For example, each p is independently an integer selected from 2 to 4.

For example, each p is independently selected from 3 and 4.

Each q is independently an integer selected from 1 to 4.

For example, each q is independently an integer selected from 2 to 4.

For example, each q is independently selected from 3 and 4.

For example, Z may be -N(R¹⁰)-(CH₂)₃-N(R¹⁰)-(CH₂)₄-NR¹¹R¹².

For example, Z may be -N(R¹⁰)-(CH₂)₄-N(R¹⁰)-(CH₂)₃-NR¹¹R¹².

Each r is independently an integer selected from 1 to 4.

For example, each r is independently an integer selected from 2 to 4.

For example, each r is independently selected from 3 and 4.

For example, Z may be -N(R¹⁰)-(CH₂)ᵣ-N(R¹⁰)-(CH₂)ᵣ-N(R¹⁰)-(CH₂)ᵣ-NR¹¹R¹².

For example, Z is -NR¹¹R¹². For example, Z is -NR¹¹R¹²; R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, Z is -N(R¹⁰)-(CH₂)ₚ-NR¹¹R¹². For example, Z is -N(R¹⁰)-(CH₂)ₚ-NR¹¹R¹²; R¹⁰ is H or C₁₋₆ alkyl; and R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

For example, Z is -N(R¹⁰)-(CH₂)ₚ-NR¹¹R¹²; and p is 1-4. For example, p is 2-4, for example 2 or 3.

For example, Z is -N(R¹⁰)-(CH₂)_{q}-N(R¹⁰)-(CH₂)_{q}-NR¹¹R¹²; and q is independently selected from 1-4. For example, q may be 2, 3 or 4. For example, Z is -N(R¹⁰)-(CH₂)_{q}-N(R¹⁰)-(CH₂)_{q}-NR¹¹R¹²; each R¹⁰ is independently selected from H and C₁₋₆ alkyl; and and R¹¹ and R¹² are independently selected from H; C₁₋₆ alkyl; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl.

The disclosure provides a compound selected from compounds listed in Table A.

**Table A:**

| | |
|---|---|
| **SND 437** | 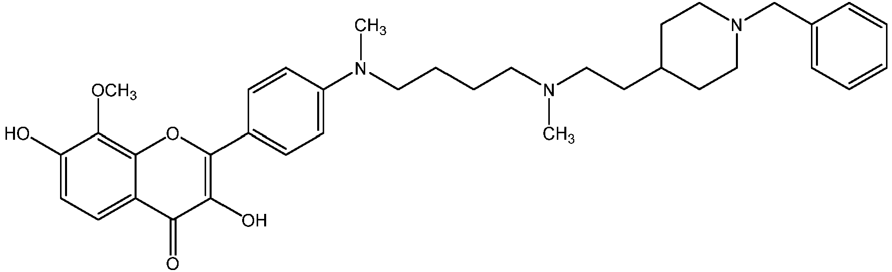 |
| **SND 438** | 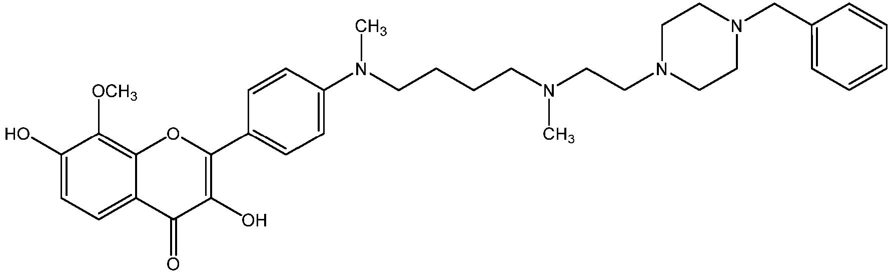 |
| **SND 439** | 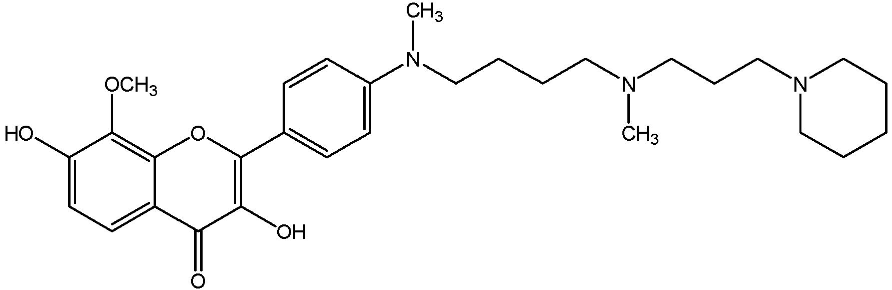 |
| **SND 440** | 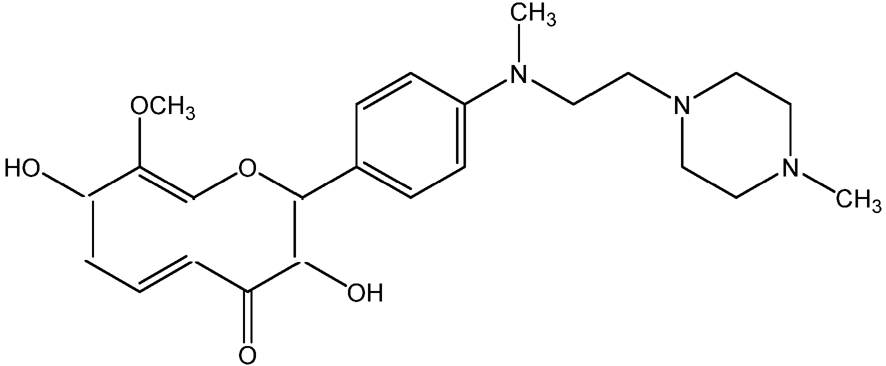 |
| **SND 441** | 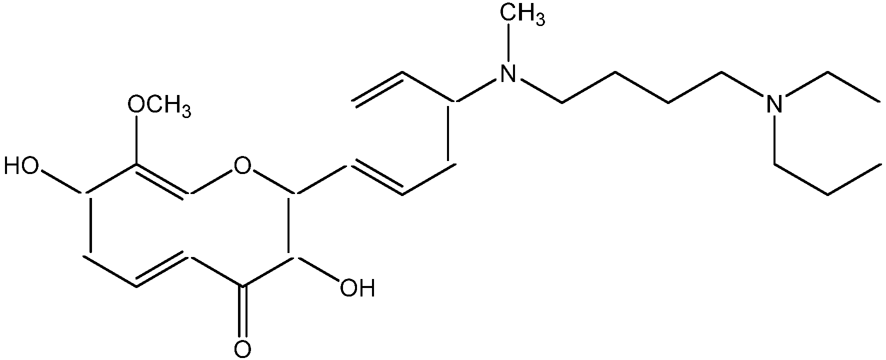 |
| **SND 443** | 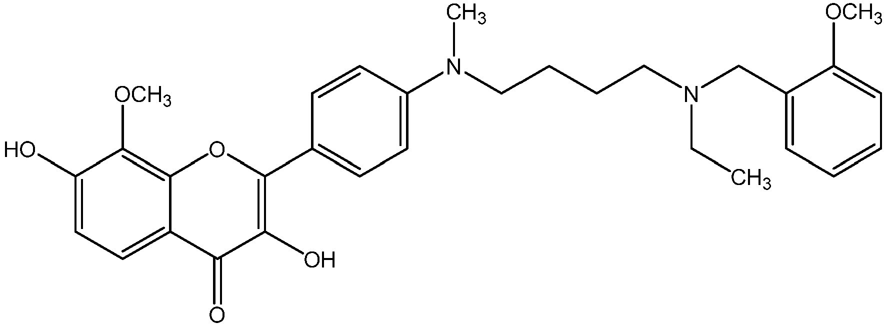 |
| **SND 444** | 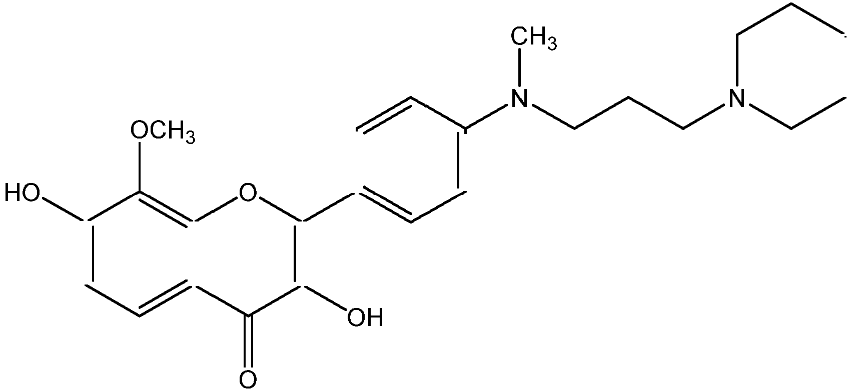 |
| **SND 445** | 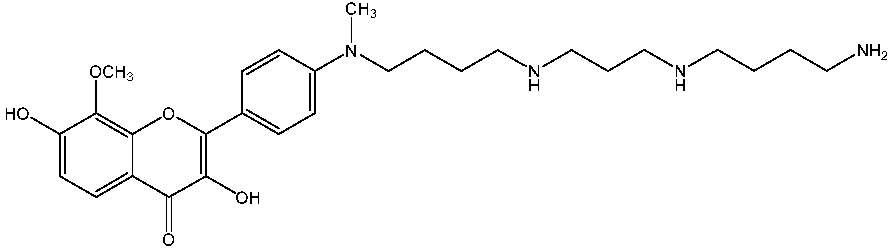 |
| **SND 446** | 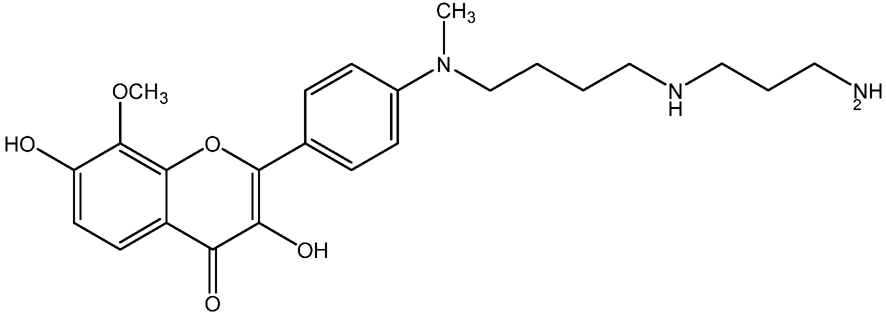 |
| **SND 447** | 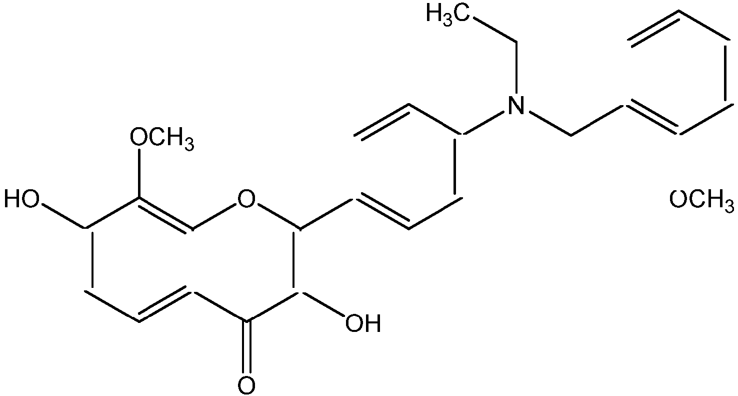 |
| **SND 448** | 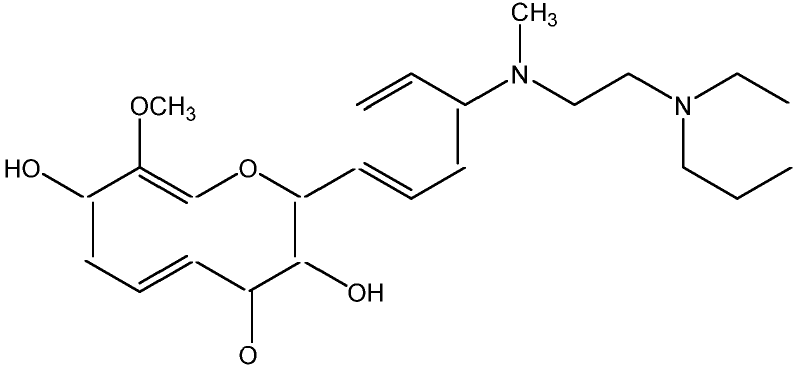 |
| **SND 449** | 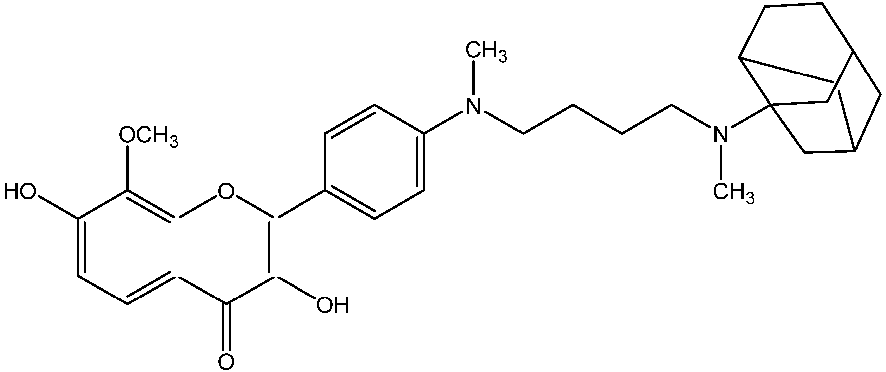 |
| **SND 450** | 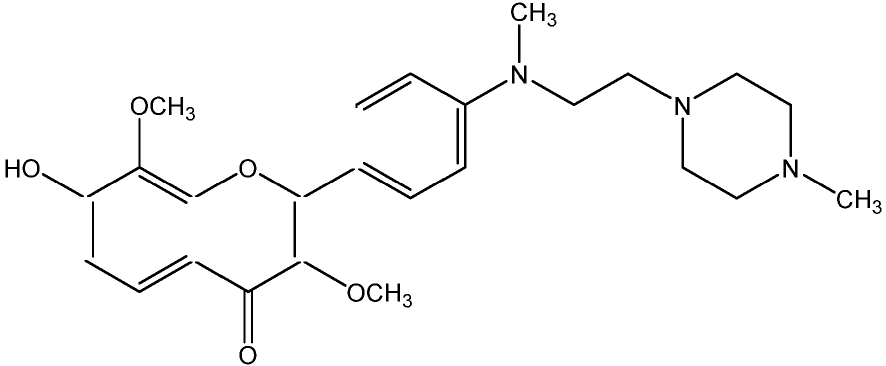 |
| **SND 451** | 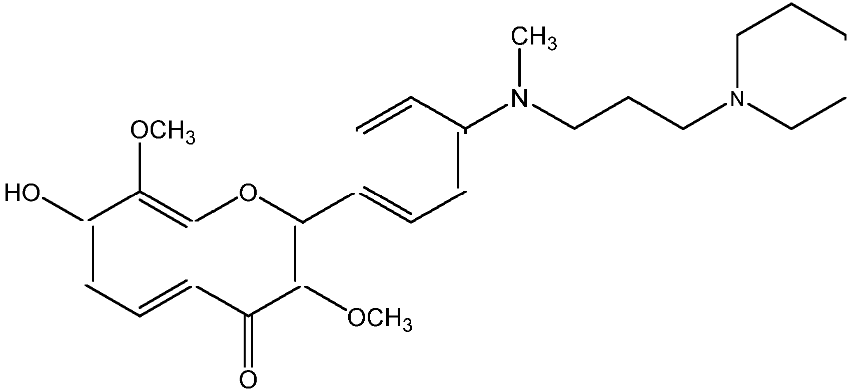 |
| **SND 453** | 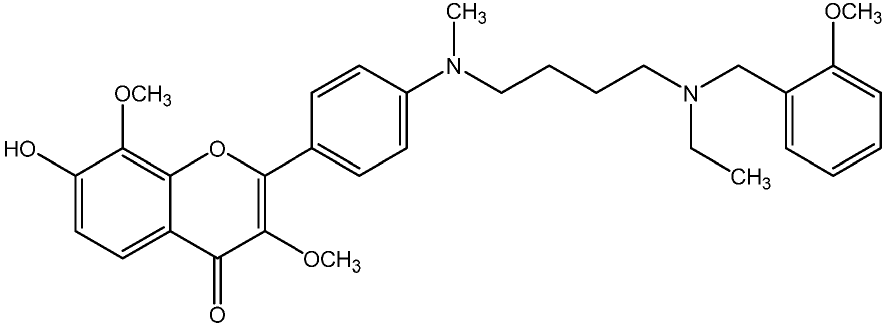 |
| **SND 454** | 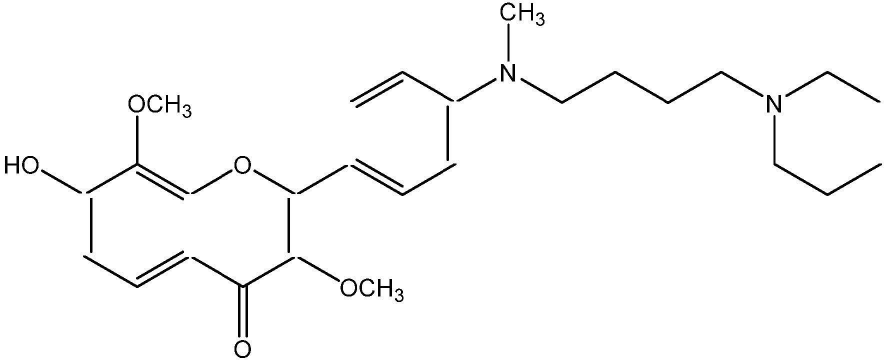 |
| **SND 455** | 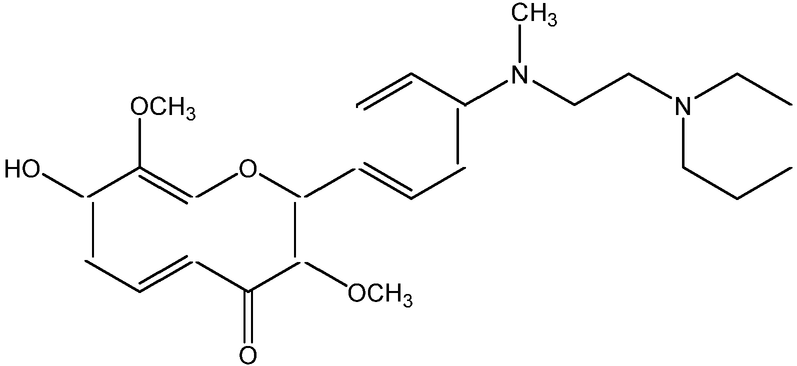 |
| **SND 456** | 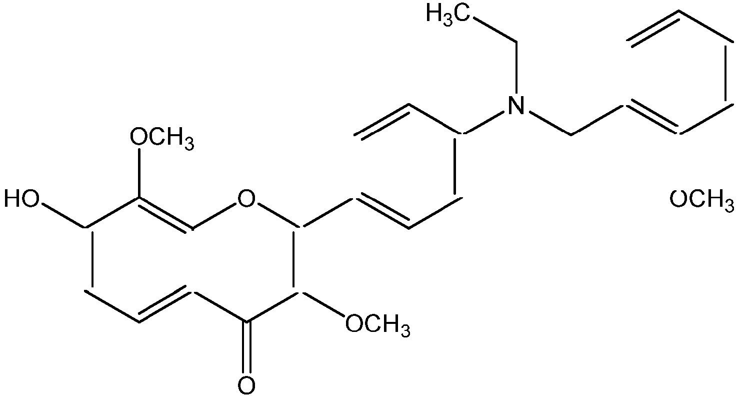 |
| **SND 457** | 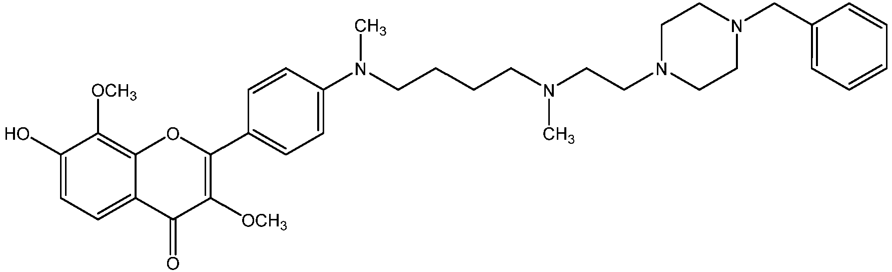 |
| **SND 458** | 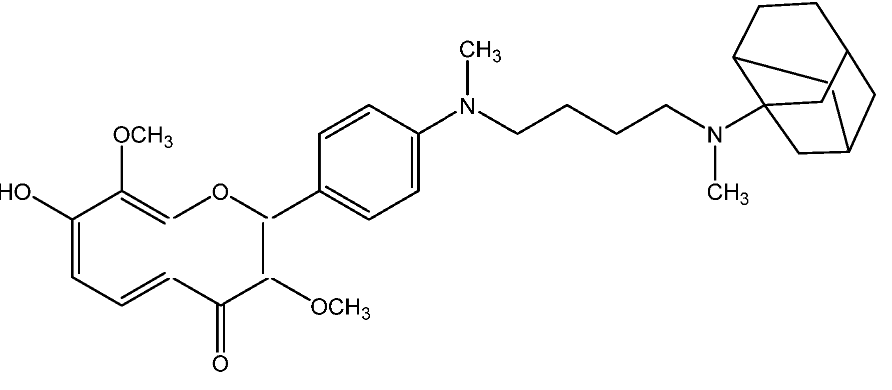 |
| **SND 459** | 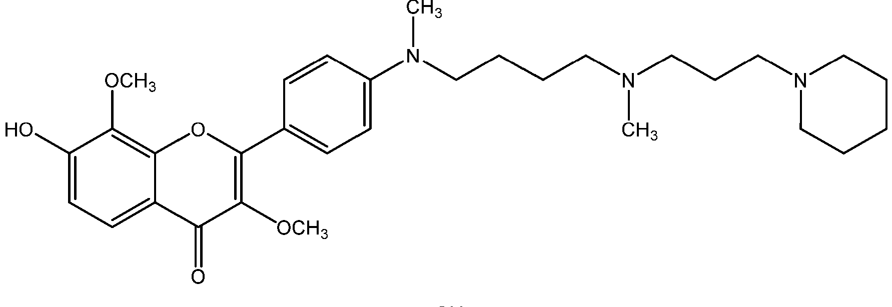 |
| **SND 461** | 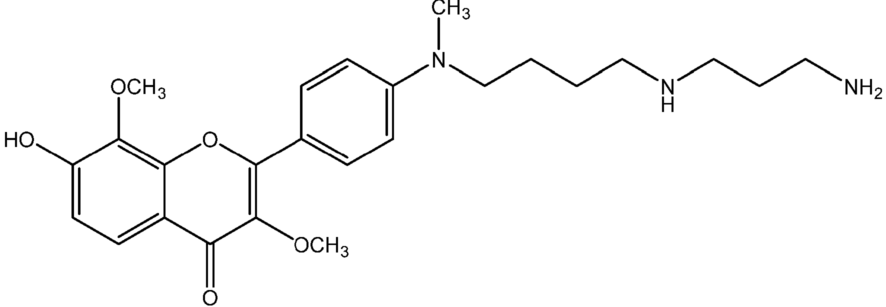 |
| **SND 462** | 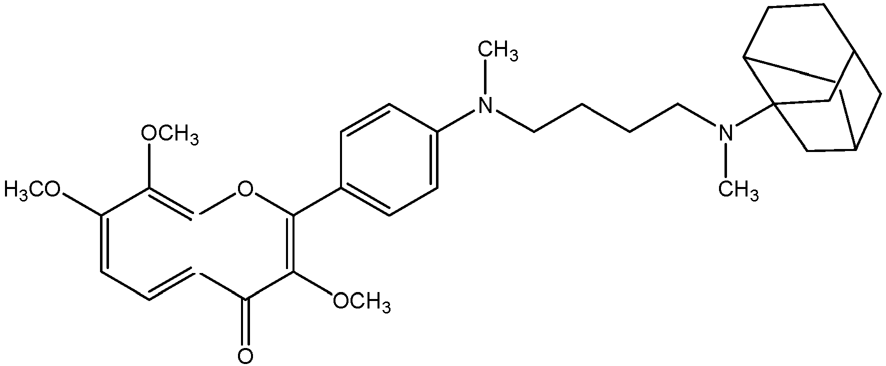 |
| **SND 463** | 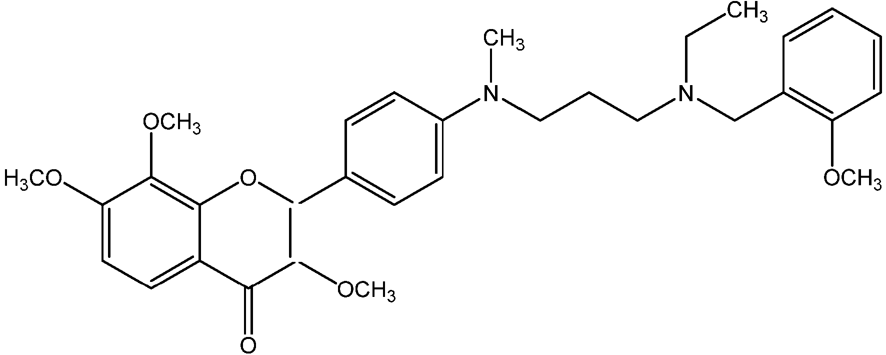 |
| **SND 464** | 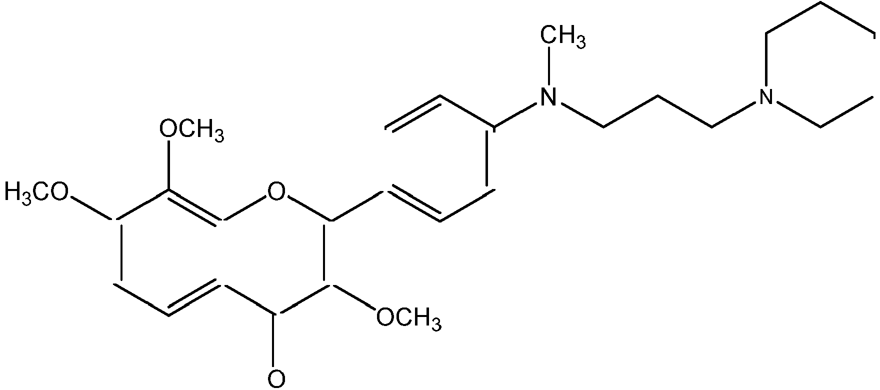 |
| **SND 465** | 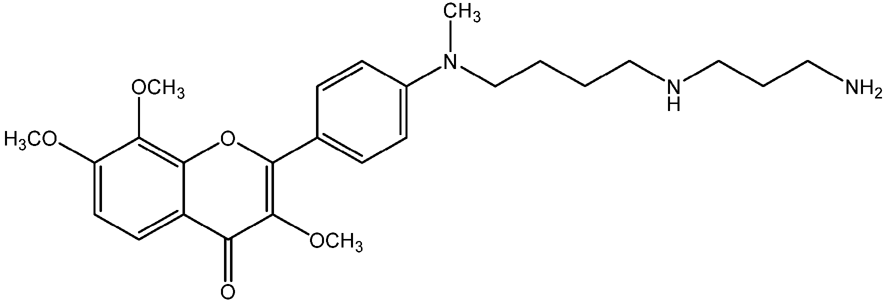 |

The disclosure provides a pharmaceutically acceptable salt, multi-salt, or solvate of the compound of the disclosure.

The compounds of the present disclosure can be used both in their quaternary salt form (as a single salt). Additionally, the compounds of the present disclosure may contain one or more (e.g. one or two) acid addition or alkali addition salts to form a multi-salt. A multi-salt includes a quaternary salt group as well as a salt of a different group of the compound of the disclsoure.

For the purposes of this disclosure, a "multi-salt" of a compound of the present disclosure includes an acid addition salt. Acid addition salts are preferably pharmaceutically acceptable, non-toxic addition salts with suitable acids, including but not limited to inorganic acids such as hydrohalogenic acids (for example, hydrofluoric, hydrochloric, hydrobromic or hydroiodic acid) or other inorganic acids (for example, nitric, perchloric, sulfuric or phosphoric acid); or organic acids such as organic carboxylic acids (for example, propionic, butyric, glycolic, lactic, mandelic, citric, acetic, benzoic, salicylic, succinic, malic or hydroxysuccinic, tartaric, fumaric, maleic, hydroxymaleic, mucic or galactaric, gluconic, pantothenic or pamoic acid), organic sulfonic acids (for example, methanesulfonic, trifluoromethanesulfonic, ethanesulfonic, 2-hydroxyethanesulfonic, benzenesulfonic, toluene-p-sulfonic, naphthalene-2-sulfonic or camphorsulfonic acid) or amino acids (for example, ornithinic, glutamic or aspartic acid). The acid addition salt may be a mono-, di-, tri- or multi-acid addition salt. A preferred salt is a hydrohalogenic, sulfuric, phosphoric or organic acid addition salt. A preferred salt is a hydrochloric acid addition salt.

The compounds of the present disclosure can be used both, in quaternary salt form and their multi-salt form. For the purposes of this disclosure, a "multi-salt" of a compound of the present disclosure includes one formed between a protic acid functionality (such as a carboxylic acid group) of a compound of the present disclosure and a suitable cation. Suitable cations include, but are not limited to lithium, sodium, potassium, magnesium, calcium and ammonium. The salt may be a mono-, di-, tri- or multi-salt. Preferably the salt is a mono- or di-lithium, sodium, potassium, magnesium, calcium or ammonium salt. More preferably the salt is a mono- or di-sodium salt or a mono- or dipotassium salt.

Preferably any multi-salt is a pharmaceutically acceptable non-toxic salt. However, in addition to pharmaceutically acceptable multi-salts, other salts are included in the present disclosure, since they have potential to serve as intermediates in the purification or preparation of other, for example, pharmaceutically acceptable salts, or are useful for identification, characterisation or purification of the free acid or base.

The compounds and/or multi-salts of the present disclosure may be anhydrous or in the form of a hydrate (e.g. a hemihydrate, monohydrate, dihydrate or trihydrate) or other solvate. Such solvates may be formed with common organic solvents, including but not limited to, alcoholic solvents e.g. methanol, ethanol or isopropanol.

The compounds, multi-salts, and solvates of the present disclosure may contain at least one chiral centre. The compounds, multi-salts, and solvates may therefore exist in at least two isomeric forms. The present disclosure encompasses racemic mixtures of the compounds, multi-salts, and solvates of the present disclosure as well as enantiomerically enriched and substantially enantiomerically pure isomers. For the purposes of this disclosure, a "substantially enantiomerically pure" isomer of a compound comprises less than 5% of other isomers of the same compound, more typically less than 2%, and most typically less than 0.5% by weight.

The compounds, multi-salts, and solvates of the present disclosure may contain any stable isotope including, but not limited to ¹²C, ¹³C, ¹H, ²H (D), ¹⁴N, ¹⁵N, ¹⁶O, ¹⁷O, ¹⁸O, ¹⁹F and ¹²⁷I, and any radioisotope including, but not limited to ¹¹C, ¹⁴C, ³H (T), ¹³N, ¹⁵O, ¹⁸F, ¹²³I, ¹²⁴I, ¹²⁵I and ¹³¹I.

The compounds, multi-salts, and solvates of the present disclosure may be in any polymorphic or amorphous form.

The disclosure provides a pharmaceutical composition comprising a compound, or a pharmaceutically acceptable salt, multi-salt, or solvate of the disclosure, and a pharmaceutically acceptable excipient.

Conventional procedures for the selection and preparation of suitable pharmaceutical formulations are described in, for example, "Aulton's Pharmaceutics - The Design and Manufacture of Medicines", M. E. Aulton and K. M. G. Taylor, Churchill Livingstone Elsevier, 4th Ed., 2013.

Pharmaceutically acceptable excipients including adjuvants, diluents or carriers that may be used in the pharmaceutical compositions of the disclosure are those conventionally employed in the field of pharmaceutical formulation, and include, but are not limited to, sugars, sugar alcohols, starches, ion exchangers, alumina, aluminium stearate, lecithin, serum proteins such as human serum albumin, buffer substances such as phosphates, glycerine, sorbic acid, potassium sorbate, partial glyceride mixtures of saturated vegetable fatty acids, water, salts or electrolytes such as protamine sulfate, disodium hydrogen phosphate, potassium hydrogen phosphate, sodium chloride, zinc salts, colloidal silica, magnesium trisilicate, polyvinylpyrrolidone, cellulose-based substances, polyethylene glycol, sodium carboxymethylcellulose, polyacrylates, waxes, polyethylene-polyoxypropylene-block polymers, polyethylene glycol and wool fat.

The disclosure provides a compound, or a pharmaceutically acceptable salt, multi-salt, or solvate , or a pharmaceutical composition of the disclosure, for use in medicine, and/or for use in the treatment or prevention of a disease, disorder or condition. Typically the use comprises the administration of the compound, salt, multi-salt, solvate or pharmaceutical composition to a subject.

For example, the disease, disorder or condition is cancer.

The term "treatment" as used herein refers equally to curative therapy, and ameliorating or palliative therapy. The term includes obtaining beneficial or desired physiological results, which may or may not be established clinically. Beneficial or desired clinical results include, but are not limited to, the alleviation of symptoms, the prevention of symptoms, the diminishment of extent of disease, the stabilisation (i.e., not worsening) of a condition, the delay or slowing of progression/worsening of a condition/symptoms, the amelioration or palliation of the condition/symptoms, and remission (whether partial or total), whether detectable or undetectable. The term "palliation", and variations thereof, as used herein, means that the extent and/or undesirable manifestations of a physiological condition or symptom are lessened and/or time course of the progression is slowed or lengthened, as compared to not administering a compound, salt, multi-salt, solvate, or pharmaceutical composition of the present disclosure. The term "prevention" as used herein in relation to a disease, disorder or condition, relates to prophylactic or preventative therapy, as well as therapy to reduce the risk of developing the disease, disorder or condition. The term "prevention" includes both the avoidance of occurrence of the disease, disorder or condition, and the delay in onset of the disease, disorder or condition. Any statistically significant avoidance of occurrence, delay in onset or reduction in risk as measured by a controlled clinical trial may be deemed a prevention of the disease, disorder or condition. Subjects amenable to prevention include those at heightened risk of a disease, disorder or condition as identified by genetic or biochemical markers. Typically, the genetic or biochemical markers are appropriate to the disease, disorder or condition under consideration and may include for example, beta-amyloid 42, tau and phosphor-tau.

For example, the disease, disorder or condition is selected from but not limited to: breast cancer, brain cancer, colon cancer, lung cancer, leukaemia, lymphoma, muscle carcinoma, melanoma, renal cancer, prostate cancer and pancreatic cancer. The cancers may include resistant types of such tumors.

For example, the disease, disorder or condition is selected from but not limited to: brain cancer, colon cancer, leukaemia, lung cancer, lymphoma, muscle carcinoma, and melanoma.

For example, the disease, disorder or condition is cancer.

For example the cancer is brain cancer.

For example the cancer is breast cancer.

For example the cancer is colon cancer.

For example the cancer is leukaemia.

For example the cancer is lung cancer.

For example the cancer is lymphoma.

For example the cancer is muscle carcinoma.

For example the cancer is melanoma.

For example the cancer is ovarian cancer.

For example the cancer is pancreatic cancer.

For example the cancer is prostate cancer.

For example the cancer is renal cancer.

Unless stated otherwise, in the disclosure, the subject may be any human or other animal. Typically, the subject is a mammal, more typically a human or a domesticated mammal such as a cow, pig, lamb, goat, horse, cat, dog, etc. Most typically, the subject is a human.

Any of the medicaments employed in the present disclosure can be administered by oral, parental (including intravenous, subcutaneous, intramuscular, intradermal, intratracheal, intraperitoneal, intraarticular, intracranial and epidural), airway (aerosol), rectal, vaginal or topical (including transdermal, buccal, mucosal and sublingual) administration.

Typically, the mode of administration selected is that most appropriate to the disorder or disease to be treated or prevented.

For oral administration, the compounds, salts, multi-salts, or solvates of the present disclosure will generally be provided in the form of tablets, capsules, hard or soft gelatine capsules, caplets, troches or lozenges, as a powder or granules, or as an aqueous solution, suspension or dispersion.

Tablets for oral use may include the active ingredient mixed with pharmaceutically acceptable excipients such as inert diluents, disintegrating agents, binding agents, lubricating agents, sweetening agents, flavouring agents, colouring agents and preservatives. Suitable inert diluents include sodium and calcium carbonate, sodium and calcium phosphate, and lactose. Corn starch and alginic acid are suitable disintegrating agents. Binding agents may include starch and gelatine. The lubricating agent, if present, may be magnesium stearate, stearic acid or talc. If desired, the tablets may be coated with a material, such as glyceryl monostearate or glyceryl distearate, to delay absorption in the gastrointestinal tract. Tablets may also be effervescent and/or dissolving tablets.

Capsules for oral use include hard gelatine capsules in which the active ingredient is mixed with a solid diluent, and soft gelatine capsules wherein the active ingredient is mixed with water or an oil such as peanut oil, liquid paraffin or olive oil.

Powders or granules for oral use may be provided in sachets or tubs. Aqueous solutions, suspensions or dispersions may be prepared by the addition of water to powders, granules or tablets.

Any form suitable for oral administration may optionally include sweetening agents such as sugar, flavouring agents, colouring agents and/or preservatives.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

For parenteral use, the compounds, salts, multi-salts, or solvates of the present disclosure will generally be provided in a sterile aqueous solution or suspension, buffered to an appropriate pH and isotonicity. Suitable aqueous vehicles include Ringer's solution and isotonic sodium chloride or glucose. Aqueous suspensions according to the disclosure may include suspending agents such as cellulose derivatives, sodium alginate, polyvinylpyrrolidone and gum tragacanth, and a wetting agent such as lecithin. Suitable preservatives for aqueous suspensions include ethyl and n-propyl p-hydroxybenzoate. The compounds of the disclosure may also be presented as liposome formulations.

For transdermal and other topical administration, the compounds, salts, multi-salts, or solvates of the disclosure will generally be provided in the form of ointments, cataplasms (poultices), pastes, powders, dressings, creams, plasters or patches.

Suitable suspensions and solutions can be used in inhalers for airway (aerosol) administration.

The dose of the compounds, salts, multi-salts, or solvates of the present disclosure will, of course, vary with the disorder or disease to be treated or prevented. In general, a suitable dose will be in the range of 0.01 to 500 mg per kilogram body weight of the recipient per day. The desired dose may be presented at an appropriate interval such as once every other day, once a day, twice a day, three times a day or four times a day. The desired dose may be administered in unit dosage form, for example, containing 1 mg to 50 g of active ingredient per unit dosage form.

For the avoidance of doubt, insofar as is practicable any embodiment of a given aspect of the present invention may occur in combination with any other embodiment of the same aspect of the present invention. In addition, insofar as is practicable it is to be understood that any preferred, typical or optional embodiment of any aspect of the present invention should also be considered as a preferred, typical or optional embodiment of any other aspect of the present invention.

### EXAMPLES

### Synthesis of Compound 006-004 (SND 448)

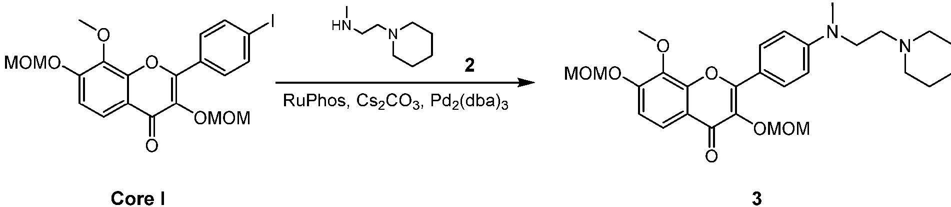

To a solution of **Core I** (300 mg, 602.09 umol, 1.00 eq), **Compound 2**(85.64 mg, 602.09 umol, 1.00 eq) in DMF (3 mL) was added Cs₂CO₃ (588.52 mg, 1.81 mmol, 3.00 eq), RuPhos (28.10 mg, 60.21 umol, 0.10 eq), Pd₂(dba)₃ (55.13 mg, 60.21 umol, 0.10 eq), and the mixture was stirred at 100 °C for 16h under N₂. LCMS showed **Core I** was consumed and desired mass was detected. The reaction mixture was filtered and concentrated under reduced pressure to give a residue. **Compound 3**(300 mg, crude) was obtained as a red oil.

**LCMS** MS (ESI) Retention time: 0.714 min, (M+1) ⁺ = 513.3.

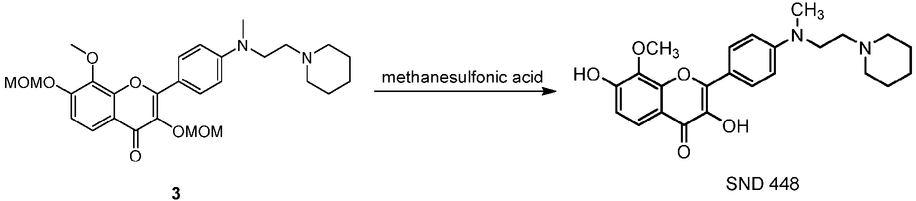

To a solution of **Compound 3** (300 mg, 585.26 umol, 1.00 eq) in DCM (0.3 mL) and EtOH (2.1 mL) was added methanesulfonic acid (674.96 mg, 7.02 mmol, 499.97 uL, 12.0 eq) , and the mixture was stirred at 60 °C or 1 h. LCMS showed **Compound 3** was consumed and desired mass was detected. The reaction mixture was concentrated under reduced pressure to give a residue. The crude product was purified by reversed-phase HPLC (0.1% HCl condition). **Compound 006-004 (SND 448)** (180 mg, 382.69 umol, 65.39% yield, 98% purity, HCl) was obtained as a yellow solid, which was confirmed by LCMS and HNMR.

**LCMS:** MS (ESI) Retention time: 0.679 min, (M+1) ⁺ =425.2.

**LCMS:** MS (ESI) Retention time: 1.127 min, (M+1) ⁺ =425.1.

**¹H NMR** (400 MHz, DMSO-d6) δ = 11.03 (s, 1H), 8.10-8.08 (d, J = 9.2 Hz, 2H), 7.66-7.64 (d, J = 8.8 Hz, 1H), 7.04-7.00 (t, J = 9.2 Hz, 3H), 3.92 (s, 5H), 3.49-3.46 ( d, J = 11.2 Hz, 2H), 3.17-3.16 (d, J = 2.8 Hz, 2H), 3.02 (s, 3H), 2.95-2.88 (m, 2H), 1.85-1.69 (m, 5H), 1.38-1.35 (m, 1H).

**TLC** (SiO2, DCM/MeOH=10/1, Rf= 0.4).

### Synthesis of Compound 006-005 (SND 440)

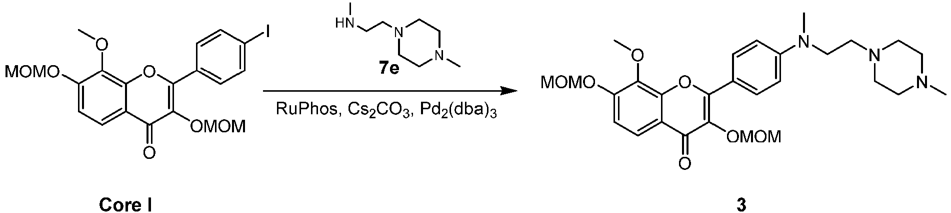

To a solution of **Compound 7e** (94.68 mg, 602.09 µmol, 5.72 µL, 1.00 **eq**) and **Core I** (300 mg, 602.09 µmol, 1.00 **eq**) in DMF (3 mL) was added RuPhos (56.19 mg, 120.42 µmol, 0.20 **eq**), Cs₂CO₃ (588.52 mg, 1.81 mmol, 3.00 **eq**) and Pd₂ (dba)₃ (110.27 mg, 120.42 µmol, 0.20 **eq**) at 25 °C. The mixture was stirred at 100 °C for 12 hr under N₂. LCMS showed **Core I** was consumed. Several new peaks were shown on LCMS and 51.233% of desired compound was detected. The reaction mixture was filtered and concentrated under reduced pressure to give a residue. Without purification. It was put in the next reaction. **Compound 3** (300 mg, 568.60 µmol, 94.44% yield) was obtained as a red oil.

**LCMS:** MS (ESI) Retention time: 0.691min (M+H)⁺ = 528.4.

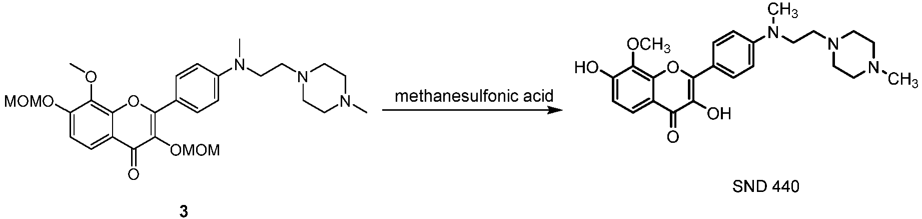

To a solution of **Compound 3** (290 mg, 549.65 µmol, 1.00 *eq*) in EtOH (0.7 mL) and DCM (0.1 mL) was added methanesulfonic acid (633.92 mg, 6.60 mmol, 469.57 µL, 12 **eq**). The mixture was stirred at 60 °C for 1 hr. LCMS showed **Compound 3** was consumed completely and 62.352% of desired mass was detected. The reaction mixture was concentrated under reduced pressure to give a residue. The crude product was purified by re-crystallization from MeOH (60 mL) at 25 °C. **Compound 006-005** (SND **440**) (239.42 mg, 533.20 µmol, 97.01% yield, 97.88% purity) was obtained as a yellow solid, which was confirmed by HNMR, Melting Point and LCMS.

**TLC** (SiO₂, DCM: MeOH = 10/1, Rf = 0.1) and Melting Point = 261.3 °C.

**LCMS:** MS (ESI) Retention time: 0.656min (M+H)⁺= 440.2.

**LCMS:** MS (ESI) Retention time: 1.380min (M+H)⁺ = 440.2.

**¹H NMR** (400 MHz, DMSO-d₆) δ = 8.09-8.07 (d, *J =* 9.2 Hz, 2H), 7.68-7.65 (d, *J =* 8.8 Hz, 1H), 7.00-6.98 (d, *J =* 8.8 Hz, 1H), 6.93-6.91 (d, *J =* 9.2 Hz, 2H), 3.72-3.69 (t, *J =* 7.0 Hz, 2H), 3.42-3.29 (m, 6H), 3.13-3.12 (t, *J =* 6.5 Hz, 2H), 3.00 (s, 3H), 2.84 (s, 3H), 2.43 (s, 5H).

### Synthesis of Compound 006-006 (SND 447)

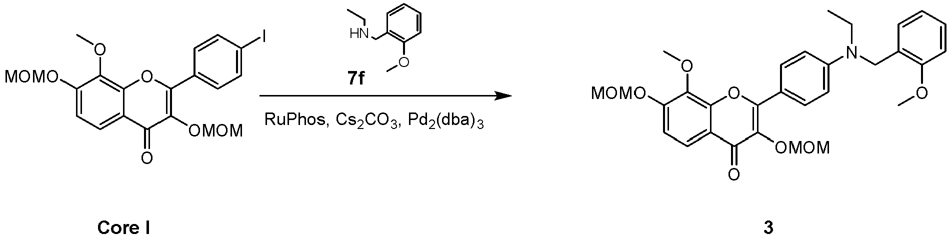

To a solution of **Compound 7f** (99.48 mg, 602.09 µmol, 5.72 µL, 1.00 **eq**) and **Core I** (300 mg, 602.09 µmol, 1_{∘} 00 eq) in DMF (3 mL) was added RuPhos (56.19 mg, 120.42 µmol, 0.20 **eq**), Cs₂CO₃ (588.52 mg, 1.81 mmol, 3.00 **eq**) and Pd₂ (dba)₃ (110.27 mg, 120.42 µmol, 0.20 **eq**) at 25 °C. And that mixture was degassed and purged with N₂ for 3 times. The mixture was stirred at 100 °C for 12 hr under N₂ atmosphere. LCMS showed **Core I** was consumed. Several new peaks were shown on LCMS and 28.925% of desired compound was detected. The reaction mixture was concentrated under reduced pressure to give a residue. The residue was purified by prep-HPLC (column: Waters Xbridge C18 150×50 mm× 10um;mobile phase: [water (NH₄HCO₃) -ACN];B%: 50%-80%, 10min). **Compound3** (100 mg, 186.71 µmol, 31.01% yield) was obtained as a red oil.

**LCMS:** MS (ESI) Retention time: 1.003min (M+H)⁺= 536.2.

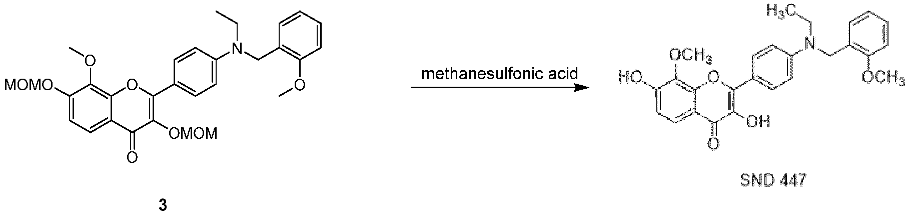

To a solution of **Compound 3** (100 mg, 186.71 µmol, 1 **eq**) in MeOH (1.4 mL) and DCM (0.2 mL) was added methanesulfonic acid (215.33 mg, 2.24 mmol, 159.50 µL, 12 **eq**). The mixture was stirred at 60 °C for 1 hr. LCMS showed **Compound 3** was consumed completely and 90.397% of desired mass was detected. The reaction mixture was concentrated under reduced pressure to give a residue. The residue was purified by prep-HPLC (column: 3_Phenomenex Luna C18; 75 × 30 mm × 3 um; mobile phase: [water (HCl)-ACN]; B%: 51%-71%, 6 min). **Compound 006-006 (SND 447)** (90 mg, 185.97 µmol, 99.60% yield, 100% purity, HCl) was obtained as a yellow oil. Which was determined by HNMR and LCMS.

**TLC** (SiO₂, PE: EA = 0/1, Rf = 0.5).

**LCMS:** MS (ESI) Retention time: 0.960min (M+H)⁺ = 448.2.

**LCMS:** MS (ESI) Retention time: 1.872min (M+H)⁺ = 448.2.

**¹H NMR** (400 MHz, DMSO-d₆) δ = 8.03-8.00 (d, *J =* 9.2 Hz, 2H), 7.66-7.63 (d, *J =* 8.8 Hz, 1H), 7.25 (m, 1H), 7.05-7.03 (d, *J =* 8.4 Hz, 1H), 6.98-6.95 (m, 2H), 6.88-6.86 (m, 1H), 6.78-6.76 (d, *J =* 8.4 Hz, 2H), 4.55 (s, 2H), 3.92 (s, 3H), 3.86 (s, 3H), 3.58-3.53 (q, *J* = 6.8 Hz, 2H), 1.20-1.17 (t, *J =* 7.2 Hz, 3H).

### Synthesis of Compound 006-007 (SND 441)

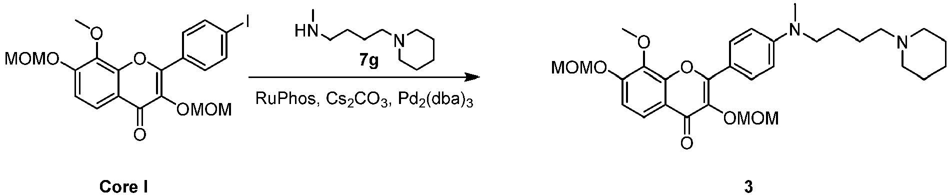

To a solution of **Compound 7g** (102.53 mg, 602.09 µmol, 5.72 µL, 1.00 **eq**) and **Core I (300** mg, 602.09 µmol, 1.00 **eq**) in DMF (1 mL) was added RuPhos (56.19 mg, 120.42 µmol, 0.20 **eq**), Cs₂CO₃ (588.52 mg, 1.81 mmol, 3.00 **eq**) and Pd₂ (dba)₃ (110.27 mg, 120.42 µmol, 0.20 **eq**) at 25 °C. The mixture was stirred at 100 °C for 12 hr. LCMS showed **Core I** was consumed. Several new peaks were shown on LCMS and 48.736% of desired compound was detected. The reaction mixture was filtered and concentrated under reduced pressure to give a residue. Without purification. It was put in the next reaction. **Compound 3** (300 mg, 560.14 µmol, 93.03% yield) was obtained as a red oil.

**LCMS:** MS (ESI) Retention time: 0.740min (M+H)⁺ = 541.4.

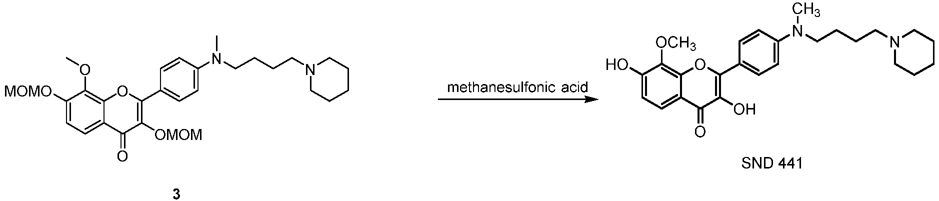

To a solution of **Compound 3** (300 mg, 554.89 µmol, 1.00 **eq**) in EtOH (3.5 mL) and DCM (0.5 mL) was added methanesulfonic acid (639.94 mg, 6.66 mmol, 474.03 µL, 12 **eq**). The mixture was stirred at 60 °C for 0.5 hr. LCMS showed **Compound 3** was consumed completely and 55.545% of desired mass was detected. The reaction mixture was concentrated under reduced pressure to give a residue. The crude product was purified by reversed-phase HPLC (0.1% HCl condition). **Compound 006-007 (SND 441**) (190 mg, 381.94 µmol, 68.83% yield, 98.3% purity, HCl) was obtained as a yellow oil, which was determined by HNMR, LCMS and Melting Point.

**TLC** (SiO₂, DCM: MeOH = 10/1, Rf = 0.4) and Melting Point = 189.4 °C.

**LCMS:** MS (ESI) Retention time: 0.689min (M+H)⁺ = 453.2.

**LCMS:** MS (ESI) Retention time: 1.573min (M+H)⁺ = 453.2.

**¹H NMR** (400 MHz, DMSO-d₆) δ = 10.40 (br s, 1H), 8.11-8.09 (d, *J =* 9.2 Hz, 2H), 7.6-7.64 (d, *J* = 8.8 Hz, 1H), 7.04-7.02 (d, *J* = 8.8 Hz, 3H), 3.92 (s, 3H), 3.48-3.44 (t, *J =* 7.2 Hz, 2H), 3.38-3.35 (d, *J =* 11.6 Hz, 2H), 3.03-2.99 (m, 5H), 2.80-2.78 (m, 2H), 1.76-1.71 (m, 7H), 1.58-1.54 (m, 2H), 1.37 (m, 1H).

### Synthesis of Compound 006-012 (SND 444)

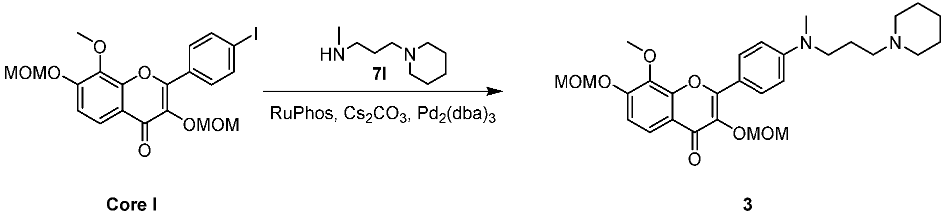

To a solution of **Compound 71** (94.09 mg, 602.09 µmol, 5.72 µL, 1.00 **eq**) and **Core I** (300 mg, 602.09 µmol, 1.00 **eq**) in DMF (3 mL) was added RuPhos (56.19 mg, 120.42 µmol, 0.20 **eq**), Cs₂CO₃ (588.52 mg, 1.81 mmol, 3.00 **eq**) and Pd₂(dba)₃ (110.27 mg, 120.42 µmol, 0.20 **eq**) at 25 °C. The mixture was stirred at 100 °C for 12 hr under N₂. LCMS showed **Core I** consumed. Several new peaks were shown on LCMS and 24.518% of desired compound was detected. The reaction mixture was filtered and concentrated under reduced pressure to give a residue. Without purification. It was put in the next reaction. **Compound** 3 (300 mg, 569.67 µmol, 94.62% yield) was obtained as a red oil.

**LCMS:** MS (ESI) Retention time: 0.735min (M+H)⁺ = 527.4.

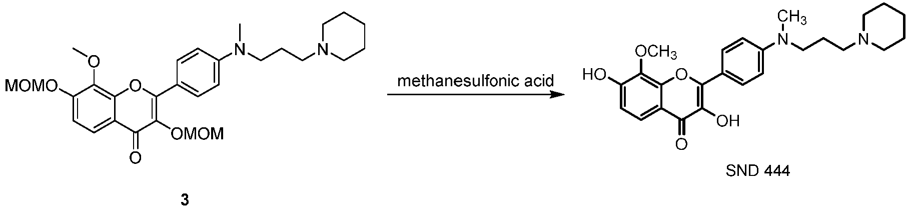

To a solution of **Compound 3** (300 mg, 569.67 µmol, 1.00 **eq**) in MeOH (2.1 mL) and DCM (0.3 mL) was added methanesulfonic acid (54.75 mg, 569.67 µmol, 40.55 µL, 1 **eq**). The mixture was stirred at 60 °C for 1 hr. LCMS showed **Compound 3** was consumed completely and 24.010% of desired mass was detected. The reaction mixture was concentrated under reduced pressure to give a residue. The residue was purified by prep-HPLC (column: 3_Phenomenex Luna C18; 75 × 30 mm × 3 um; mobile phase: [water (HCl) -ACN]; B%: 20%-40%, 6 min). **Compound 006-012 (SND 444)** (40 mg, 84.21 µmol, 14.78% yield, 100% purity, HCl) was obtained as a yellow oil, which was determined by HNMR and LCMS.

**TLC** (SiO₂, DCM: MeOH = 10/1, Rf = 0.1).

**LCMS:** MS (ESI) Retention time: 0.782min (M+H)⁺ = 439.2.

**LCMS:** MS (ESI) Retention time: 1.189min (M+H)⁺ = 439.1.

**¹H NMR** (400 MHz, DMSO-d₆) δ = 8.10-8.08 (d, *J =* 9.2 Hz, 2H), 7.66-7.64 (d, *J =* 8.8 Hz, 1H), 7.02-7.00 (m, 1H), 6.95-6.93 (d, *J* = 8.8 Hz, 2H), 3.92 (s, 3H), 3.50 (m, 2H), 3.40-3.37 (m, 2H), 3.37-3.03 (m, 2H), 3.01 (s, 3H), 2.83-2.81 (m, 2H), 2.02-1.98 (m, 2H), 1.76-1.66 (m, 5), 1.37-1.34 (m, 1H).

### Synthesis of Compound 006-014 (SND 439)

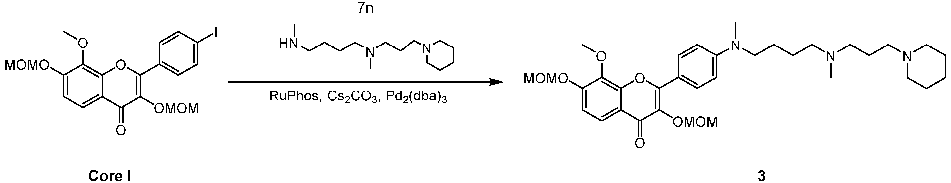

To a solution of **Compound 7n** (145.35 mg, 602.09 µmol, 1.00 **eq**) and **Core I** (300 mg, 602.09 µmol, 1.00 **eq**) in DMF (5 mL) was added RuPhos (56.19 mg, 120.42 µmol, 0.20 **eq**), Cs₂CO₃ (588.52 mg, 1.81 mmol, 3.00 **eq**) and Pd₂(dba)₃ (110.27 mg, 120.42 µmol, 0.20 **eq**) at 25 °C. The mixture was degassed and purged with N₂ for 3 times, and then the mixture was stirred at 100 °C for 12 hr under N₂ atmosphere. LCMS showed **Core I** consumed. Several new peaks were shown on LCMS and 15.143% of desired compound was detected. The reaction mixture was filtered and concentrated under reduced pressure to give a residue. Without purification. It was put in the next reaction. **Compound 3** (300 mg, 490.38 µmol, 81.45% yield) was obtained as a red oil.

**LCMS:** MS (ESI) Retention time: 0.688min (M+H)⁺ = 612.5.

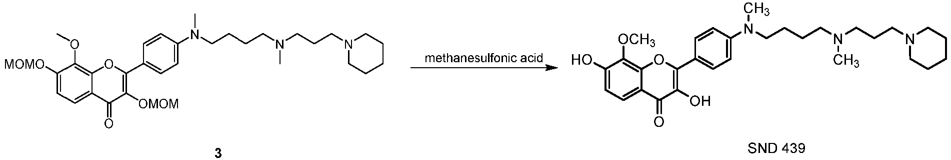

To a solution of **Compound 3** (300 mg, 490.38 µmol, 1 *eq*) in MeOH (2.1 mL) and DCM (0.3 mL) was added methanesulfonic acid (565.54 mg, 5.88 mmol, 418.92 µL, 12 *eq*). The mixture was stirred at 60 °C for 1 hr. LCMS showed **Compound 3** was consumed completely and 19.646% of desired mass was detected. The reaction mixture was concentrated under reduced pressure to give a residue. The residue was purified by prep-HPLC (column: Phenomenex Synergi C18; 150 × 25 mm × 10 um; mobile phase: [water (HCl) -ACN]; B%: 13%-33%, 9 min). **Compound 006-014 (SND 439)** (55 mg, 94.08 µmol, 19.19% yield, 95.816% purity, HCl) was obtained as a yellow solid, which was determined by HNMR and LCMS.

**TLC** (SiO₂, DCM: MeOH = 10/1, Rf = 0.1).

**LCMS:** MS (ESI) Retention time: 0.732min (M+H)⁺ = 524.5.

**LCMS:** MS (ESI) Retention time: 0.657min (M+H)⁺ = 524.5.

**¹H NMR** (400 MHz, DMSO-d₆) δ = 10.83 (br d, *J* = 3.6 Hz, 2H), 8.11-8.09 (d, *J* = 9.2 Hz, 2H), 7.66-7.64 (d, *J =* 8.8 Hz, 1H), 7.05-7.03 (d, *J =* 8.8 Hz, 3H), 3.92 (s, 3H), 3.49-3.46 (t, *J =* 7.2 Hz, 2H), 3.40-3.38 (d, *J =* 11.2 Hz, 2H), 3.27-3.04 (m, 6H), 3.03 (s, 3H), 2.84-2.82 (m, 2H), 2.72-2.71 (m, 3H), 2.20-2.18 (m, 2H), 1.85-1.73 (m, 7H), 1.58 (m, 2H), 1.38 (m, 1H).

### Synthesis of Compound 006-016 (SND 443)

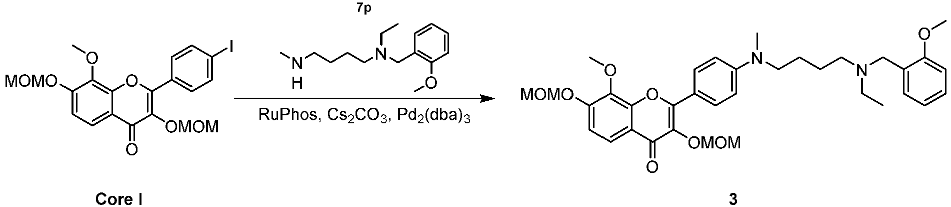

To a solution of **Core I** (300 mg, 602.09 µmol, 1.00 *eq*)*,* RuPhos (56.19 mg, 120.42 µmol, 0.20 *eq*), Pd₂(dba)₃ (110.27 mg, 120.42 µmol, 0.20 *eq*) and Cs₂CO₃ (588.52 mg, 1.81 mmol, 3.00 *eq*) in DMF (3 mL), then added **Compound 7p** (150.75 mg, 602.09 µmol, 1.00 *eq*) at 25 °C, the mixture was strried at 100 °C for 16 hr. LCMS showed **Core I** consumed. Several new peaks were shown on LCMS and 33.183% of desired compound was detected. The reaction mixture was filtered and concentrated under reduced pressure to give a residue. Without purification. It was put in the next reaction. **Compound 3** (300 mg, 483.30 µmol, 80.27% yield) was obtained as a red oil.

**LCMS:** MS (ESI) Retention time: 0.776min (M+H)⁺ = 621.4.

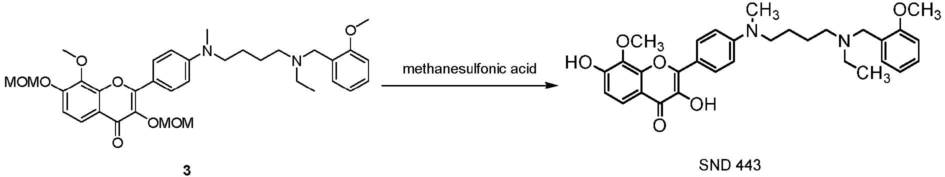

To a solution of **Compound 3** (300 mg, 483.30 µmol, 1.00 **eq**) in MeOH (2.1 mL) and DCM (0.3 mL) was added methanesulfonic acid (557.38 mg, 5.80 mmol, 412.87 µL, 12 **eq**). The mixture was stirred at 60 °C for 0.5 hr. LCMS showed **Compound 3** was consumed completely and 41.981% of desired mass was detected. The reaction mixture was concentrated under reduced pressure to give a residue. The crude product was purified by reversed-phase HPLC (0.1% HCl condition). **Compound 006-016 (SND 443**) (155 mg, 258.85 µmol, 53.56% yield, 95.036% purity, HCl) was obtained as a yellow oil, which was determined by HNMR and LCMS.

**TLC** (SiO₂, DCM: MeOH = 10/1, Rf = 0.1).

**LCMS:** MS (ESI) Retention time: 0.830min (M+H)⁺= 533.4.

**LCMS:** MS (ESI) Retention time: 1.755min (M+H)⁺ = 533.3.

**¹H NMR** (400 MHz, DMSO-d₆) δ = 9.82 (br s, 1H), 8.11-8.09 (d, *J =* 9.2 Hz, 2H), 7.67-7.64 (d, *J =* 8.8 Hz, 1H), 7.57 (m, 1H), 7.46-7.44 (m, 1H), 7.11-7.09 (d, *J =* 8.4 Hz, 1H), 7.04-6.99 (m, 4H), 4.24-4.22 (t, *J* = 4.4 Hz, 2H), 3.92 (s, 3H), 3.82 (s, 3H), 3.46 (m, 2H), 3.01 (s, 7H), 1.79-1.73 (m, 2H), 1.56-1.53 (m, 2H), 1.28-1.25 (t, *J =* 7.2 Hz, 3H).

### Synthesis of Compound 006-017 (SND 438)

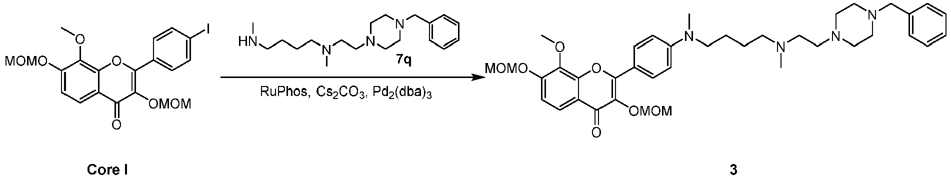

To a solution of **Core I** (300 mg, 602.09 µmol, 1.00 **eq**), RuPhos (56.19 mg, 120.42 µmol, 0.20 **eq**), Pd₂(dba)₃ (110.27 mg, 120.42 µmol, 0.20 **eq**) and Cs₂CO₃ (588.52 mg, 1.81 mmol, 3.00 **eq**) in DMF (5 mL), then added **Compound 7q** (191.77 mg, 602.09 µmol, 1.00 **eq**) at 25 °C, that mixture was degassed and purged with N₂ for 3 times, the mixture was strried at 100 °C for 12 hr under N₂ atmosphere. LCMS showed **Core I** consumed. Several new peaks were shown on LCMS and 44.988% of desired compound was detected. The reaction mixture was filtered and concentrated under reduced pressure to give a residue. Without purification. It was put in the next reaction. **Compound3** (300 mg, 435.51 µmol, 72.33% yield) was obtained as a red oil. **LCMS:** MS (ESI) Retention time: 0.686min (M+H)⁺ = 689.6.

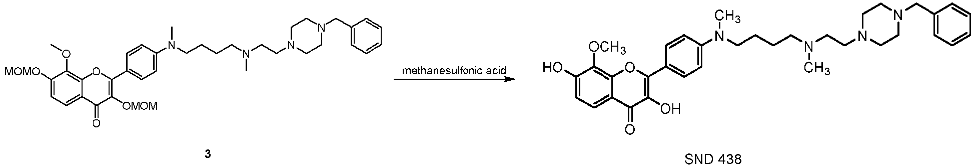

To a solution of **Compound 3** (300 mg, 435.51 µmol, 1.00 **eq**) in MeOH (4.2 mL) and DCM (0.6 mL) was added methanesulfonic acid (502.26 mg, 5.23 mmol, 372.04 µL, 12 **eq**). The mixture was stirred at 60 °C for 1 hr. LCMS showed **Compound 3** was consumed completely and 48.006% of desired mass was detected. The reaction mixture was concentrated under reduced pressure to give a residue. The crude product was purified by reversed-phase HPLC (0.1% HCl condition). **Compound 006-017 (SND 438)** (215 mg, 310.89 µmol, 71.39% yield, 92.140% purity, HCl) was obtained as a yellow solid, which was determined by HNMR and LCMS.

**TLC** (SiO₂, DCM: MeOH = 10/1, Rf = 0.2).

**LCMS:** MS (ESI) Retention time: 0.676min (M+H)⁺= 601.4.

**LCMS:** MS (ESI) Retention time: 1.507min (M+H)⁺ = 601.4.

**¹H NMR** (400 MHz, DMSO-d₆) δ = 10.74 (br s, 1H), 8.10-8.08 (d, *J* = 9.2 Hz, 2H), 7.67-7.64 (m, 3H), 7.46-7.44 (m, 3H), 7.03-7.00 (d, *J =* 8.8 Hz, 1H), 6.96 (m, 2H), 4.39 (s, 2H), 3.92 (s, 3H), 3.66-3.59 (m, 2H), 3.46 (m, 8H), 3.36-3.32 (m, 4H), 3.14 (m, 2H), 3.02 (s, 3H), 2.78 (s, 3H), 1.75-1.73 (m, 2H), 1.59-1.57 (m, 2H).

### Synthesis of Compound 006-018 (SND 437)

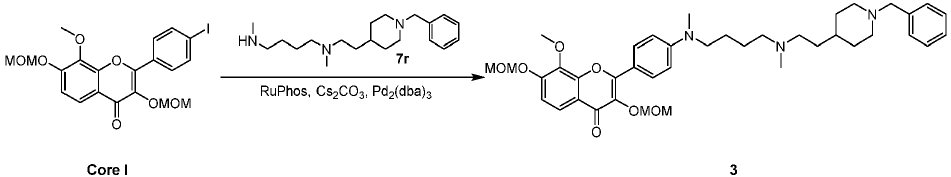

To a solution of **Core I** (300 mg, 602.09 µmol, 1.00 **eq**), RuPhos (56.19 mg, 120.42 µmol, 0.20 **eq**), Pd₂(dba)₃ (110.27 mg, 120.42 µmol, 0.20 **eq**) and Cs₂CO₃ (588.52 mg, 1.81 mmol, 3.00 **eq**) in DMF (5 mL), then added **Compound 7r** (191.17 mg, 602.09 µmol, 1.00 **eq**) at 25 °C, that mixture was degassed and purged with N₂ for 3 times, the mixture was strried at 100 °C for 12 hr under N₂ atmosphere. LCMS showed **Core I** consumed. Several new peaks were shown on LCMS and 41.616% of desired compound was detected. The reaction mixture was filtered and concentrated under reduced pressure to give a residue. Without purification. It was put in the next reaction. **Compound 3** (300 mg, 436.13 µmol, 72.44% yield) was obtained as a red oil.

**LCMS:** MS (ESI) Retention time: 0.796min (M+H)⁺= 688.6.

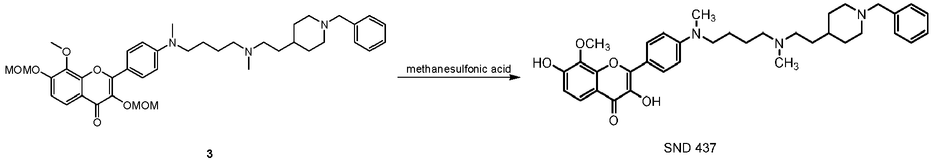

To a solution of **Compound 3** (299.57 mg, 435.51 µmol, 1.00 **eq**) in MeOH (4.2 mL) and DCM (0.6 mL) was added methanesulfonic acid (502.26 mg, 5.23 mmol, 372.04 µL, 12 **eq**). The mixture was stirred at 60 °C for 1 hr. LCMS showed **Compound 3** was consumed completely and 51.445% of desired mass was detected. The reaction mixture was concentrated under reduced pressure to give a residue. The crude product was purified by reversed-phase HPLC (0.1% HCl condition). **Compound 006-018 (SND 437)** (190 mg, 289.56 µmol, 66.49% yield, 96.960% purity, HCl) was obtained as a yellow solid, which was determined by HNMR and LCMS.

**TLC** (SiO₂, DCM: MeOH = 10/1, Rf = 0.2).

**LCMS:** MS (ESI) Retention time: 0.686min (M+H)⁺ = 600.5.

**LCMS:** MS (ESI) Retention time: 1.523min (M+H)⁺ = 600.4.

**¹H NMR** (400 MHz, DMSO-d₆) δ = 11.03 (br s, 1H), 10.62 (br s, 1H), 8.10-8.08 (d, *J =* 9.2 Hz, 2H), 7.66-7.62 (m, 3H),7.44-7.42 (m, 3H), 7.04-7.01 (m, 3H), 4.23-4.21 (d, *J =* 5.2 Hz, 2H), 3.92 (s, 3H), 3.70 (m, 2H), 3.47-3.45 (m, 2H), 3.28-3.25 (m, 2H), 3.06-2.98 (m, 8H), 2.89-2.75 (m, 2H), 2.67-2.66 (m, 3H), 1.81-1.72 (m, 4H), 1.60-1.55 (m, 7H).

### Synthesis of Compound 006-019 (SND 445)

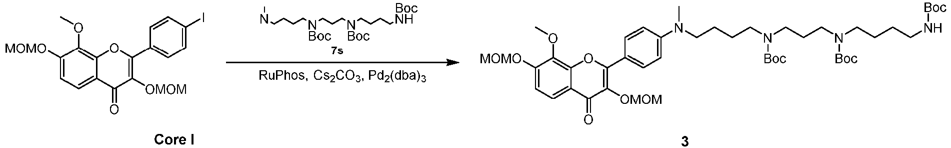

To a solution of **Compound 7s** (200 mg, 401.39 µmol, 1.00 **eq**), RuPhos (37.46 mg, 80.28 µmol, 0.20 **eq**), Pd₂(dba)₃ (73.51 mg, 80.28 µmol, 0.20 **eq**) and Cs₂CO₃ (392.35 mg, 1.20 mmol, 3.00 **eq**) in DMF (1 mL), then added **Core I** (213.04 mg, 401.39 µmol, 1.00 **eq**) at 25 °C, the mixture was stirred at 100 °C for 16 hr. LCMS showed **Core I** was consumed. Several new peaks were shown on LCMS and 47.340% of desired compound was detected. The reaction mixture was filtered and concentrated under reduced pressure to give a residue. Without purification. It was put in the next reaction. **Compound 3** (300 mg, 332.93 µmol, 82.94% yield) was obtained as a red oil.

**LCMS:** MS (ESI) Retention time: 1.157min (M+H)⁺ = 901.7.

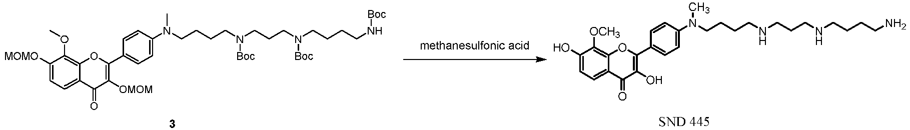

To a solution of **Compound 3** (300 mg, 332.93 µmol, 1.00 **eq**) in MeOH (2.1 mL) and DCM (0.3 mL) was added methanesulfonic acid (383.96 mg, 4.00 mmol, 284.41 µL, 12 **eq**). The mixture was stirred at 60 °C for 1 hr. LCMS showed **Compound 3** was consumed completely and 47.286% of desired mass was detected. The reaction mixture was concentrated under reduced pressure to give a residue. The crude product was purified by reversed-phase HPLC (0.1% HCl condition). **Compound 006-019 (SND 445)** (135 mg, 245.86 µmol, 73.85% yield, 100% purity, HCl) was obtained as a yellow oil, which was determined by HNMR and LCMS.

**TLC** (SiO₂, DCM: MeOH = 10/1, Rf = 0.05).

**LCMS:** MS (ESI) Retention time: 0.710min (M+H)⁺ = 513.4.

**LCMS:** MS (ESI) Retention time: 0.962min (M+H)⁺ = 513.2.

**¹H NMR** (400 MHz, DMSO-d₆) δ = 8.10-8.08 (d, *J =* 8.8 Hz, 2H), 7.66-7.64 (d, *J =* 8.8 Hz, 1H), 7.07-7.06 (m, 2H), 7.01-6.99 (d, *J* = 8.8 Hz, 1H), 3.90 (s, 3H), 3.48-3.46 (m, 2H), 3.02 (s, 3H), 2.99-2.96 (t, *J* = 7.6 Hz, 4H), 2.9-2.89 (m, 4H), 2.79 (m, 2H), 2.02-1.99 (m, 2H), 1.64-1.60 (m, 8H).

### Synthesis of Compound 006-020 (SND 446)

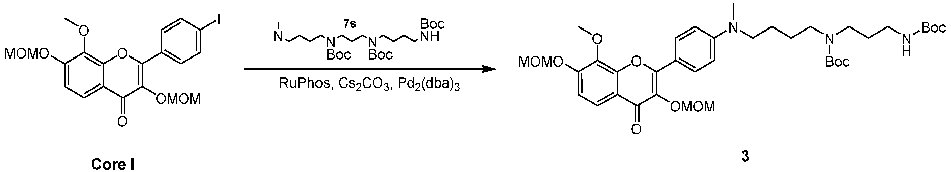

To a solution of **Compound 7s** (208.01 mg, 602.09 µmol, 1.00 **eq**) and **Core I** (300 mg, 602.09 µmol, 1.00 **eq**) in DMF (3 mL) was added RuPhos (56.19 mg, 120.42 µmol, 0.20 **eq**), Cs₂CO₃ (588.52 mg, 1.81 mmol, 3.00 **eq**) and Pd₂(dba)₃ (110.27 mg, 120.42 µmol, 0.20 **eq**) at 25 °C. The mixture was stirred at 100 °C for 12 hr under N₂. LCMS showed **Core I** was consumed completely and 40.463% of desired mass was detected. The reaction mixture was filtered and concentrated under reduced pressure to give a residue. Without purification. It was put in the next reaction. **Compound 3** (300 mg, 419.10 µmol, 69.61% yield) was obtained as a red oil.

**LCMS:** MS (ESI) Retention time: 1.056min (M+H)⁺ = 716.5.

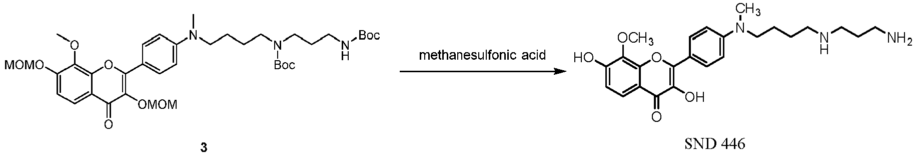

To a solution of **Compound 3** (300 mg, 419.10 µmol, 1.00 **eq**) in MeOH (2.1 mL) and DCM (0.3 mL) was added methanesulfonic acid (483.33 mg, 5.03 mmol, 358.02 µL, 12 **eq**). The mixture was stirred at 60 °C for 1 hr. LCMS showed **Compound 3** was consumed completely and 28.727% of desired mass was detected. The reaction mixture was concentrated under reduced pressure to give a residue. The crude product was purified by reversed-phase HPLC (0.1% HCl condition). **Compound 006-020 (SND 446)** (105 mg, 216.61 µmol, 51.69% yield, 95.712% purity, HCl) was obtained as a yellow solid, which was determined by HNMR and LCMS.

**TLC** (SiO₂, DCM: MeOH = 10/1, Rf = 0.05).

**LCMS:** MS (ESI) Retention time: 0.699min (M+H)⁺ = 428.3.

**LCMS:** MS (ESI) Retention time: 1.283min (M+H)⁺ = 428.2.

**¹H NMR** (400 MHz, DMSO-d₆) δ = 8.02-7.80 (d, *J =* 8.8 Hz, 2H), 7.67-7.65 (d, *J =* 8.8 Hz, 1H), 7.00-6.98 (d, *J =* 8.8 Hz, 1H), 6.83-6.80 (d, *J =* 8.8 Hz, 2H), 3.91 (s, 3H), 3.17-3.15 (m, 2H), 2.99-2.86 (m, 6H), 1.94-1.90 (m, 2H), 1.71-1.63 (m, 4H).

### Synthesis of Compound 006-021 (SND 449)

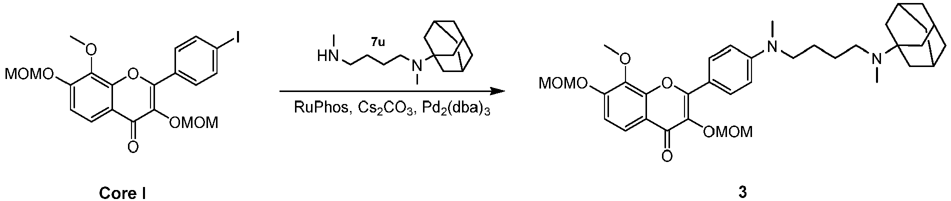

To a solution of **Core I** (300 mg, 619.53 µmol, 1.00 **eq**), RuPhos (57.82 mg, 123.91 µmol, 0.20 **eq**), Pd₂(dba)₃ (113.46 mg, 123.91 µmol, 0.20 **eq**) and Cs₂CO₃ (605.57 mg, 1.86 mmol, 3.00 **eq**) in DMF (1 mL), then added **Compound 7n** (155.14 mg, 619.53 µmol, 1.00 **eq**) at 25 °C, the mixture was stirred at 100 °C for 16 hr under N₂. LCMS showed **Core I** consumed. Several new peaks were shown on LCMS and 44.365% of desired compound was detected. The reaction mixture was filtered and concentrated under reduced pressure to give a residue. Without purification. It was put in the next reaction. **Compound 3** (300 mg, 483.27 µmol, 78.01% yield) was obtained as a red oil.

**LCMS:** MS (ESI) Retention time: 0.861min (M+H)⁺ = 621.5.

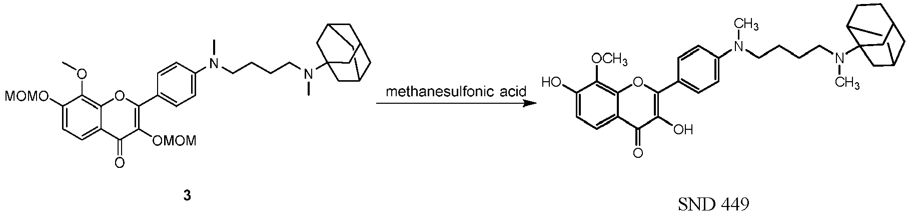

To a solution of **Compound 3** (300 mg, 483.27 µmol, 1.00 **eq**) in MeOH (1.4 mL) and HCl (0.2 mL) was added methanesulfonic acid (557.34 mg, 5.80 mmol, 412.84 µL, 12 *eq*). The mixture was stirred at 60 °C for 1 hr. LCMS showed **Compound 3** was consumed completely and 42.243% of desired mass was detected. The reaction mixture was concentrated under reduced pressure to give a residue. The crude product was purified by reversed-phase HPLC (0.1% HCl condition). **Compound 006-021 (SND 449)** (145 mg, 254.77 µmol, 52.72% yield, 100% purity, HCl) was obtained as a yellow solid, which was determined by HNMR and LCMS.

**TLC** (SiO₂, DCM: MeOH = 10/1, Rf = 0.05).

**LCMS:** MS (ESI) Retention time: 0.760min (M+H)⁺= 533.4.

**LCMS:** MS (ESI) Retention time: 1.798min (M+H)⁺ = 533.4.

**¹H NMR** (400 MHz, DMSO-d₆) δ = 9.79 (br s, 1H), 8.11-8.08 (d, *J =* 8.8 Hz, 2H), 7.67-7.64 (d, *J =* 8.8 Hz, 1H), 7.03-6.97 (m, 3H), 3.92 (s, 3H), 3.49-3.44 (m, 2H), 3.40-3.32 (m, 1H), 3.02 (s, 3H), 2.74-2.66 (m, 1H), 2.62-2.61 (m, 3H), 2.13 (s, 3H), 1.99-1.96 (m, 3H), 1.91-1.88 (m, 3H), 1.75-1.72 (m, 3H), 1.61 (m, 7H).

### Synthesis of Compound 007-004 (SND 455)

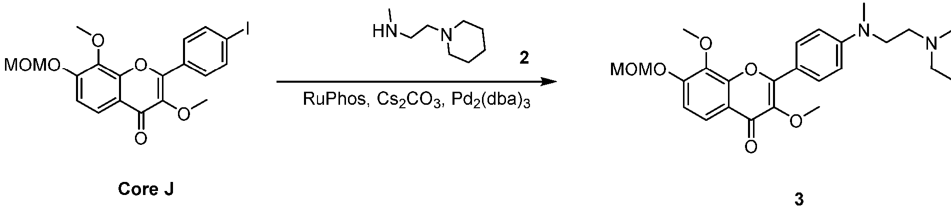

To a solution of **Core J** (300 mg, 640.70 umol, 1.00 eq), **Compound 2** (114.49 mg, 640.70 umol, 1.00 eq, HCl) in DMF (3 mL) was added Cs₂CO₃ (626.26 mg, 1.92 mmol, 3.00 eq), RuPhos (29.90 mg, 64.07 umol, 0.10 eq), Pd₂(dba)₃ (58.67 mg, 64.07 umol, 0.1 eq), and the mixture was stirred at 100 °C for 16 h under N₂. LCMS showed **Core J** was consumed and desired mass was detected. The reaction mixture was filtered and concentrated under reduced pressure to give a residue. **Compound 3** (320 mg, crude) was obtained as a red oil.

**LCMS** MS (ESI) Retention time: 0.721 min, (M+1)⁺ = 483.3.

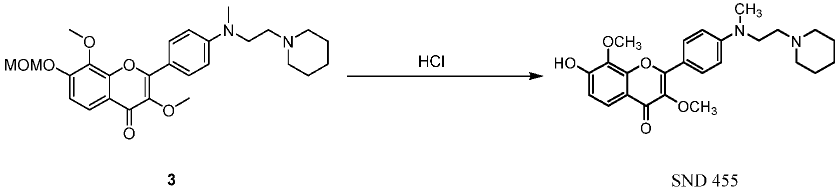

To a solution of **Compound 3** (320 mg, 663.12 umol, 1.00 eq) in dioxane (2 mL) was added HCl/dioxane (4 M, 2 mL, 12.06 eq), and the mixture was stirred at 25 °C for 1 h. LCMS showed **Compound 3** was consumed and desired mass was detected. The reaction mixture was concentrated under pressure to give a residue. The crude product was purified by reversed-phase HPLC (0.1% HCl condition). **Compound 007-004 (SND 455)** (220 mg, 453.92 umol, 68.45% yield, 98% purity, HCl) was obtained as an orange solid, which was confirmed by LCMS and HNMR.

**LCMS:** MS (ESI) Retention time: 0.682 min, (M+1) ⁺ =439.3.

**LCMS:** MS (ESI) Retention time: 1.465 min, (M+1) ⁺ =425.1.

**¹H NMR** (400 MHz, DMSO-d6) δ = 7.99-7.97 (d, J = 8.8 Hz, 2H), 7.64-7.62 (d, J = 8.8 Hz, 1H), 7.01-6.96 (m, 3H), 3.90 (s, 3H), 3.87-3.85 (m, 2H), 3.75 (s, 3H), 3.50-3.48 (d, J = 11.6 hZ, 2H), 3.21-3.17 (m, 2H), 3.03 (s, 3H), 2.97-2.92 (m, 2H), 1.83-1.69 (m, 5H), 1.40-1.37 (m, 1H).

**TLC** (SiO2, DCM/MeOH=10/1, Rf= 0.5).

### Synthesis of Compound 007-005 (SND 450)

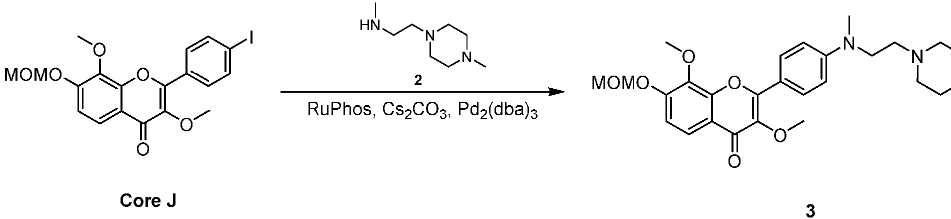

To a solution of **Core J** (300 mg, 640.70 umol, 1.00 eq), **Compound 2** (100.75 mg, 640.70 umol, 1.00 eq) in DMF (4 mL) was added Cs₂CO₃ (626.26 mg, 1.92 mmol, 3.00 eq), RuPhos (29.90 mg, 64.07 umol, 0.10 eq), Pd₂(dba)₃ (58.67 mg, 64.07 umol, 0.10 eq), and the mixture was stirred at 100 °C for 16 h under N₂. LCMS showed **Core J** was consumed and 67% of desired mass was detected. The reaction mixture was filtered and concentrated under reduced pressure to give a residue. **Compound 3** (320 mg, crude) was obtained as a red oil.

**LCMS** MS (ESI) Retention time: 0.689 min, (M+1) ⁺= 498.2.

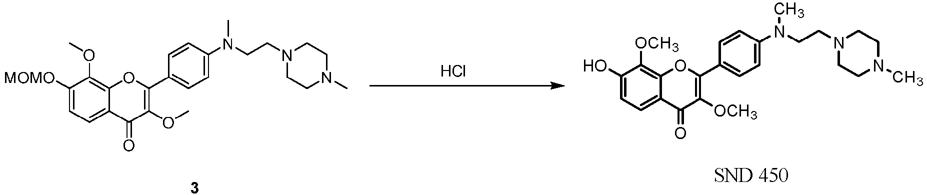

To a solution of **Compound 3** (320 mg, 643.11 umol, 1.00 eq) in dioxane (2 mL) was added HCl/dioxane (4 M, 2 mL, 12.4 eq), and the mixture was stirred at 25 °C for 2 h. LCMS showed **Compound 3** was consumed and desired mass was detected. The reaction mixture was concentrated under reduced pressure to give a residue. The crude product was purified by reversed-phase HPLC (0.1% HCl condition). **Compound 007-005 (SND 450)** (280 mg, 560.01 umol, 87.08% yield, 98% purity, HCl) was obtained as a red solid, which was confirmed by HNMR and LCMS.

**LCMS:** MS (ESI) Retention time: 0.658 min, (M+1) ⁺ =454.2.

**LCMS:** MS (ESI) Retention time: 1.390 min, (M+1) ⁺ =454.2.

**¹H NMR** (400 MHz, DMSO-d6) δ = 12.12-12.06 (br s, 1H), 8.00-7.97 (d, J = 9.2 Hz, 2H), 7.64-7.62 (d, J = 8.8 Hz, 1H), 7.04-7.01 (m, 3H), 3.96-3.92 (m, 2H), 3.91 (s, 3H), 3.81 (m, 2H), 3.77 (s, 3H), 3.72-3.70 (m, 2H), 3.51 (s, 4H), 3.37 (s, 2H), 3.06 (s, 3H), 2.84 (s, 3H).

**TLC** (SiO2, DCM/MeOH=10/1, Rf= 0.5.

### Synthesis of Compound 007-006 (SND 456)

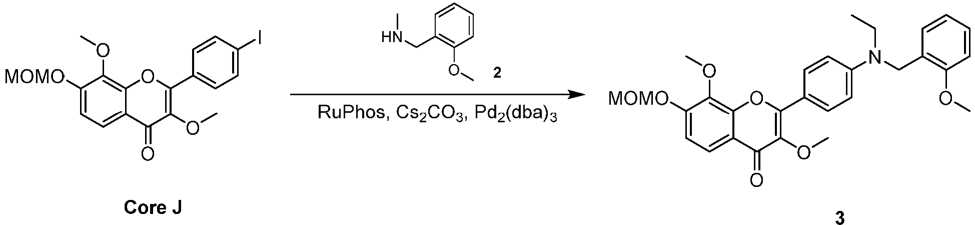

To a solution of **Core J** (300 mg, 640.70 umol, 1.00 eq), **Compound 2** (129.22 mg, 640.70 umol, 1.00 eq, HCl) in DMF (3 mL) was added Cs₂CO₃ (626.26 mg, 1.92 mmol, 3.00 eq), RuPhos (29.90 mg, 64.07 umol, 0.10 eq), Pd₂(dba)₃ (58.67 mg, 64.07 umol, 0.10 eq), and the mixture was stirred at 100 °C for 16 h under N₂. LCMS showed **Core J** was consumed and desired mass was detected. The reaction mixture was filtered and concentrated under pressure to give a residue. The crude product was purified by reversed-phase HPLC (0.1% FA condition). **Compound** 3 (180 mg, crude) was obtained as a yellow oil.

**LCMS** MS (ESI) Retention time: 0.972 min, (M+1) ⁺ = 506.3.

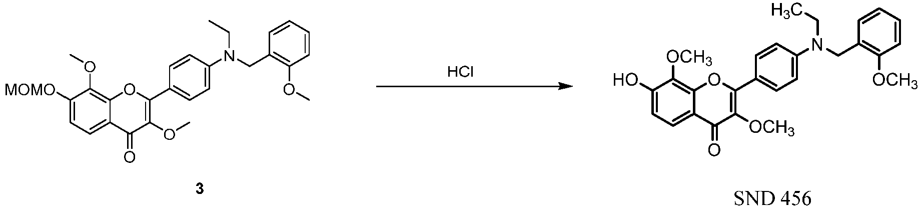

To a solution of **Compound 3** (180 mg, 356.04 umol, 1.00 eq) in dioxane (2 mL) was added HCl/dioxane (4 M, 2 mL, 22.47 eq), and the mixture was stirred at 25 °C for 1 h. LCMS showed **Compound 3** was consumed and desired mass was detected. The reaction mixture was concentrated under reduced pressure to give a residue. The residue was purified by prep-HPLC (column: 3_Phenomenex Luna C18; 75 × 30 mm × 3 um; mobile phase: [water (HCl)-ACN]; B%: 45%-65%, 8 min). **Compound 007-006 (SND 456)** (105 mg, 206.64 umol, 58.04% yield, 98% purity, HCl) was obtained as a yellow solid, which was confirmed by LCM and HNMR.

**LCMS:** MS (ESI) Retention time: 0.896 min, (M+1) ⁺ =462.2.

**LCMS:** MS (ESI) Retention time: 2.305 min, (M+1) ⁺ =462.2.

**1H NMR** (400 MHz, DMSO-d6) δ = 7.94-7.92 (d, J = 9.2 Hz, 2H), 7.63-7.60 ( d, J = 8.8 Hz, 1H), 7.25-7.23 (m, 1H), 7.05-7.00 (m, 3H), 6.98-6.95 (t, J = 7.6 Hz, 1H), 6.87-6.77 (m, 2H), 4.56 ( s, 3H), 3.89 (s, 2H), 3.86 (s, 3H), 3.76 (s, 3H), 3.55-3.54 (m, 2H), 1.21-1.17 (m, 3H).

**TLC** (SiO2, DCM/MeOH=10/1, Rf= 0.3).

### Synthesis of Compound 007-007 (SND 454)

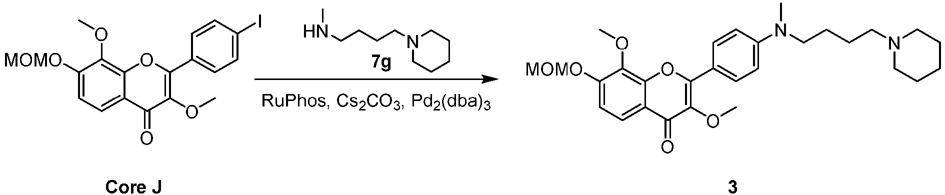

To a solution of **Compound 7g** (109.11 mg, 640.70 µmol, 5.72 µL, 1.00 **eq**) and **Core J** (300 mg, 640.70 µmol, 1.00 **eq**) in DMF (1.00 mL) was added RuPhos (59.80 mg, 128.14 µmol, 0.20 **eq**), Cs₂CO₃ (626.26 mg, 1.92 mmol, 3.00 **eq**) and Pd₂(dba)₃ (117.34 mg, 128.14 µmol, 0.20 **eq**) at 25 °C. The mixture was stirred at 100 °C for 12 hr under N₂. LCMS showed **Core J** was consumed. Several new peaks were shown on LCMS and 65.733% of desired compound was detected. The reaction mixture was filtered and concentrated under reduced pressure to give a residue. Without purification. It was put in the next reaction. **Compound 3** (300 mg, 587.52 µmol, 91.70% yield) was obtained as a red oil.

**LCMS:** MS (ESI) Retention time: 0.819min (M+H)⁺= 511.4.

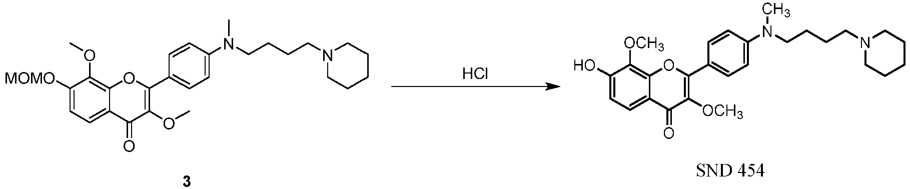

To a solution of **Compound 3** (300 mg, 587.52 µmol, 1.00 **eq**) in HCl/dioxane (8 mL). The mixture was stirred at 25 °C for 1 hr. LCMS showed **Compound 3** consumed. Several new peaks were shown on LCMS and 78.683% of desired compound was detected. The reaction mixture was concentrated under reduced pressure to give a residue. The crude product was purified by reversed-phase HPLC (0.1% HCl condition). **Compound 007-007 (SND 454)** (265 mg, 519.22 µmol, 88.37% yield, 98.559% purity, HCl) was obtained as a yellow solid, which was determined by HNMR and LCMS.

**TLC** (SiO₂, DCM: MeOH = 10/1, Rf = 0.2).

**LCMS:** MS (ESI) Retention time: 0.773min (M+H)⁺ = 467.4.

**LCMS:** MS (ESI) Retention time: 1.575min (M+H)⁺ = 467.3.

**¹H NMR** (400 MHz, DMSO-d₆) δ = 8.00-7.97 (d, *J =* 9.2 Hz, 2H), 7.63-7.61 (d, *J =* 8.8 Hz, 1H), 7.03-7.01 (d, *J =* 8.8 Hz, 1H), 6.93-6.91 (d, *J* = 8.8 Hz, 2H), 3.91 (s, 3H), 3.77 (s, 3H), 3.48-3.45 (t, *J =* 7.2 Hz, 2H), 3.39-3.36 (d, *J =* 11.6 Hz, 2H), 3.03-2.99 (m, 5H), 2.82-2.80 (m, 2H), 1.80-1.75 (m, 7H), 1.71-1.67 (m, 2H), 1.59-1.57 (m, 1H).

### Synthesis of Compound 007-012 (SND 451)

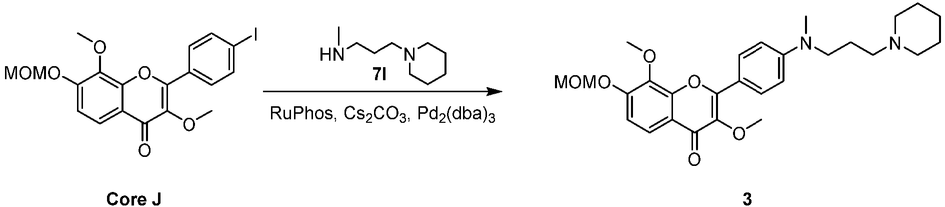

To a solution of **Compound 71** (100.12 mg, 640.70 µmol, 5.72 µL, 1.00 **eq**) and **Core J** (300 mg, 640.70 µmol, 1.00 **eq**) in DMF (5 mL) was added RuPhos (59.80 mg, 128.14 µmol, 0.20 **eq**), Cs₂CO₃ (626.26 mg, 1.92 mmol, 3.00 **eq**) and Pd₂(dba)₃ (117.34 mg, 128.14 µmol, 0.20 **eq**) at 25 °C, that mixture was degassed and purged with N₂ for 3 times. The mixture was stirred at 100 °C for 12 hrs under N₂ atmosphere. LCMS showed **Core J** was consumed. Several new peaks were shown on LCMS and 27.235% of desired compound was detected. The reaction mixture was filtered and concentrated under reduced pressure to give a residue. Without purification. It was put in the next reaction. **Compound 3** (300 mg, 604.12 µmol, 94.29% yield) was obtained as a red oil.

**LCMS:** MS (ESI) Retention time: 0.805min (M+H)⁺ = 497.3.

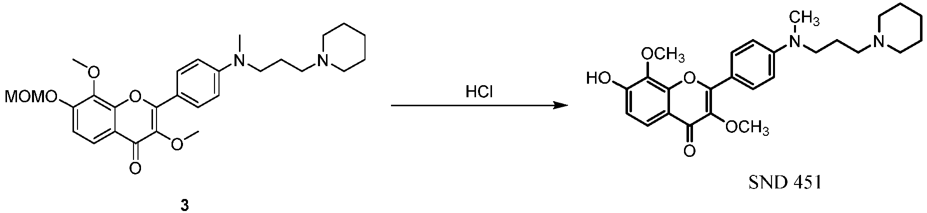

To a solution of **Compound 3** (300 mg, 604.12 µmol, 1.00 **eq**) in HCl/dioxane (3 mL). The mixture was stirred at 25 °C for 1 hr. LCMS showed **Compound 3** was consumed completely and 38.776% of desired mass was detected. The reaction mixture was concentrated under reduced pressure to give a residue. The crude product was purified by reversed-phase HPLC (0.1% HCl condition). **Compound 007-012 (SND 451)** (100 mg, 202.45 µmol, 33.51% yield, 98.997% purity, HCl) was obtained as a yellow oil, which was determined by HNMR and LCMS.

**TLC** (SiO₂, DCM: MeOH = 10/1, Rf = 0.3).

**LCMS:** MS (ESI) Retention time: 0.763min (M+H)⁺ = 453.3.

**LCMS:** MS (ESI) Retention time: 1.176min (M+H)⁺ = 453.1.

**¹H NMR** (400 MHz, METHANOL-d₄) δ = 8.25-8.23 (d, *J =* 9.2 Hz, 2H), 7.79-7.77 (d, *J* = 8.8 Hz, 1H), 7.35-7.33 (d, *J =* 8.8 Hz, 2H), 7.03-7.01 (d, *J =* 8.8 Hz, 1H), 4.01 (s, 3H), 3.82 (s, 3H), 3.70-3.67 (t, *J =* 7.5 Hz, 2H), 3.56-3.53 (d, *J =* 11.6 Hz, 2H), 3.23-3.20 (m, 5H), 2.98-2.95 (m, 2H), 2.15-2.11 (m, 2H), 1.92 (m, 2H), 1.81 (m, 3H), 1.59-1.46 (m, 1H).

### Synthesis of Compound 007-014 (SND 459)

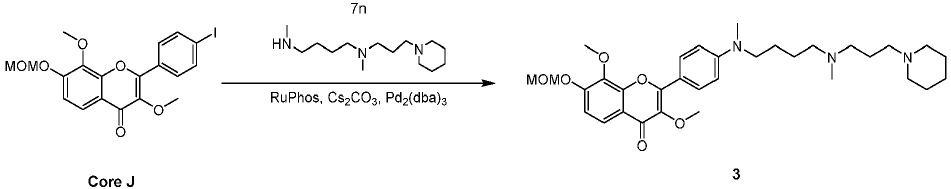

To a solution of **Compound 7n** (154.67 mg, 640.70 µmol, 1.00 **eq**) and **Core J** (300 mg, 640.70 µmol, 1.00 **eq**) in DMF (5 mL) was added RuPhos (59.80 mg, 128.14 µmol, 0.20 **eq**), Cs₂CO₃ (626.26 mg, 1.92 mmol, 3.00 **eq**) and Pd₂(dba)₃ (117.34 mg, 128.14 µmol, 0.20 **eq**) at 25 °C, that mixture was degassed and purged with N₂ for 3 times, the mixture was strried at 100 °C for 12 hrs under N₂ atmosphere. LCMS showed **Core J** consumed. Several new peaks were shown on LCMS and 20.164% of desired compound was detected. The reaction mixture was filtered and concentrated under reduced pressure to give a residue. Without purification. It was put in the next reaction. **Compound 3** (300 mg, 515.69 µmol, 80.49% yield) was obtained as a red oil.

**LCMS:** MS (ESI) Retention time: 0.688min (M+H)⁺ = 582.4.

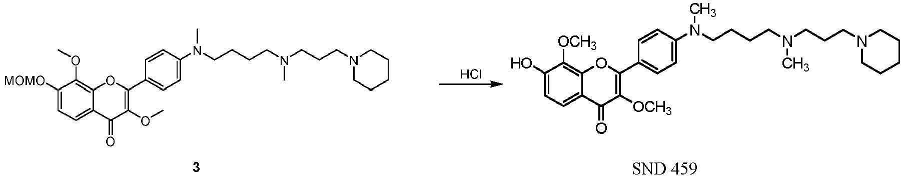

To a solution of **Compound 3** (300 mg, 515.69 µmol, 1.00 *eq*) in HCl/dioxane (5 mL). The mixture was stirred at 25 °C for 1 hr. LCMS showed **Compound 3** was consumed completely and 22.223% of desired mass was detected. The reaction mixture was concentrated under reduced pressure to give a residue. The residue was purified by prep-HPLC (column: 3_Phenomenex Luna C18; 75 × 30 mm × 3 um; mobile phase: [water (HCl) -ACN]; B%: 9%-29%, 8 min). **Compound 007-014 (SND 459)** (90 mg, 156.75 µmol, 30.40% yield, 100% purity, HCl) was obtained as a yellow oil, which was determined by HNMR and LCMS.

**TLC** (SiO₂, DCM: MeOH = 10/1, Rf = 0.1).

**LCMS:** MS (ESI) Retention time: 0.709min (M+H)⁺= 538.4.

**LCMS:** MS (ESI) Retention time: 1.031min (M+H)⁺ = 538.2.

**¹H NMR** (400 MHz, DMSO-d₆) δ = 10.75 (br s, 2H), 8.00-7.97 (d, *J =* 9.2 Hz, 2H), 7.63-7.61 (d, *J =* 8.8 Hz, 1H), 7.03-7.01 (d, *J =* 8.8 Hz, 1H), 6.93-6.91 (d, *J =* 9.2 Hz, 2H), 3.91 (s, 3H), 3.77 (s, 3H), 3.50-3.47 (m, 4H), 3.16-3.07 (m, 6H), 3.03 (s, 3H), 2.85 (m, 2H), 2.74-2.72 (d, *J =* 4.8 Hz, 3H), 2.54 (s, 3H), 2.19 (m, 2H), 1.78-1.60 (m, 10 H).

### Synthesis of Compound 007-016 (SND 453)

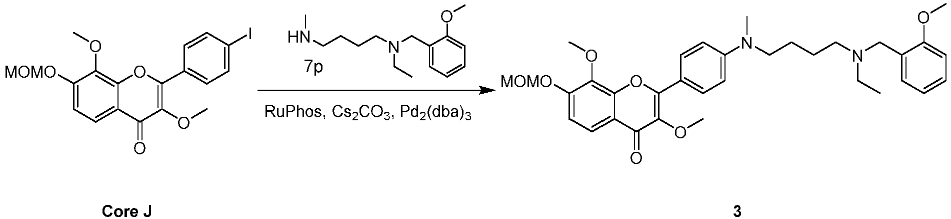

To a solution of **Core J** (300 mg, 640.70 µmol, 1.00 **eq**), RuPhos (59.80 mg, 128.14 µmol, 0.20 **eq**), Pd₂(dba)₃ (117.34 mg, 128.14 µmol, 0.20 **eq**) and Cs₂CO₃ (626.26 mg, 1.92 mmol, 3.00 **eq**) in DMF (5 mL), then added **Compound 7p** (159.15 mg, 640.70 µmol, 1.00 **eq**) at 25 °C, that mixture was degassed and purged with N₂ for 3 times, the mixture was stirred at 100 °C for 16 hr under N₂ atmosphere. LCMS showed **Core J** was consumed. Several new peaks were shown on LCMS and 61.559% of desired compound was detected. The reaction mixture was filtered and concentrated under reduced pressure to give a residue. Without purification. It was put in the next reaction. **Compound 3** (300 mg, 507.87 µmol, 79.27% yield) was obtained as a red oil.

**LCMS:** MS (ESI) Retention time: 0.865min (M+H)⁺ = 591.4.

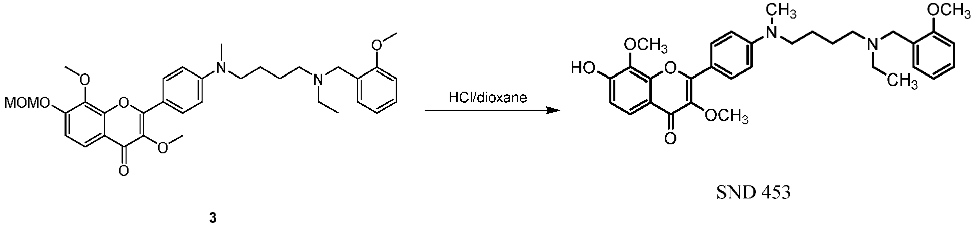

To a solution of **Compound 3** (300 mg, 507.87 µmol, 1.00 *eq*) in HCl/dioxane (3 mL). The mixture was stirred at 25 °C for 1 hr. LCMS (EW30189 - 396-P1A) showed **Compound 3** was consumed completely and 76.482% of desired mass was detected. The reaction mixture was concentrated under reduced pressure to give a residue. The crude product was purified by reversed-phase HPLC (0.1% HCl condition). **Compound 007-016 (SND 453)** (160 mg, 274.39 µmol, 54.03% yield, 100% purity, HCl) was obtained as a yellow oil, which was determined by HNMR and LCMS.

**TLC** (SiO₂, PE: EA = 0/1, Rf = 0.4).

**LCMS:** MS (ESI) Retention time: 0.823min (M+H)⁺ = 547.4.

**LCMS:** MS (ESI) Retention time: 1.438min (M+H)⁺ = 547.2.

**¹H NMR** (400 MHz, DMSO-d₆) δ = 9.87 (br s, 1H), 7.99-7.97 (d, *J =* 9.2 Hz, 2H), 7.63-7.61 (d, *J =* 8.8 Hz, 1H), 7.50 (m, 1H), 7.46-7.40 (m, 1H), 7.11-7.09 (d, *J =* 8.4 Hz, 1H), 7.04-7.00 (m, 2H), 6.94-6.92 (d, *J =* 8.8 Hz, 2H), 4.25-4.22 (t, *J =* 4.4 Hz, 2H), 3.90 (s, 3H), 3.82 (s, 3H), 3.77 (s, 3H), 3.46 (s, 2H), 3.01 (m, 7H), 7.80-7.76 (m, 2H), 1.59-1.53 (m, 2H), 1.28-1.25 (t, *J =* 7.2 Hz, 3H).

### Synthesis of Compound 007-017 (SND 417)

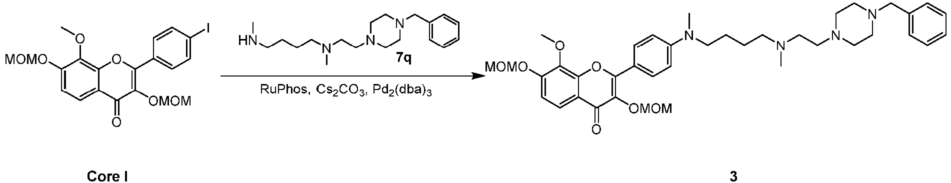

To a solution of **Core J** (300 mg, 640.70 µmol, 1.00 **eq**), RuPhos (59.80 mg, 128.14 µmol, 0.2 o**eq**), Pd, (dba)₃ (117.34 mg, 128.14 µmol, 0.20 **eq**) and Cs₂CO₃ (626.26 mg, 1.92 mmol, 3.00 **eq**) in DMF (1 mL), then added **Compound 7q** (204.06 mg, 640.70 µmol, 1.00 **eq**) at 25 °C, that mixture was degassed and purged with N₂ for 3 times, the mixture was stirred at 100 °C for 12 hrs under N₂ atmosphere. LCMS showed **Core J** consumed. Several new peaks were shown on LCMS and 38.038% of desired compound was detected. The reaction mixture was filtered and concentrated under reduced pressure to give a residue. Without purification. It was put in the next reaction. **Compound3** (300 mg, 455.36 µmol, 71.07% yield) was obtained as a red oil. **LCMS:** MS (ESI) Retention time: 0.783min (M+H)⁺ = 659.5.

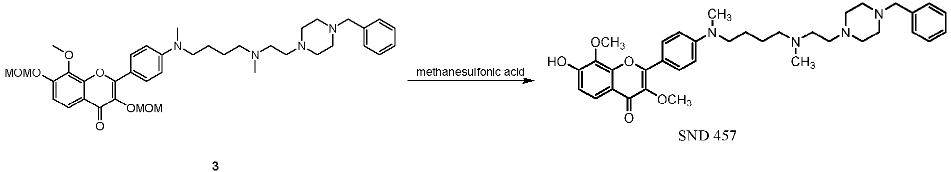

To a solution of **Compound 3** (300 mg, 455.36 µmol, 1.00 **eq**) in HCl/dioxane (10 mL). The mixture was stirred at 25 °C for 1 hr. LCMS showed **Compound 3** was consumed completely and 47.494% of desired mass was detected. The reaction mixture was concentrated under reduced pressure to give a residue. The crude product was purified by reversed-phase HPLC (0.1% HCl condition). **Compound 007-017 (SND 457)** (230 mg, 353.18 µmol, 77.56% yield, 100% purity, HCl) was obtained as a yellow oil, which was determined by HNMR and LCMS.

**TLC** (SiO₂, DCM: MeOH = 10/1, Rf = 0.3).

**LCMS:** MS (ESI) Retention time: 0.494min (M+H)⁺ = 615.2.

**LCMS:** MS (ESI) Retention time: 1.159min (M+H)⁺ = 615.2.

**¹H NMR** (400 MHz, DMSO-d₆) δ = 11.01 (br s, 1H), 8.00-7.98 (d, *J* = 9.2 Hz, 2H), 7.69-7.68 (m, 2H), 7.64-7.62 (d, *J* = 8.8 Hz, 1H), 7.47-7.45 (m, 3H), 7.05-7.03 (d, *J =* 8.8 Hz, 1H), 6.95 (br d, *J =* 8.4 Hz, 2H), 4.43 (br s, 2H), 3.91 (s, 3H), 3.77 (s, 3H), 3.72-3.70 (m, 14H), 3.54-3.46 (m, 14H), 3.16 (m, 2H), 3.04 (s, 3H), 2.79 (s, 3H), 1.79-1.76 (m, 2H), 1.60-1.59 (m, 2H).

### Synthesis of Compound 007-020 (SND 461)

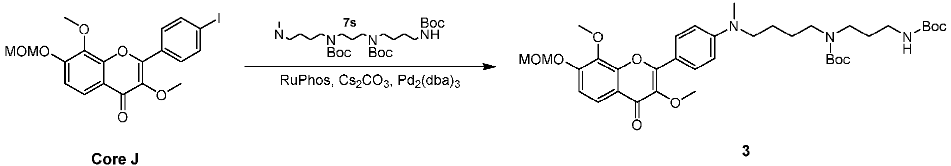

To a solution of **Compound 7s** (221.35 mg, 640.70 µmol, 1.00 **eq**) and **Core J** (300 mg, 640.70 µmol, 1.00 **eq**) in DMF (5 mL) was added RuPhos (59.80 mg, 128.14 µmol, 0.20 **eq**), Cs₂CO₃ (626.26 mg, 1.92 mmol, 3.00 **eq**) and Pd₂(dba)₃ (117.34 mg, 128.14 µmol, 0.20 **eq**) at 25 °C. And that mixture was degassed and purged with N₂ for 3 times. The mixture was stirred at 100 °C for 12 hrs under N₂ atmosphere. LCMS showed **Core J** consumed. Several new peaks were shown on LCMS and 57.282% of desired mass was detected. The reaction mixture was filtered and concentrated under reduced pressure to give a residue. Without purification. It was put in the next reaction. **Compound 3** (400 mg, 583.26 µmol, 91.03% yield) was obtained as a red oil.

**LCMS:** MS (ESI) Retention time: 1.049min (M+H)⁺ = 686.5.

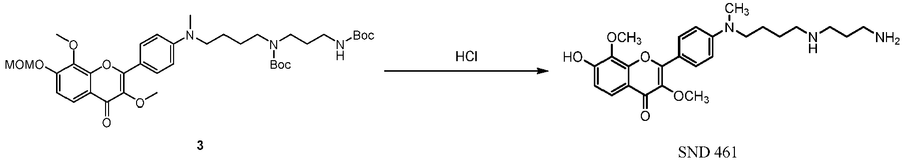

To a solution of **Compound 3** (300 mg, 437.44 µmol, 1.00 **eq**) in HCl/dioxane (5.00 mL). The mixture was stirred at 25 °C for 1 hr. LCMS showed **Compound 3** was consumed completely and 38.215% of desired mass was detected. The reaction mixture was concentrated under reduced pressure to give a residue. The crude product was purified by reversed-phase HPLC (column: 3_Phenomenex Luna C18; 75 × 30 mm × 3 um; mobile phase: [water (HCl) -ACN]; B%: 1%-26%, 8 min). **Compound 007-020 (SND 461)** (110 mg, 230.14 µmol, 52.61% yield, 100% purity, HCl) was obtained as a yellow solid. Which was determined by HNMR and LCMS.

**TLC** (SiO₂, DCM: MeOH = 10/1, Rf = 0.05).

**LCMS:** MS (ESI) Retention time: 0.424min (M+H)⁺ = 442.1.

**LCMS:** MS (ESI) Retention time: 0.879min (M+H)⁺ = 442.1.

**¹H NMR** (400 MHz, DMSO-d₆) δ = 9.29 (br s, 2H), 8.20 (br s, 3H), 7.93-7.91 (d, *J =* 8.8 Hz, 2H), 7.63-7.61 (d, *J =* 8.8 Hz, 1H), 7.03-7.01 (d, *J =* 8.8 Hz, 1H), 6.87-6.85 (d, *J* = 8.8 Hz, 2H), 3.90 (s, 3H), 3.76 (s, 3H), 3.19-3.15 (t, *J* = 6.8 Hz, 2H), 2.98-2.90 (m, 6H), 2.04-1.98 (m, 2H), 1.77-1.75 m, 2H), 1.67-1.64 (m, 2H).

### Synthesis of Compound 007-021 (SND 458)

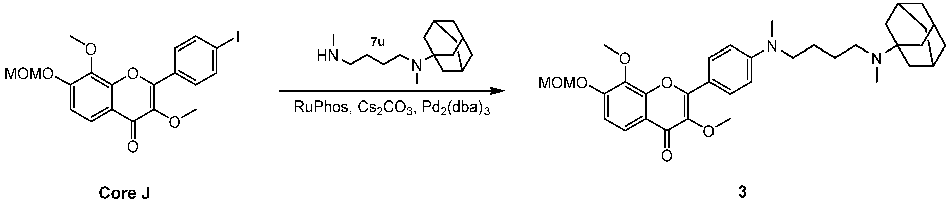

To a solution of **Core J** (300 mg, 660.49 µmol, 1.00 **eq**), RuPhos (61.64 mg, 132.10 µmol, 0.20 **eq**), Pd₂(dba)₃ (120.96 mg, 132.10 µmol, 0.20 **eq**) and Cs₂CO₃ (645.60 mg, 1.98 mmol, 3.00 **eq**) in DMF (5 mL), then added **Compound 7u** (165.40 mg, 660.49 µmol, 1.00 **eq**) at 25 °C, the mixture was stirred at 100 °C for 16 hrs under N₂. LCMS showed **Core J** was consumed. Several new peaks were shown on LCMS and 23.777% of desired compound was detected. The reaction mixture was filtered and concentrated under reduced pressure to give a residue. Without purification. It was put in the next reaction. **Compound 3** (300 mg, 507.83 µmol, 76.89% yield) was obtained as a red oil.

**LCMS:** MS (ESI) Retention time: 0.875min (M+H)⁺ = 591.4.

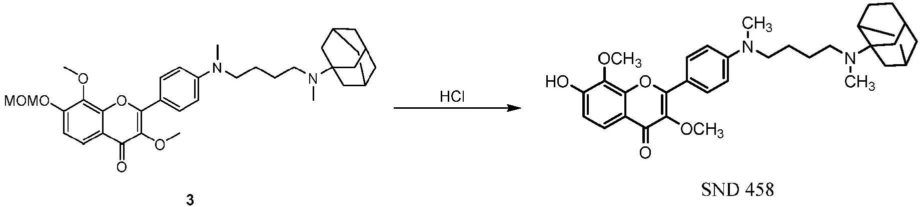

To a solution of **Compound 3** (300 mg, 507.83 µmol, 1.00 *eq*) in HCl/dioxane (5 mL). The mixture was stirred at 25 °C for 1 hr. LCMS showed **Compound 3** was consumed completely and 48.811% of desired mass was detected. The reaction mixture was concentrated under reduced pressure to give a residue. The crude product was purified by reversed-phase HPLC (0.1% HCl condition). **Compound 007-021 (SND 458)** (140 mg, 232.33 µmol, 45.75% yield, 96.774% purity, HCl) was obtained as a yellow oil, which was determined by HNMR and LCMS.

**TLC** (SiO₂, DCM: MeOH = 10/1, Rf = 0.05).

**LCMS:** MS (ESI) Retention time: 0.843min (M+H)⁺ = 547.4.

**LCMS:** MS (ESI) Retention time: 1.795min (M+H)⁺ = 547.4.

**¹H NMR** (400 MHz, DMSO-d₆) δ = 10.06 (br s, 1H), 8.00-7.99 (d, *J* = 7.2 Hz, 2H), 7.63-7.61 (m, 1H), 7.06-7.04 (m, 3H), 3.90 (d, *J =* 2.4 Hz, 3H), 3.77 (d, *J =* 2.4 Hz, 3H), 3.48 (s, 2H), 3.35 (m, 1H), 3.04-3.03 (d, *J =* 1.6 Hz, 3H), 2.70 (m, 1H), 2.61 (s, 3H), 2.13 (s, 3H), 2.00-1.89 (m, 3H), 1.79-1.75 (m, 3H), 1.65 (m, 2H), 1.60 (m, 8H).

### EXAMPLES - BIOLOGICAL STUDIES

### Experimental methodology

### Antitumor activity against a panel of cancer cell lines

Antitumor activity of the compounds and cisplatin as a positive control was assessed by using CellTiter-Glow^{®} Luminescent Cell Viability assay (Promega # G7572) according to the manufacturer's instructions. The compounds were tested at 2 concentrations, 2 and 20 µM, in triplicate well conditions.

Tumor cells were grown at 37°C in a humidified atmosphere with 5% CO₂ in RPMI 1640 or DMEM medium, supplemented with 10% (v/v) fetal calf serum and 50 µg/ml gentamicin for up to 20 passages, and were passaged once or twice weekly. Cells were harvested using TrypLE or PBS buffer containing 1 mM EDTA, and the percentage of viable cells is determined using a cell counter.

Cells were harvested from exponential phase cultures, counted and plated in 96 well flat-bottom microtiter plates at a cell density depending on the cell line's growth rate (4,000 - 20,000 cells/well depending on the cell line's growth rate, up to 60,000 for hematological cancer cell lines) in RPMI 1640 or DMEM medium supplemented with 10% (v/v) fetal calf serum and 50 µg/ml gentamicin (140 µl/well). Cultures were incubated at 37°C and 5% CO₂ in a humidified atmosphere. After 24 h, 10 µl of test compounds or control medium are added and left on the cells for another 72 h. Compounds were serially diluted in DMSO, transferred in cell culture medium, and added to the assay plates. The DMSO concentration was kept constant at < 0.2% v/v across the assay plate. Viability of cells was quantified by CellTiter-Glow^{®} Luminescent Cell Viability assay (Promega). Luminescence was measured with the microplate luminometer (EnVision Multi Label Reader).

Cell lines tested are presented in Table 1.

**Table 1. Tumour cell lines type and designation**

| **Tissue Origin/Disease** | **Cell Line Name** |
|---|---|
| Brain | SK-N-SH |
| | LN-229 |
| | U-118 MG |
| | SH-SY-5Y |
| Colon | HCT 116 |
| Leukemia | HL-60 |
| | K-562 |
| | U937 |
| Lung | NCI-H460 |
| | NCI-H1299 |
| | A549 |
| | NCI-H1437 |
| | NCI-H292 |
| | Calu-1 |
| | NCI-H69 |
| | DMS 114 |
| | NCI-H441 |
| Lymphoma | Daudi |
| | NAMALWA |
| Muscle | A-673 |
| Skin | A-375 |
| | MeWo |
| | SK-MEL-5 |
| | A-2058 |

### Example 1. Activity against brain carcinomas

The test compounds which inhibited brain cancer cell growth >50% at a concentration of 2 µM are presented in Table 2A and 2B.

The brain carcinoma cells H118MG growth was inhibited 97.6 % by SND437 at 2 µM.

**Table 2A. % Inhibition against brain carcinoma cell lines**

| **Cpd/% Inh @2 µM** | **SK-N-SH** |
|---|---|
| SND437 | 96.4 |
| SND438 | 53.5 |
| SND439 | 57.5 |

**Table 2B. % Inhibition against brain carcinoma cell lines**

| **Cpd/% Inh @2 µM** | **SH-SY-5Y** |
|---|---|
| SND446 | 55.8 |

### Example 2. Activity against colon carcinoma

The test compounds which inhibited colon cancer cell growth >50% at a concentration of 2 µM are presented in Table 3.

**Table 3. % Inhibition against colon carcinoma cell lines**

| **Cpd/% Inh @2 µM** | **HCT116** |
|---|---|
| SND437 | 83.7 |

### Example 3. Activity against leukaemia

The test compounds which inhibited leukemia cell growth >50% at a concentration of 2 µM are presented in Table 4. SND446 at 2 µM inhibited the growth of U937 cell line with 58%.

**Table 4. % Inhibition against leukemia cell lines**

| **Cpd/% Inh @2 µM** | **K562** |
|---|---|
| SND437 | 65.3 |

### Example 4. Activity against lung carcinomas

The test compounds which inhibited lung carcinoma cell growth >50% at a concentration of 2 µM are presented in Table 5. The lung carcinoma cells DMS114 growth was inhibited 98% by SND437 at 2 µM.

**Table 5. % Inhibition against lung carcinoma cell lines**

| **Cpd/% Inh @2 µM** | **NCI-H292** |
|---|---|
| SND437 | 71.6 |
| SND438 | 51.9 |
| SND439 | 62.6 |

### Example 5. Activity against lymphoma

The test compounds which inhibited lymphoma cell growth >50% at a concentration of 2 µM are presented in Table 6.

**Table 6. IC₅₀ values against lymphoma**

| **Cpd/% Inh @2 µM** | **NAWALMA** | **Daudi** |
|---|---|---|
| SND437 | 80.8 | 59.4 |
| SND438 | 65.3 | 50 |
| SND439 | 73.4 | 57.4 |
| SND446 | 75.5 | 82 |

### Example 7. Activity against muscle carcinoma (Ewings sarcoma)

The test compounds which inhibited Ewings sarcoma cell growth >50% at a concentration of 2 µM are presented in Table 7.

**Table 7. IC50 values against sarcoma**

| **Cpd/% Inh @2 µM** | **A673** |
|---|---|
| SND437 | 99.6 |
| SND438 | 55.6 |
| SND439 | 80.8 |
| SND445 | 61.8 |
| SND459 | 63.5 |

### Example 8. Activity against skin cancer (melanoma)

The test compounds which inhibited melanoma cell growth >50% at a concentration of 2 µM are presented in Table 8. SND437 and SND446 at 2 µM inhibited the growth of MeWo and SK-Mel-5 cell line 93.2% and 56.5% respectively.

**Table 8. IC50 values against melanoma cell lines**

| **Cpd/% Inh @2 µM** | **A2058** | **A375** | **SK-Mel-5** |
|---|---|---|---|
| SND437 | 97.3 | 69.5 | 99.7 |
| SND438 | 72.1 | 55.5 | 61.1 |
| SND438 | 78.2 | 60.8 | 50.7 |

It will be understood that the present invention has been described above by way of example only. The examples are not intended to limit the scope of the invention. Various modifications and embodiments can be made without departing from the scope of the invention, which is defined by the following claims only.

## Claims

1. A compound of formula (1), or a pharmaceutically acceptable salt, multi-salt or solvate thereof: 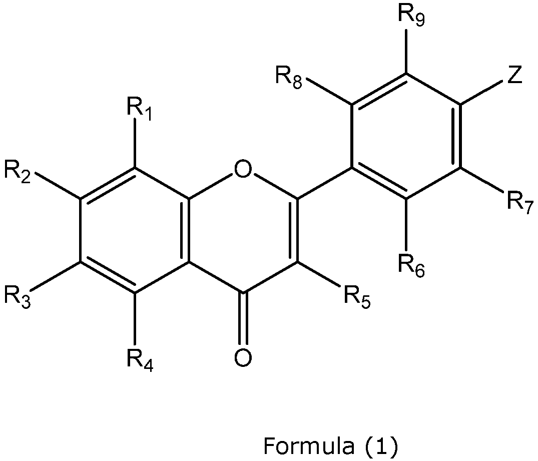 wherein:
Z is selected from:
-NR¹¹R¹²;
-N(R¹⁰)-(CH₂)ₚ-NR¹¹R¹²;
-N(R¹⁰)-(CH₂)_{q}-N(R¹⁰)-(CH₂)_{q}-NR¹¹R¹²; and
-N(R¹⁰)-(CH₂)ᵣ-N(R¹⁰)-(CH₂)ᵣ-N(R¹⁰)-(CH₂)ᵣ-NR¹¹R¹²;
R¹, R², and R⁵, independently, are selected from -OH, -O-C₁₋₄ alkyl, - OC(O)R₁₃, -OC(O)NHR¹³, -OC(O)N(R¹³)₂; or R¹ and R² together form -O-CH₂-O-;
R³, R⁴, R⁶, R⁷, R⁸, and R⁹, independently, are selected from H; halo; -CN; -NO₂; and -NH₂;
each -R^{β} is independently selected from a C₁-C₆ alkyl, C₂-C₆ alkenyl, C₂-C₆ alkynyl , -O(C₁₋₂ alkyl) or C₃-C₁₄ cyclic group, and wherein any -R^{β} may optionally be substituted with one or more C₁-C₄ alkyl, C₁-C₄ haloalkyl, C₃-C₇ cycloalkyl, -O(C₁-C₄ alkyl), -O(C₁-C₄ haloalkyl), -O(C₃-C₇ cycloalkyl), halo, -OH, -NH₂, -CN, -NO₂, -C≡CH, -CHO, -CON(CH₃)₂ or oxo (=O) groups;
each R¹⁰ is independently selected from H, C₁₋₆ alkyl, C₂-C₆ alkenyl, C₂₋₆ alkynyl, C₃₋₁₀ cycloalkyl, and benzyl, wherein each R¹⁰, when not H, is independently optionally substituted with 1 or 2 -R^{β};
R¹¹ and R¹² are independently selected from H, C₁₋₆-alkyl, C₂-C₆ alkenyl, C₂₋₆ alkynyl, C₃₋₁₀ cycloalkyl, and benzyl, wherein each R¹¹ and R¹², when is not H, are independently optionally substituted with 1 or 2 -R^{β}; or R¹¹ and R¹² together form a 5- or 6-membered heterocycle optionally having an additional heteroatom selected from N and O; wherein the 5- or 6-membered heterocycle is optionally substituted with 1 or 2 C₁₋₄ alkyl or benzyl;
each -R¹³ is independently selected from a H, C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), -N(C₁₋₆ alkyl)₂, C₁₋₆ alkylsulfinyl, C₁₋₆ alkylsulfonyl, or arylsulfonyl, wherein any -R¹³ may optionally be substituted with one or more -R¹⁴;
each R¹⁴ is independently selected from a C₁-C₆ alkyl, C₂-C₆ alkenyl, C₂-C₆ alkynyl, C₃₋₁₄ cyclic group, halo, -NO₂, -CN, -OH, -NH₂, mercapto, formyl, carboxy, carbamoyl, C₁₋₆ alkoxy, C₁₋₆ alkylthio, -NH(C₁₋₆ alkyl), - N(C₁₋₆ alkyl)₂, C₁₋₆ alkylsulfinyl, C₁₋₆ alkylsulfonyl, or arylsulfonyl, wherein any -R₁₄ may optionally be substituted with one or more -R₁₅;
each -R¹⁵ is independently selected from halogen, nitro, cyano, hydroxy, trifluoromethoxy, trifluoromethyl, amino, formyl, carboxy, carbamoyl, mercapto, sulfamoyl, methyl, ethyl, methoxy, ethoxy, acetyl, acetoxy, methylamino, ethylamino, dimethylamino, diethylamino, N-methyl-N-ethylamino, acetylamino, N-methylcarbamoyl N-ethylcarbamoyl N,N-dimethylcarbamoyl, N,N-diethylcarbamoyl, N-methyl-N-ethylcarbamoyl, methylthio, ethylthio, methylsulfinyl, ethylsulfinyl, mesyl ethylsulfonyl, methoxycarbonyl, ethoxycarbonyl, N-methylsulfamoyl N-ethylsulfamoyl N,N-dimethylsulfamoyl N,N-diethylsulfamoyl, N-methyl-N-ethylsulfamoyl, carbocyclyl, aryl, or heterocyclyl;
each p is independently an integer selected from 1 to 4;
each q is independently an integer selected from 1 to 4; and
each r is independently an integer selected from 1 to 4.

2. A compound as claimed in claim 1, wherein R¹, R², and R⁵, independently, are selected from -OH, and -O-C₁₋₄ alkyl.

3. A compound as claimed in claim 2, wherein R¹, R², and R⁵ are independently selected from -OH and -OCH₃.

4. A compound as claimed in any one or more preceding claims, wherein R³, R⁴, R⁶, R⁷, R⁸, and R⁹, are H.

5. A compound as claimed in any one or more of the preceding claims, wherein Z is -N(R¹⁰)-(CH₂)ₚ₋NR₁₁R₁₂.

6. A compound as claimed in claim 5, wherein p is selected from 3 and 4.

7. A compound as claimed in any one or more of the preceding claims; wherein R¹¹ and R¹² together form a 5- or 6-membered heterocycle optionally substituted with 1 or 2 C₁₋₄ alkyl or benzyl.

8. A compound as claimed in claim 7; wherein the 5- or 6-membered heterocycle is morpholine, piperidine, piperazine, or pyrrolidine optionally substituted with 1 or 2 C₁₋₄ alkyl or benzyl.

9. A compound as claimed in any one or more of claims 1 to 6, wherein R¹¹ and R¹² are independently selected from H and C₁₋₆ alkyl (e.g., methyl or ethyl), C₃₋₁₀ cycloalkyl (e.g., adamantyl), and benzyl; wherein each R¹¹ and R¹², when not H, are independently optionally substituted with 1 or 2 -R^{β}.

10. A compound as claimed in any one or more of the preceding claims wherein R¹⁰ is H or -CH₃.

11. A compound as claimed in one or more of any of the preceding claims, wherein the compound is a compound of Formula (1A): 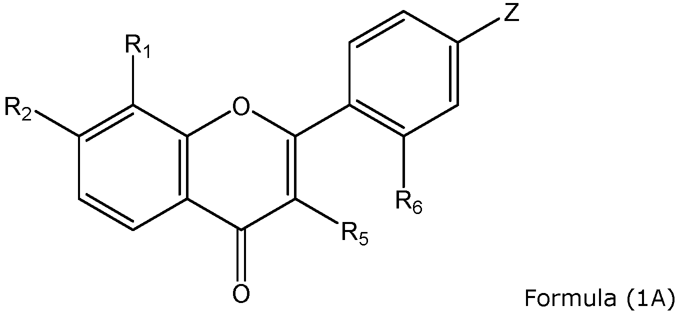 wherein R¹, R², R⁵, R⁶, and Z are as defined in claims 1 to 10.

12. A compound as claimed in claim 1, wherein the compound is selected from:

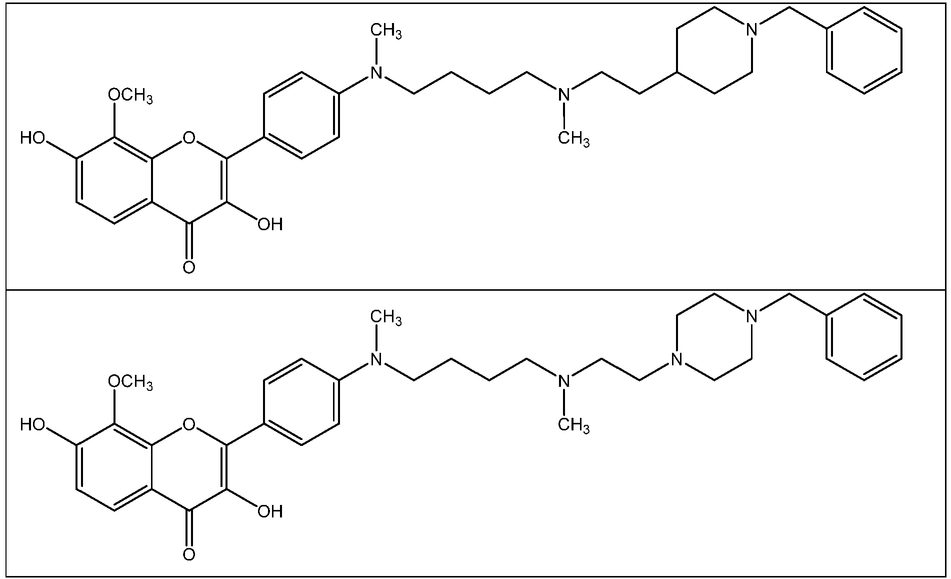

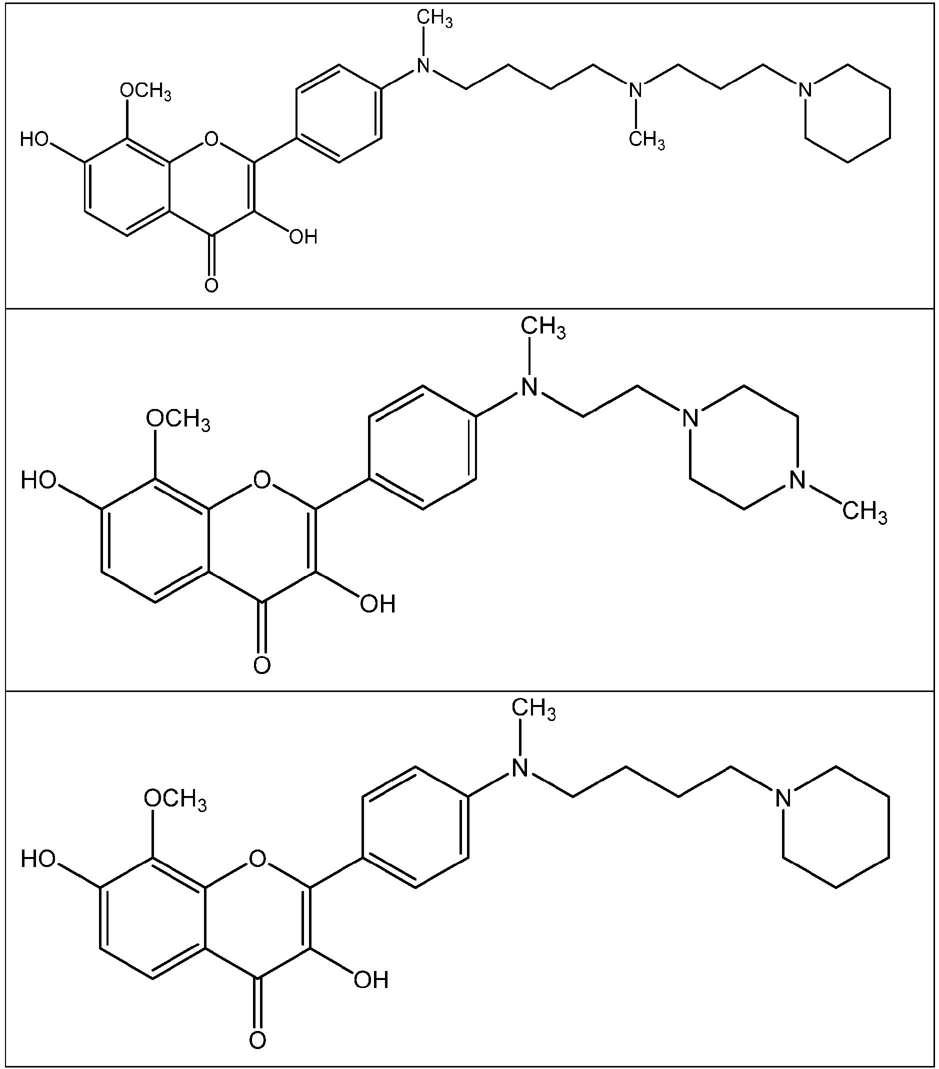

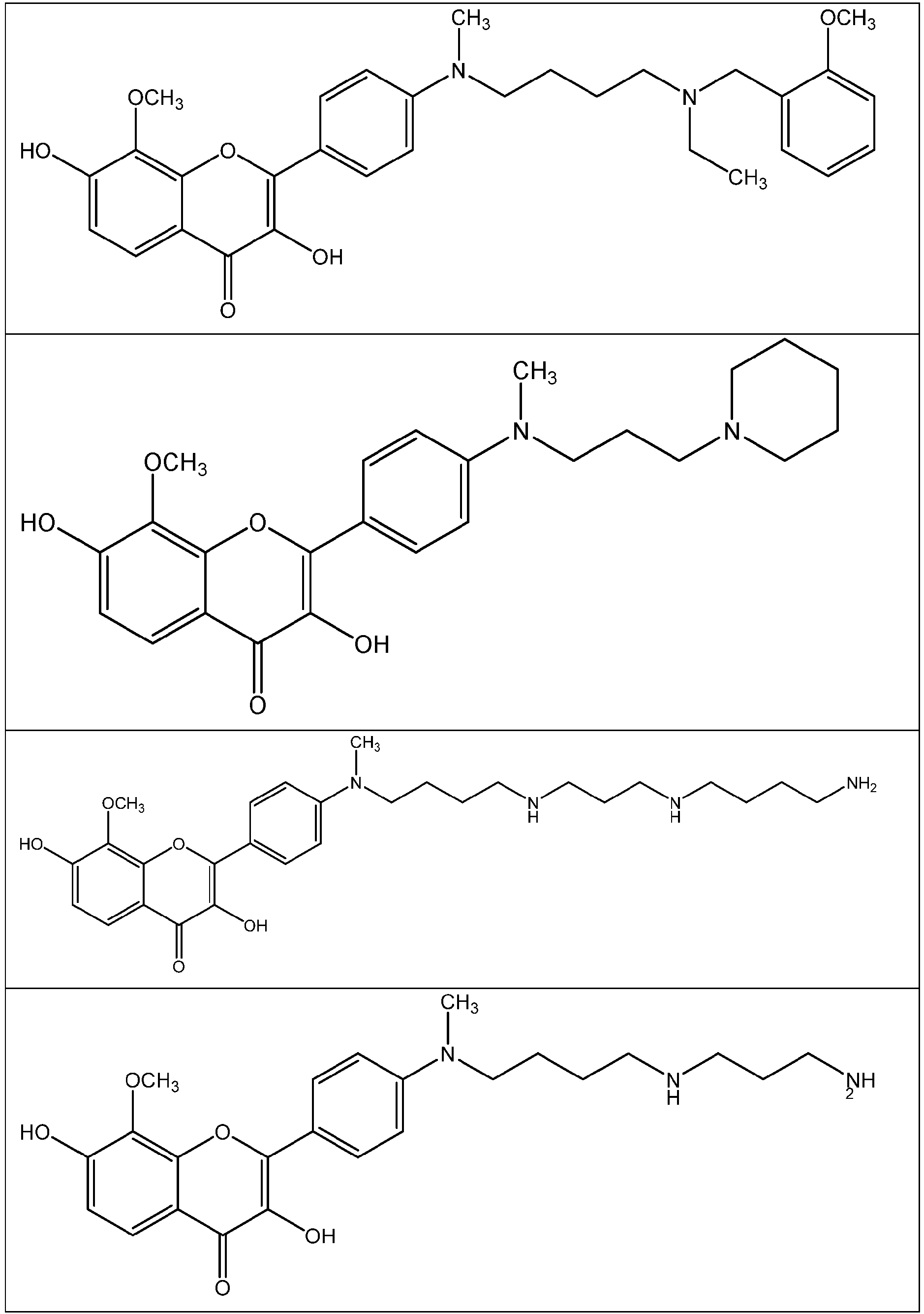

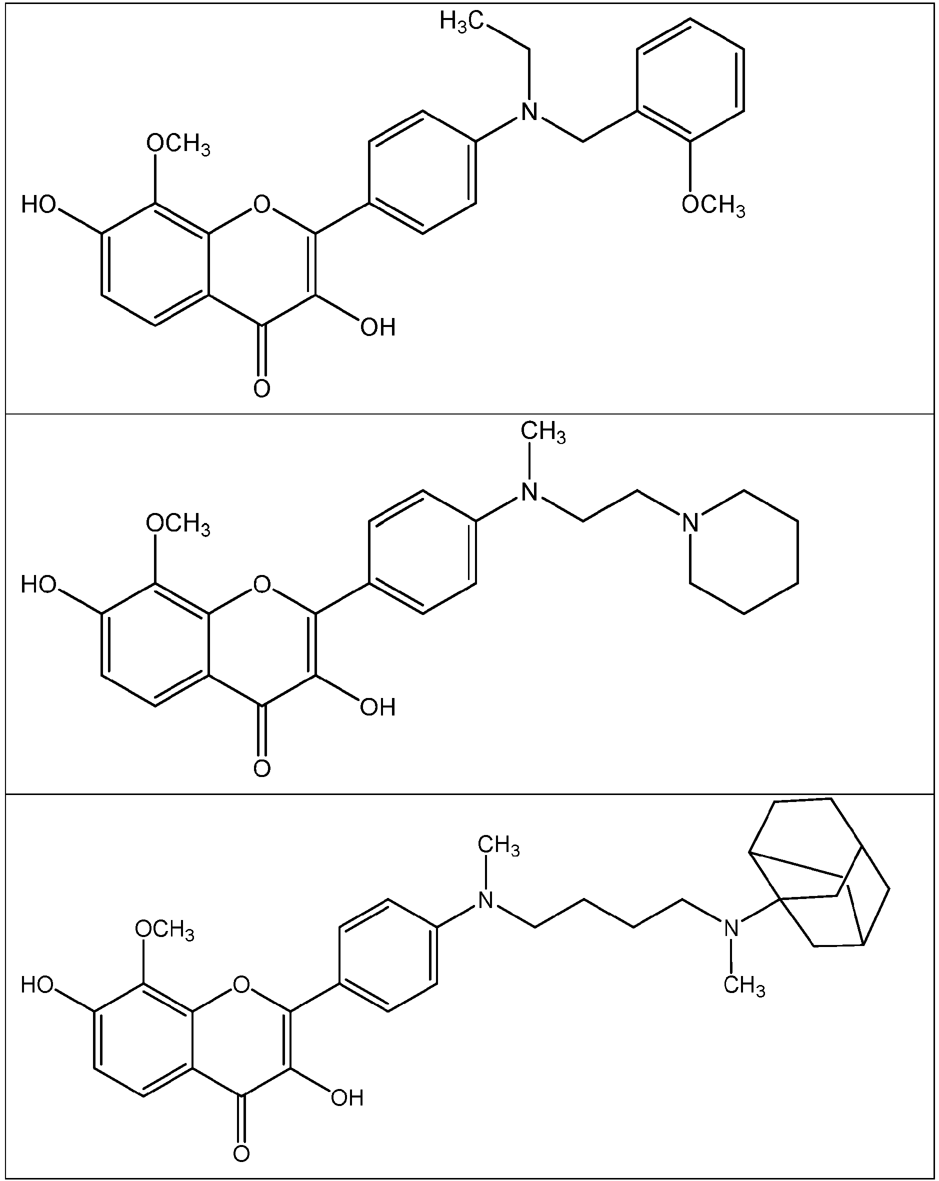

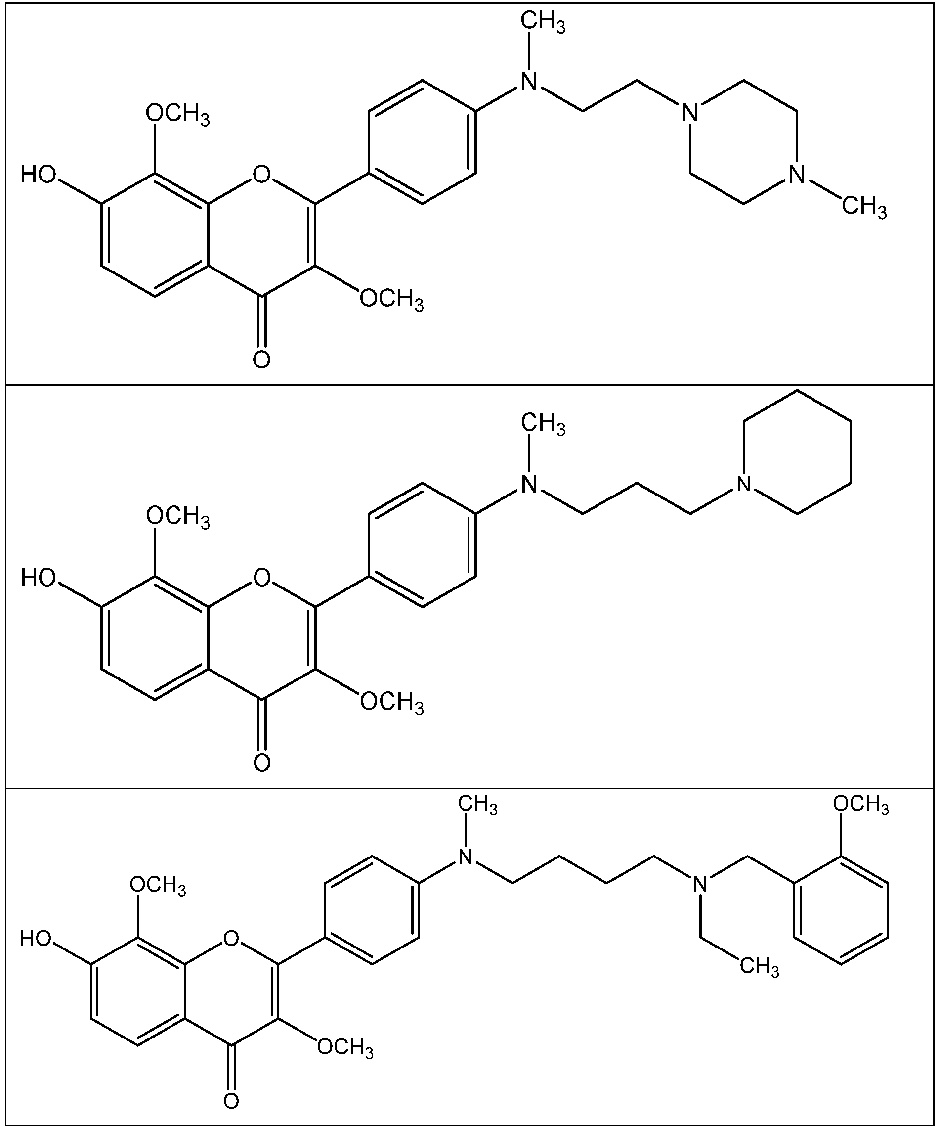

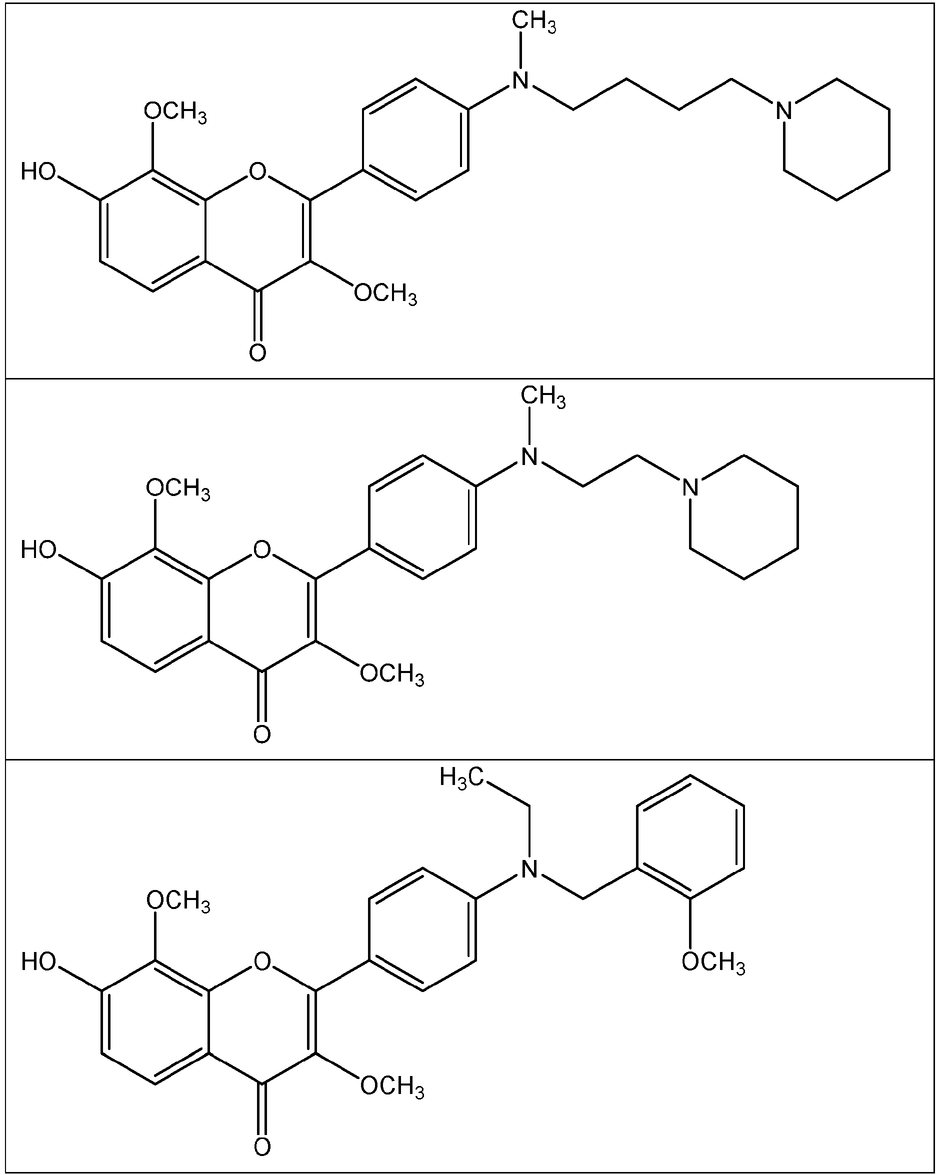

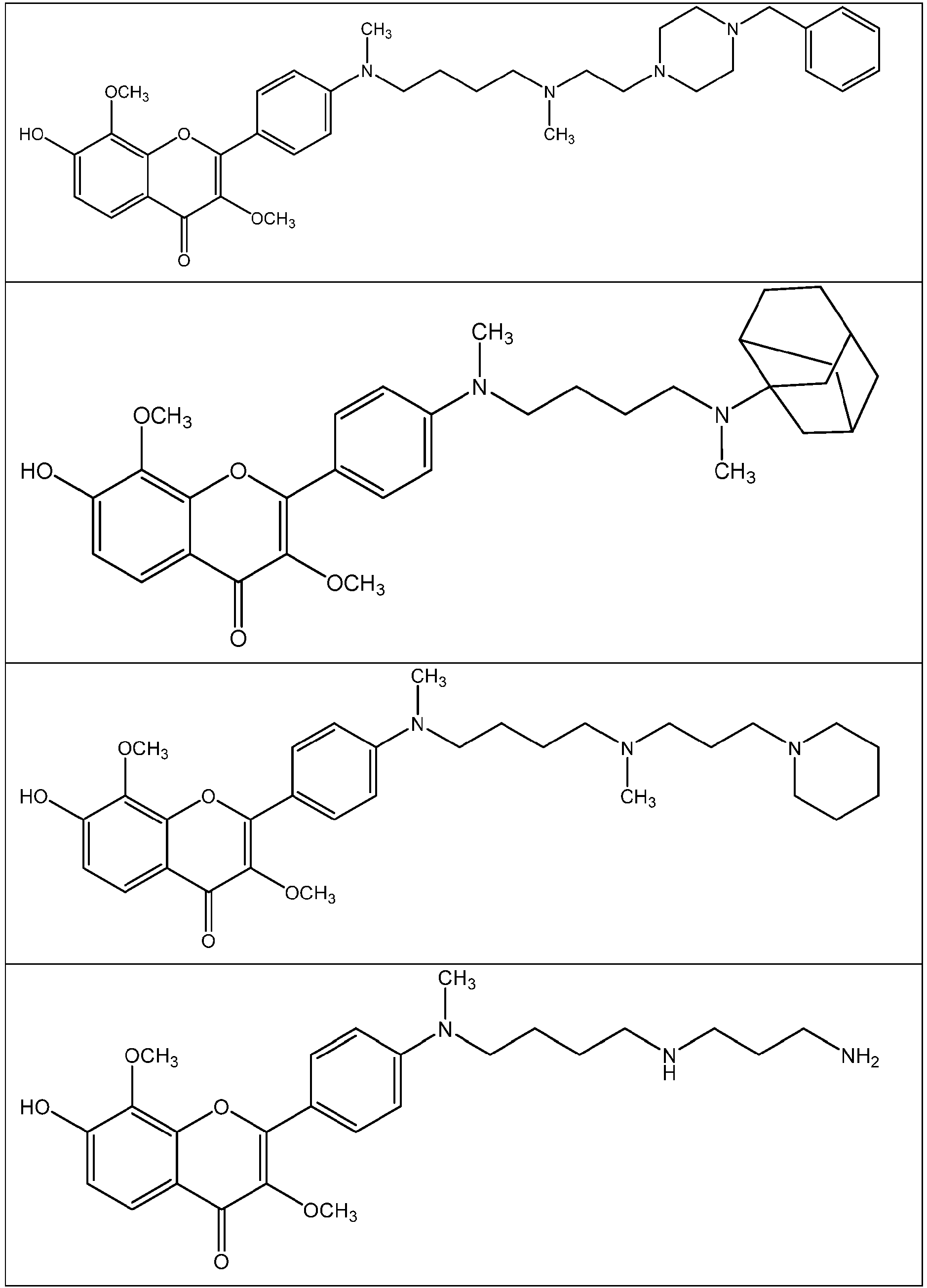

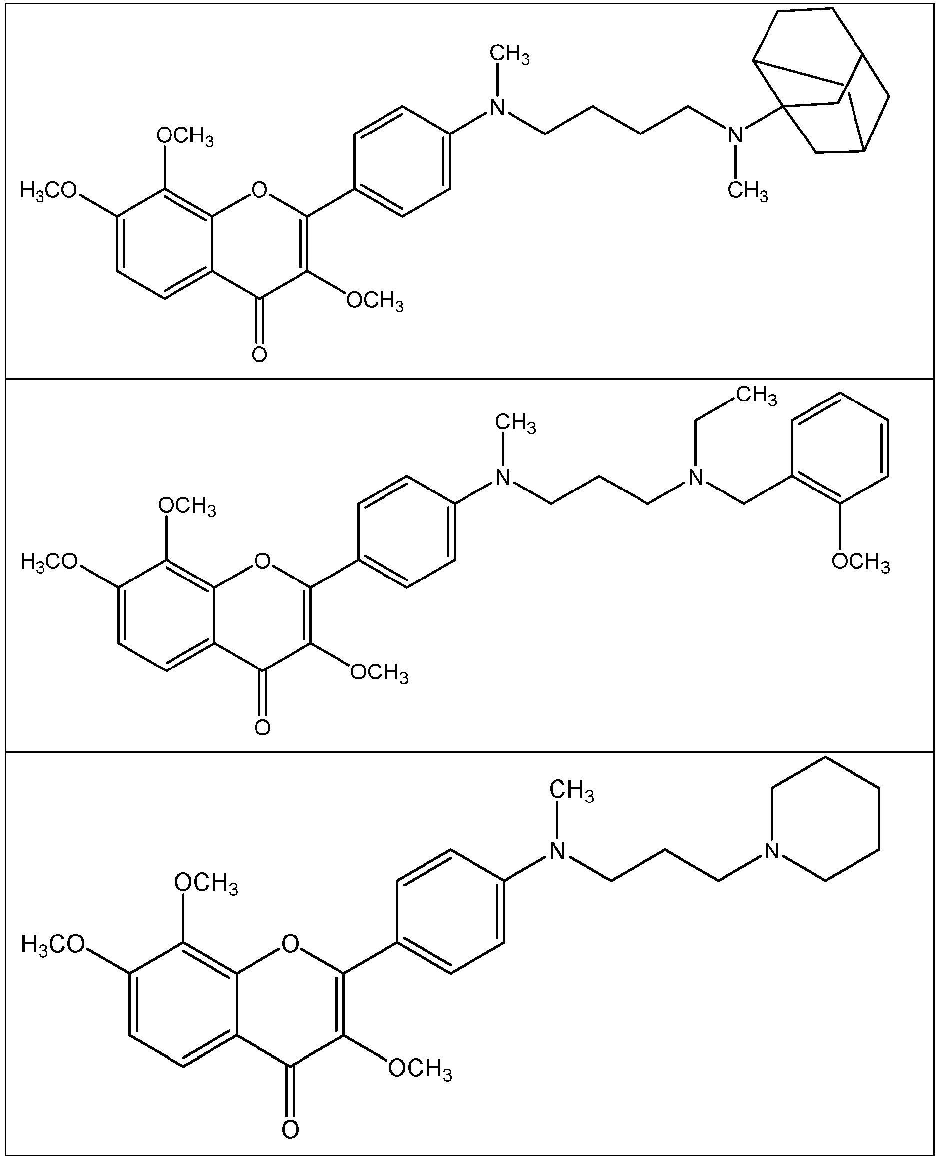

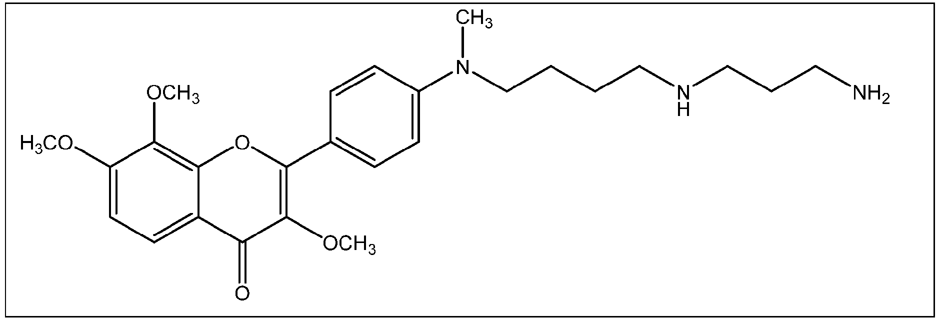

13. A pharmaceutical composition comprising a compound, or a pharmaceutically acceptable salt, multi-salt or solvate as defined in one of the preceding claims, and a pharmaceutically acceptable excipient.

14. A compound, or a pharmaceutically acceptable salt, multi-salt or solvate as defined in one or more of claims 1 to 12, or a pharmaceutical composition as defined in claim 13, for use in medicine.

15. A compound, or a pharmaceutically acceptable salt, multi-salt or solvate as defined in one or more of claims 1 to 12, or a pharmaceutical composition as defined in claim 13, for use treating or preventing cancer.

## Patentansprüche

1. Verbindung der Formel (1) oder ein pharmazeutisch unbedenkliches Salz, Mehrfachsalz oder Solvat davon: 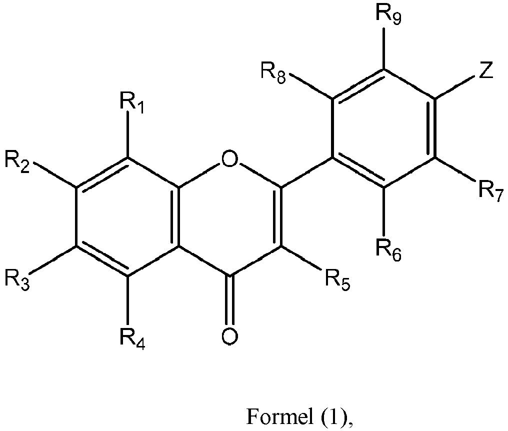 wobei:
Z ausgewählt ist aus:
-NR¹¹R¹²;
-N(R¹⁰)-(CH₂)ₚ-NR¹¹R¹²;
-N(R¹⁰)-(CH₂)_{q}-N(R¹⁰)-(CH₂)_{q}-NR¹¹R¹²; und
-N(R¹⁰)-(CH₂)ᵣ-N(R¹⁰)-(CH₂)ᵣ-N(R¹⁰)-(CH₂)ᵣ-NR¹¹R¹²;
R¹, R² und R⁵ unabhängig ausgewählt sind aus -OH, -O-C₁₋₄-Alkyl, -OC(O)R₁₃, - OC(O)NHR¹³, -OC(O)N(R¹³)₂; oder R¹ und R² zusammen -O-CH₂-O- bilden;
R³, R⁴, R⁶, R⁷, R⁸ und R⁹ unabhängig ausgewählt sind aus H; Halo; -CN; -NO₂; und -NH₂;
jedes -R^{β} unabhängig ausgewählt ist aus einem C₁-C₆-Alkyl, C₂-C₆-Alkenyl, C₂-C₆-Alkinyl, -O(C₁₋₂-Alkyl) oder einer zyklischen C₃-C₁₄-Gruppe, und wobei jedes -R^{β} optional substituiert sein kann mit einem oder mehreren C₁-C₄-Alkyl, C₁-C₄-Haloalkyl, C₃-C₇-Cycloalkyl, -O(C₁-C₄-Alkyl), -O(C₁-C₄-Haloalkyl), -O(C₃-C₇-Cycloalkyl), Halo, -OH, -NH₂, -CN, -NO₂, - C=CH, -CHO, -CON(CH₃)₂ oder Oxo(=O)-Gruppen;
jedes R¹⁰ unabhängig ausgewählt ist aus H, C₁₋₆-Alkyl, C₂-C₆-Alkenyl, C₂₋₆-Alkinyl, C₃₋₁₀-Cycloalkyl und Benzyl, wobei jedes R¹⁰, wenn es nicht H ist, unabhängig optional substituiert ist mit 1 oder 2 -R^{β};
R¹¹ und R¹² unabhängig ausgewählt sind aus H, C₁₋₆-Alkyl, C₂-C₆-Alkenyl, C₂₋₆-Alkinyl, C₃₋₁₀-Cycloalkyl und Benzyl, wobei jedes R¹¹ und R¹², wenn sie nicht H sind, unabhängig optional substituiert sind mit 1 oder 2 -R^{β}; oder R¹¹ und R¹² zusammen einen 5- oder 6-gliedrigen Heterocyclus bilden, optional mit einem zusätzlichen Heteroatom ausgewählt aus N und O; wobei der 5- oder 6-gliedrige Heterocyclus optional substituiert ist mit 1 oder 2 C₁₋₄-Alkyl oder Benzyl;
jedes -R¹³ unabhängig ausgewählt ist aus einem H, C₁-C₆-Alkyl, C₂-C₆-Alkenyl, C₂-C₆-Alkinyl, einer cyclischen C₃₋₁₄-Gruppe, Halo, -NO₂, -CN, -OH, -NH₂, Mercapto, Formyl, Carboxy, Carbamoyl, C₁₋₆-Alkoxy, C₁₋₆-Alkylthio, -NH(C₁₋₆-Alkyl), -N(C₁₋₆-Alkyl)₂, C₁₋₆-Alkylsulfinyl, C₁₋₆-Alkylsulfonyl oder Arylsulfonyl, wobei jedes -R¹³ optional substituiert sein kann mit einem oder mehreren -R¹⁴;
jedes R¹⁴ unabhängig ausgewählt ist aus einem C₁-C₆-Alkyl, C₂-C₆-Alkenyl, C₂-C₆-Alkinyl, einer cyclischen C₃₋₁₄-Gruppe, Halo, -NO₂, -CN, -OH, -NH₂, Mercapto, Formyl, Carboxy, Carbamoyl, C₁₋₆-Alkoxy, C₁₋₆-Alkylthio, -NH(C₁₋₆-Alkyl), -N(C₁₋₆-Alkyl)₂, C₁₋₆-Alkylsulfinyl, C₁₋₆-Alkylsulfonyl oder Arylsulfonyl, wobei jedes -R₁₄ optional substituiert sein kann mit einem oder mehreren -R₁₅;
jedes -R¹⁵ unabhängig ausgewählt ist aus Halogen, Nitro, Cyano, Hydroxy, Trifluormethoxy, Trifluormethyl, Amino, Formyl, Carboxy, Carbamoyl, Mercapto, Sulfamoyl, Methyl, Ethyl, Methoxy, Ethoxy, Acetyl, Acetoxy, Methylamino, Ethylamino, Dimethylamino, Diethylamino, N-Methyl-N-ethylamino, Acetylamino, N-Methylcarbamoyl, N-ethylcarbamoyl, N,N-dimethylcarbamoyl, N,N-Diethylcarbamoyl, N-Methyl-N-ethylcarbamoyl, Methylthio, Ethylthio, Methylsulfinyl, Ethylsulfinyl, Mesylethylsulfonyl, Methoxycarbonyl, Ethoxycarbonyl, N-Methylsulfamoyl, N-ethylsulfamoyl, N,N-dimethylsulfamoyl, N,N-diethylsulfamoyl, N-Methyl-N-ethylsulfamoyl, Carbocyclyl, Aryl, oder Heterocyclyl;
jedes p unabhängig eine ganze Zahl ausgewählt aus 1 bis 4 ist;
jedes q unabhängig eine ganze Zahl ausgewählt aus 1 bis 4 ist; und
jedes r unabhängig eine ganze Zahl ausgewählt aus 1 bis 4 ist.

2. Verbindung nach Anspruch 1, wobei R¹, R² und R⁵ unabhängig ausgewählt sind aus -OH und -O-C₁₋₄-Alkyl.

3. Verbindung nach Anspruch 2, wobei R¹, R² und R⁵ unabhängig ausgewählt sind aus -OH und -OCH₃.

4. Verbindung nach einem oder mehreren der vorhergehenden Ansprüche, wobei R³, R⁴, R⁶, R⁷, R⁸ und R⁹ H sind.

5. Verbindung nach einem oder mehreren der vorhergehenden Ansprüche, wobei Z -N(R¹⁰)-(CH₂)ₚ-NR¹¹R¹² ist.

6. Verbindung nach Anspruch 5, wobei p ausgewählt ist aus 3 und 4.

7. Verbindung nach einem oder mehreren der vorhergehenden Ansprüche; wobei R¹¹ und R¹² zusammen einen 5- oder 6-gliedrigen Heterocyclus bilden, der optional substituiert ist mit 1 oder 2 C₁₋₄-Alkyl oder Benzyl.

8. Verbindung nach Anspruch 7; wobei der 5- oder 6-gliedrige Heterocyclus Morpholin, Piperidin, Piperazin oder Pyrrolidin ist, das optional substituiert ist mit 1 oder 2 C₁₋₄-Alkyl oder Benzyl.

9. Verbindung nach einem oder mehreren der Ansprüche 1 bis 6, wobei R¹¹ und R¹² unabhängig ausgewählt sind aus H und C₁₋₆-Alkyl (z. B. Methyl oder Ethyl), C₃₋₁₀-Cycloalkyl (z. B. Adamantyl) und Benzyl; wobei jedes R¹¹ und R¹², wenn sie nicht H sind, unabhängig optional substituiert sind mit 1 oder 2 -R^{β}.

10. Verbindung nach einem oder mehreren der vorhergehenden Ansprüche, wobei R¹⁰ H oder -CH₃ ist.

11. Verbindung nach einem oder mehreren eines der vorhergehenden Ansprüche, wobei die Verbindung eine Verbindung der Formel (1A) ist: 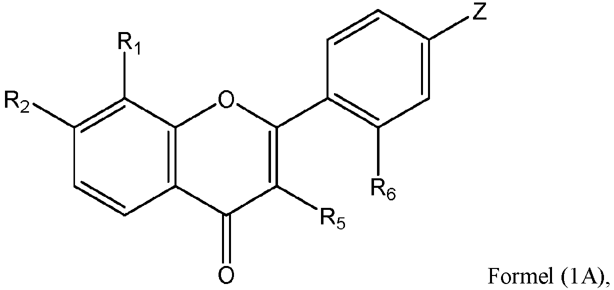 wobei R¹, R², R⁵, R⁶ und Z wie in den Ansprüchen 1 bis 10 definiert sind.

12. Verbindung nach Anspruch 1, wobei die Verbindung ausgewählt ist aus:

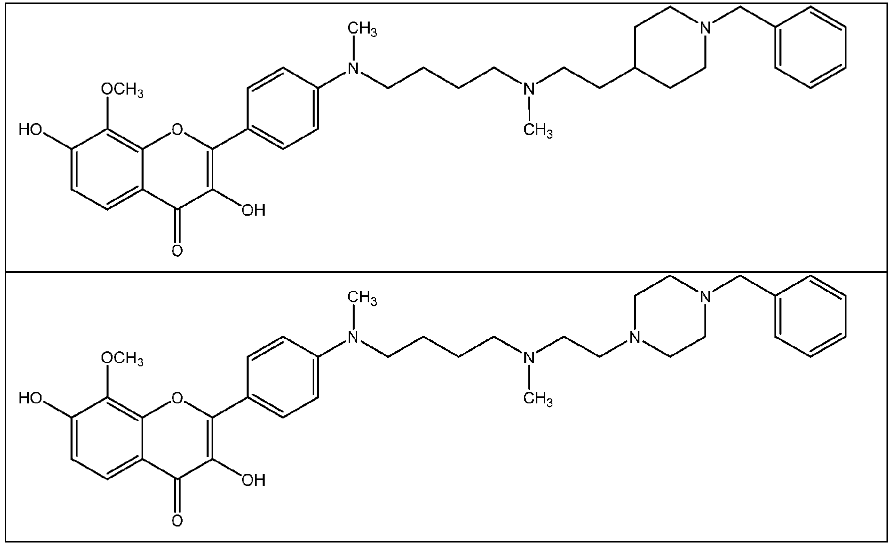

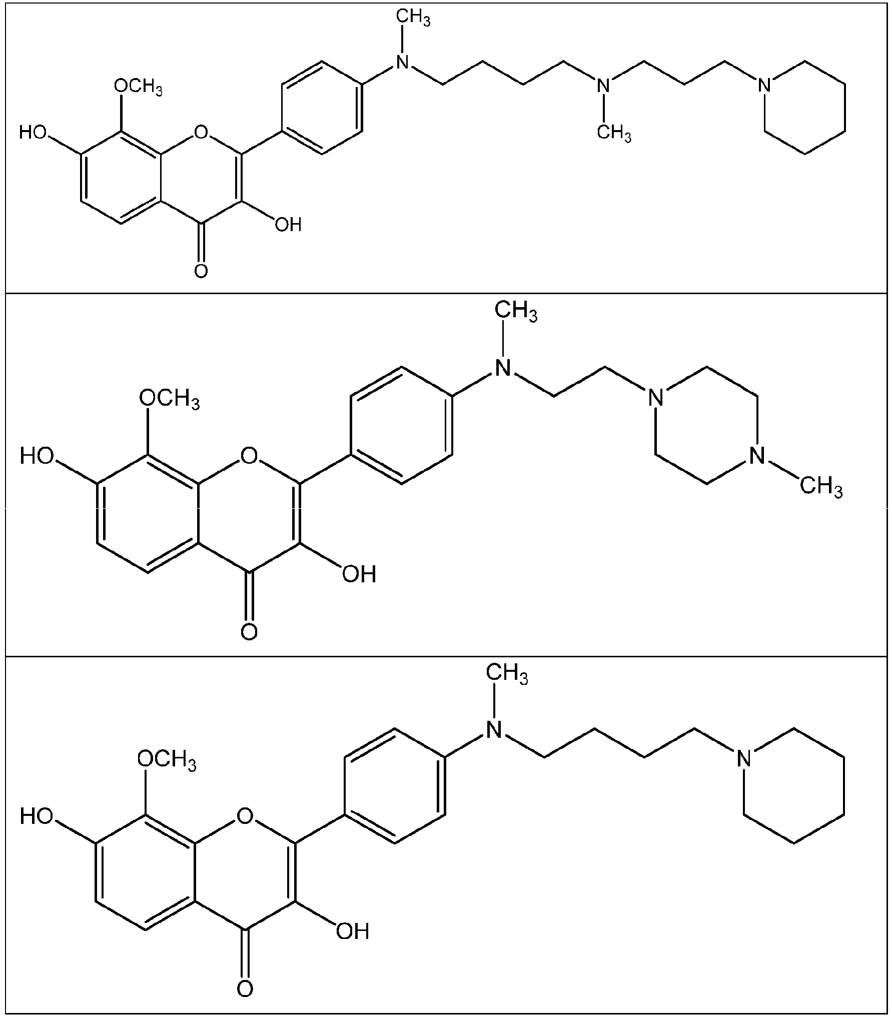

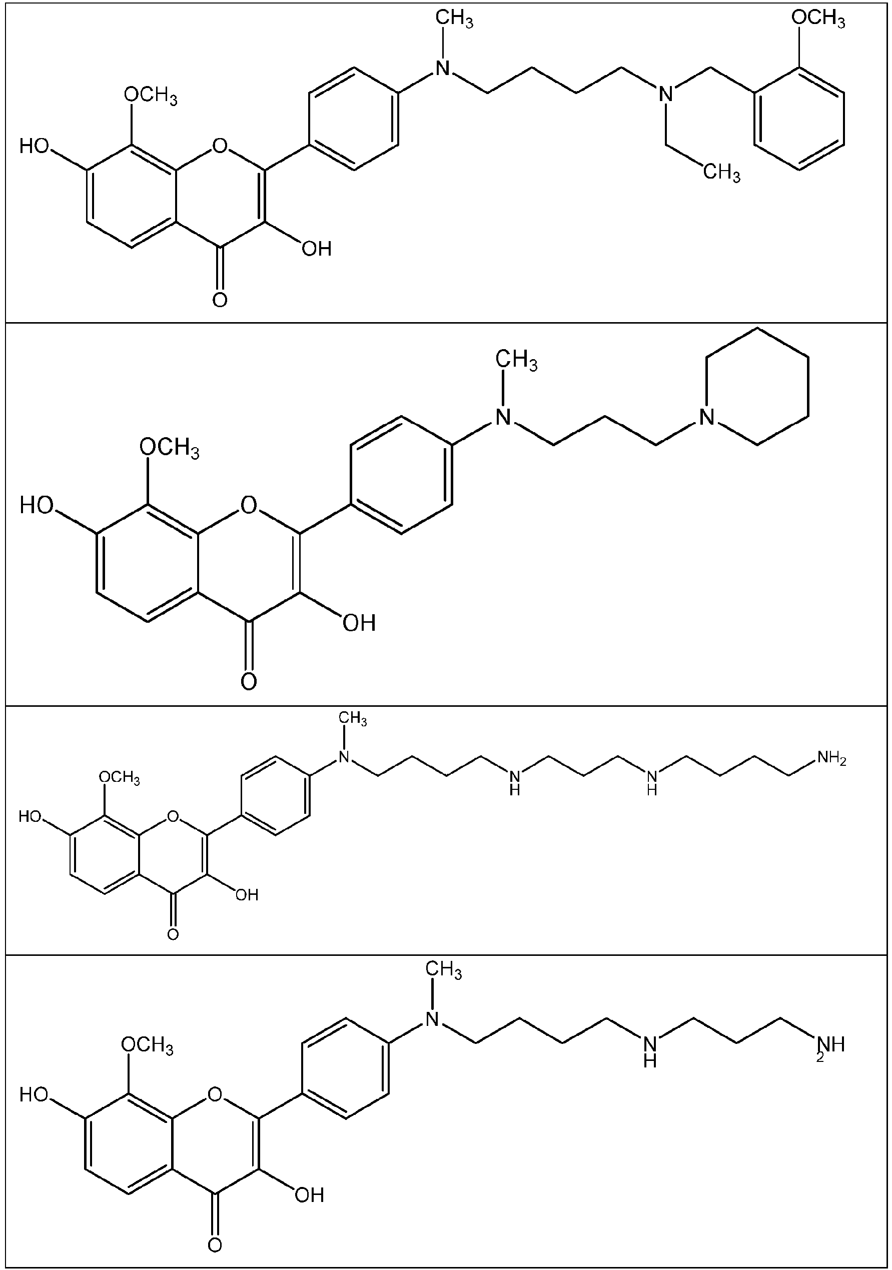

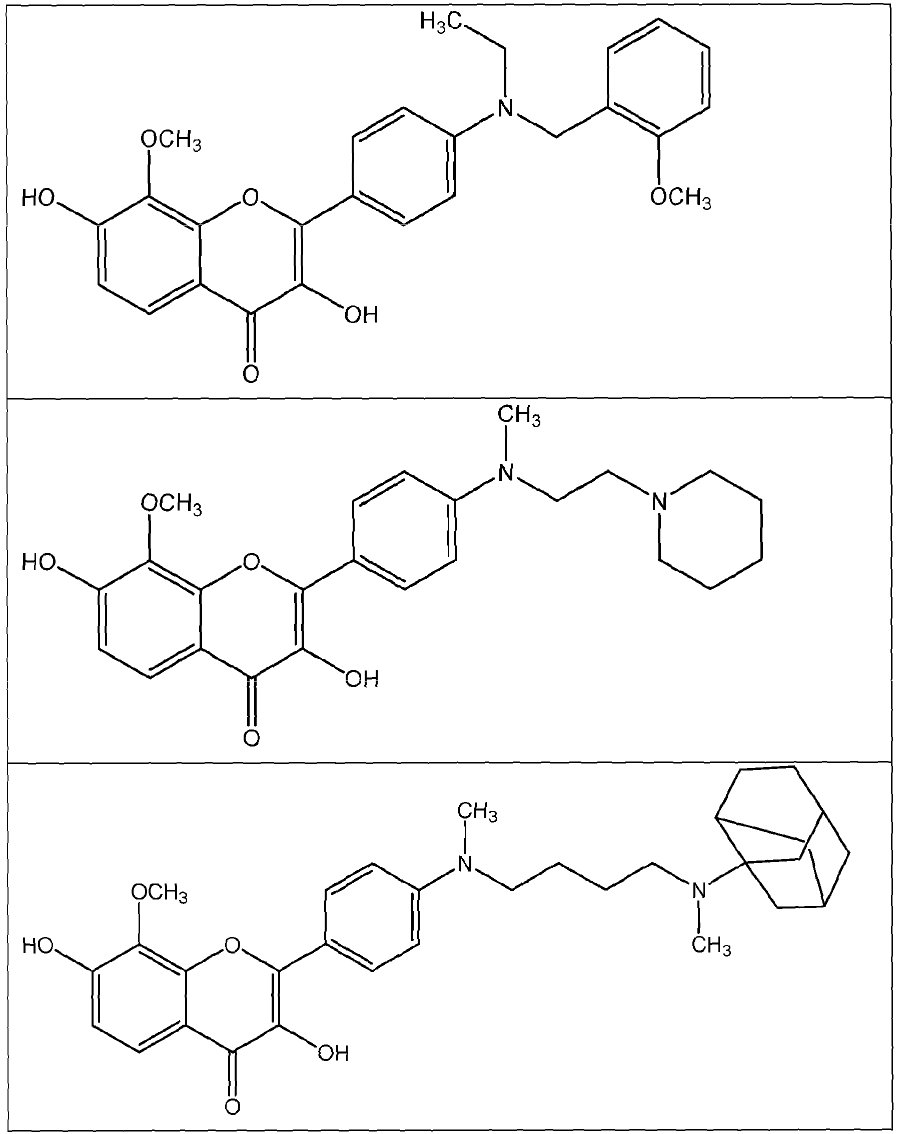

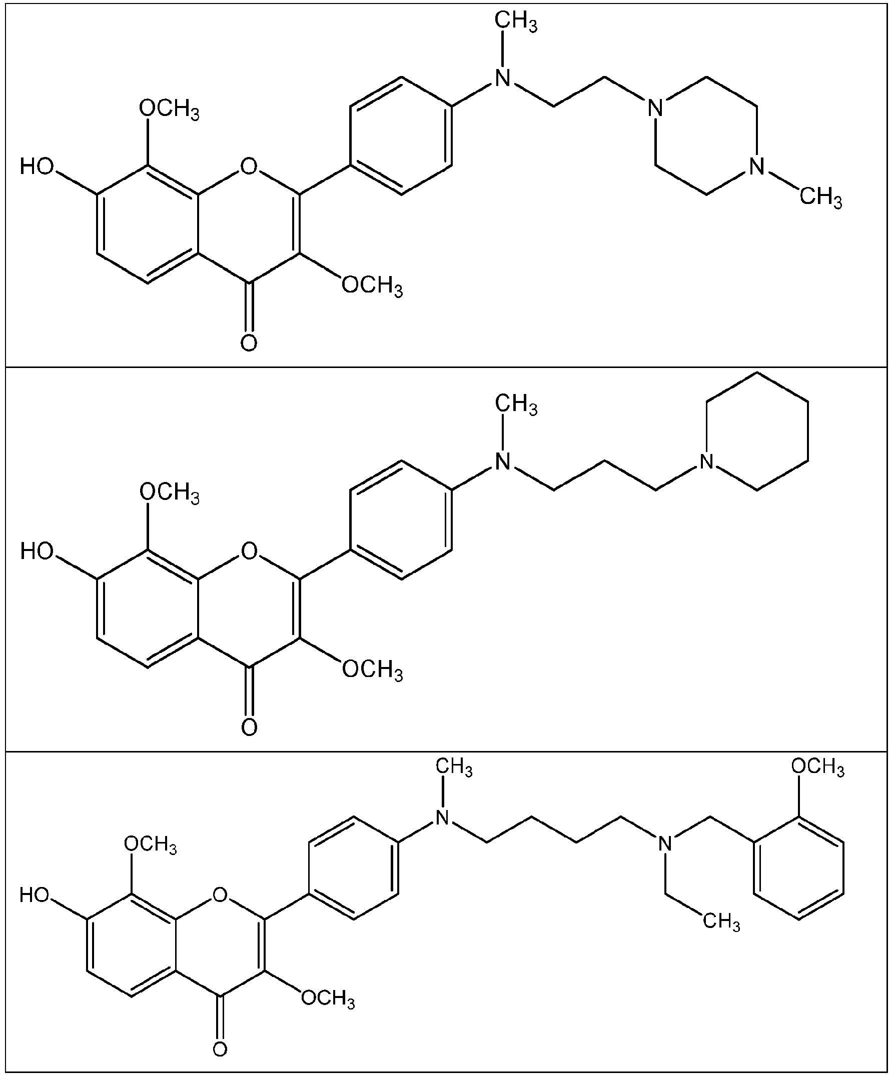

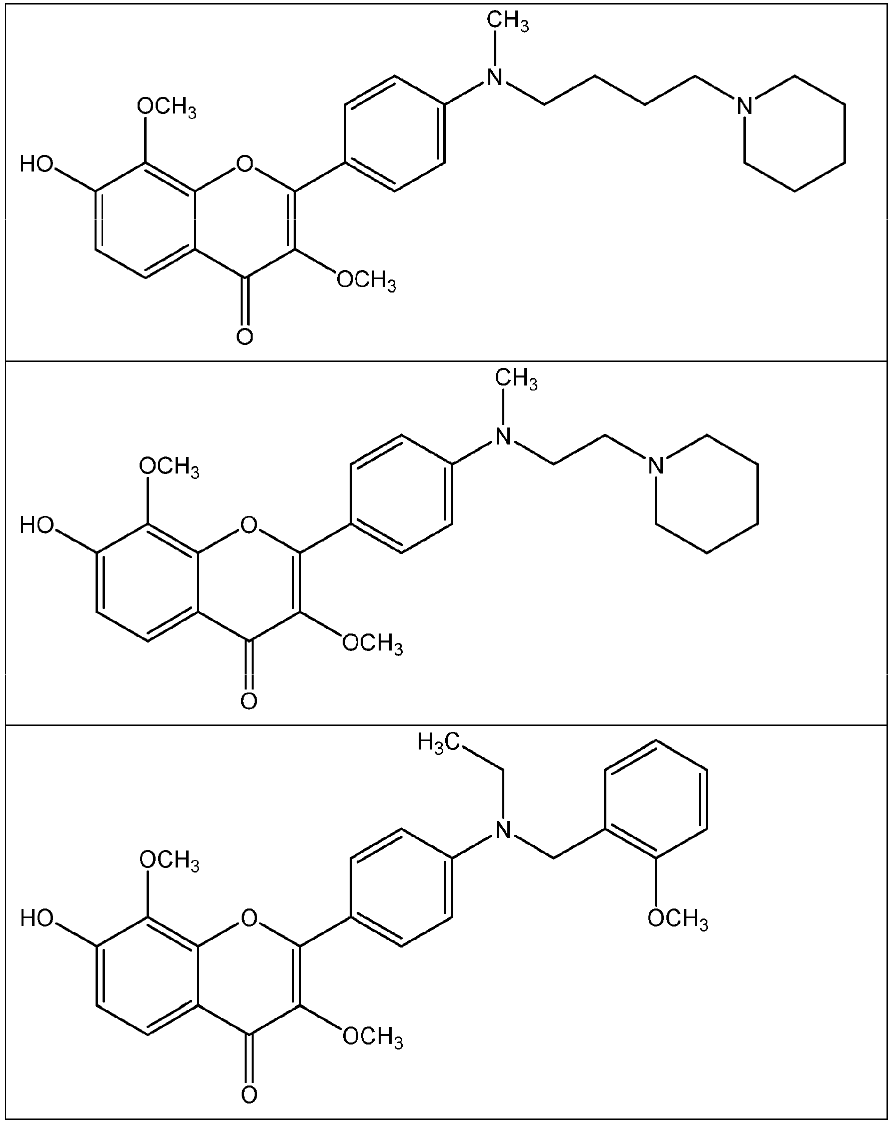

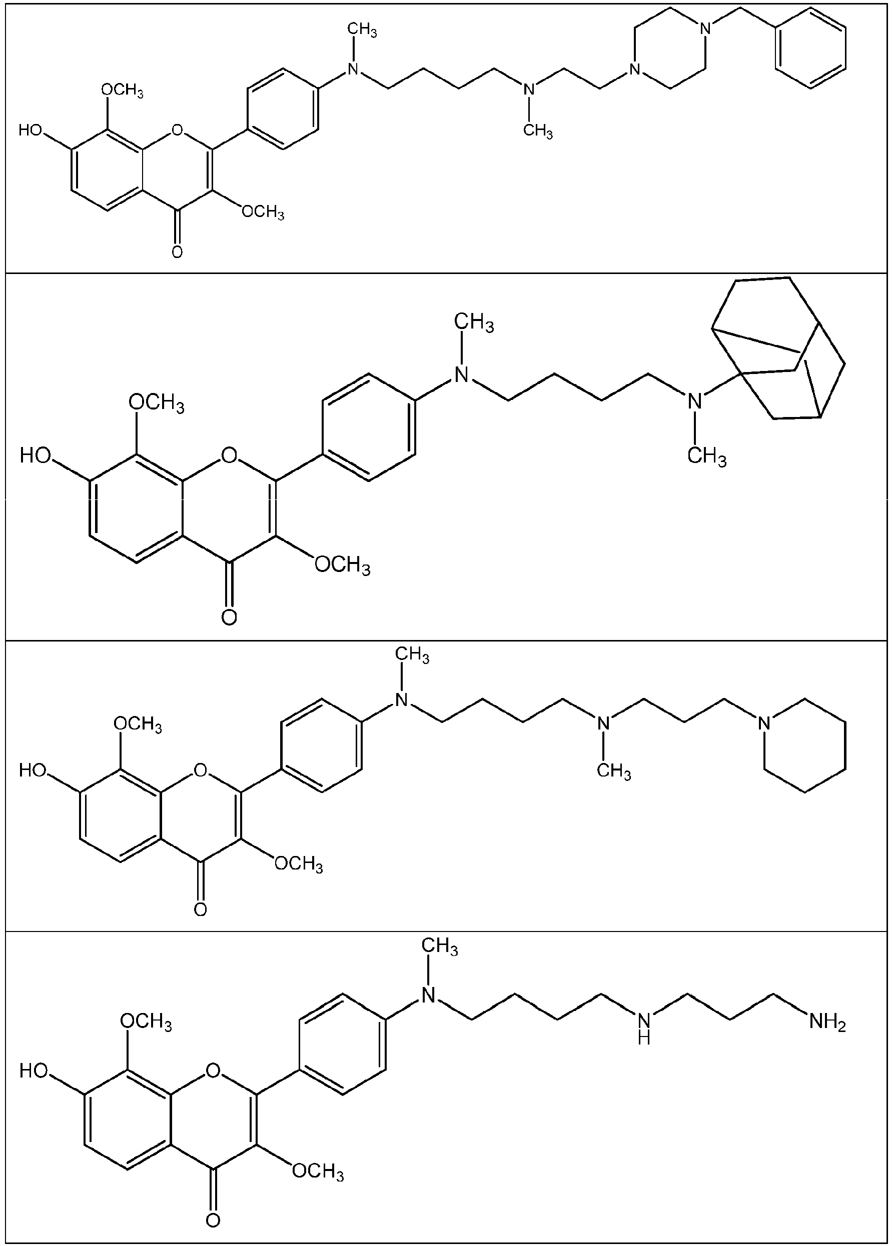

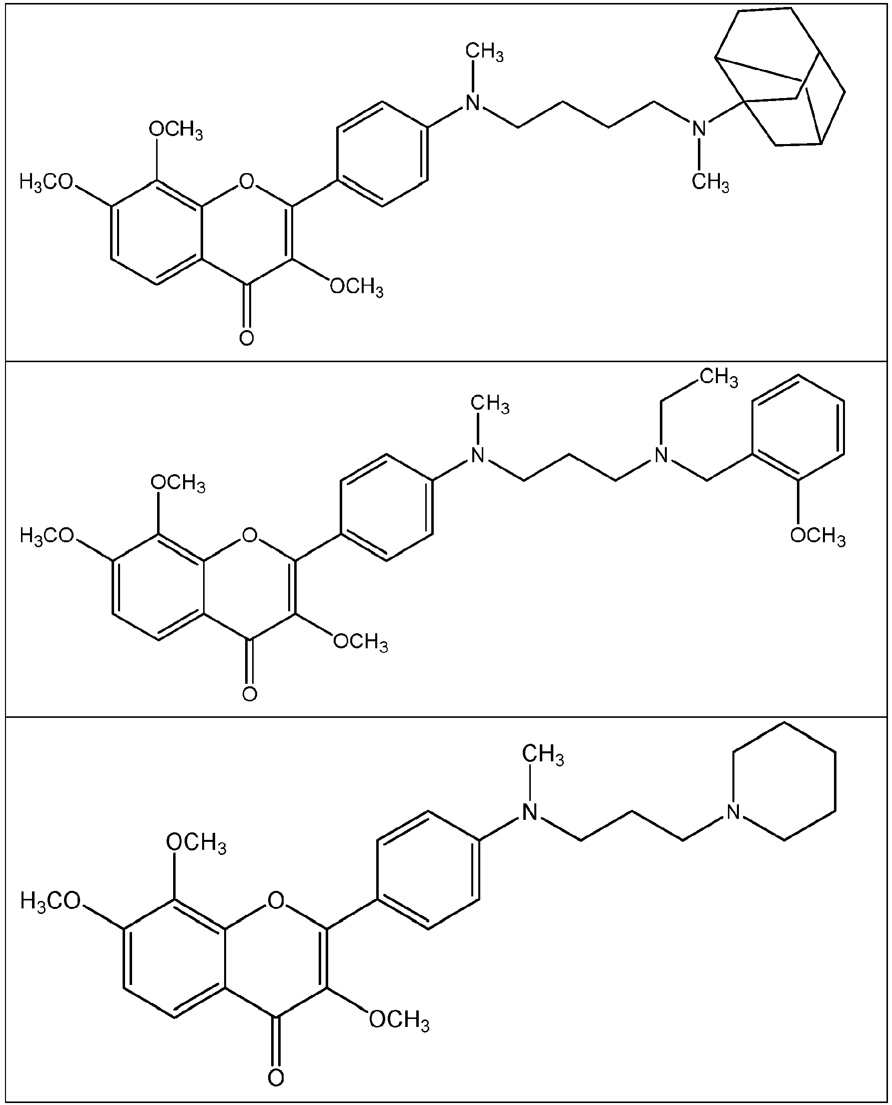

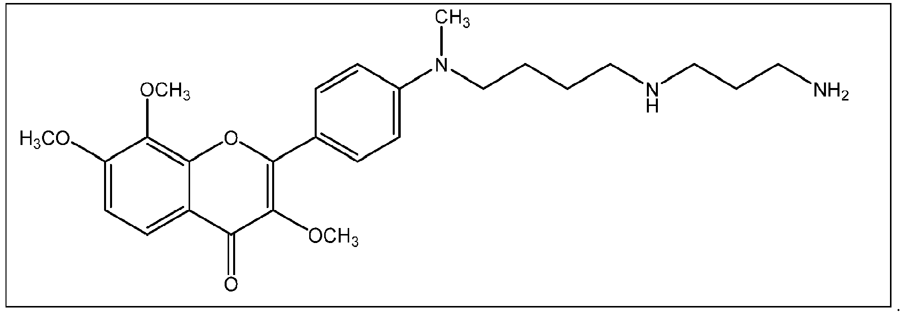

13. Pharmazeutische Zusammensetzung, umfassend eine Verbindung oder ein pharmazeutisch unbedenkliches Salz, Mehrfachsalz oder Solvat, wie in einem der vorhergehenden Ansprüche definiert, und einen pharmazeutisch unbedenklichen Hilfsstoff.

14. Verbindung oder ein pharmazeutisch unbedenkliches Salz, Mehrfachsalz oder Solvat, wie in einem oder mehreren der Ansprüche 1 bis 12 definiert, oder eine pharmazeutische Zusammensetzung, wie in Anspruch 13 definiert, zur Verwendung in der Medizin.

15. Verbindung oder ein pharmazeutisch unbedenkliches Salz, Mehrfachsalz oder Solvat, wie in einem oder mehreren der Ansprüche 1 bis 12 definiert, oder eine pharmazeutische Zusammensetzung, wie in Anspruch 13 definiert, zur Verwendung beim Behandeln oder Vorbeugen von Krebs.

## Revendications

1. Composé de formule (1), ou sel, sels multiples ou solvate acceptable(s) pharmaceutiquement de celui-ci : 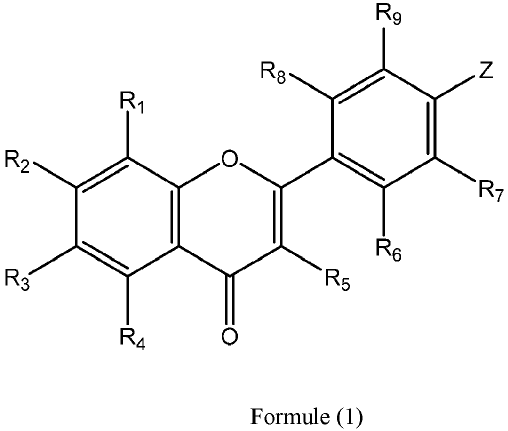 dans lequel :
Z est choisi parmi :
-NR¹¹R¹² ;
-N(R¹⁰)-(CH₂)ₚ-NR¹¹R¹² ;
-N(R¹⁰)-(CH₂)_{q}-N(R¹⁰)-(CH₂)_{q}-NR¹¹R¹² ; et
-N(R¹⁰)-(CH₂)ᵣ-N(R¹⁰)-(CH₂)ᵣ-N(R¹⁰)-(CH₂)ᵣ-NR¹¹R¹² ;
R¹, R² et R⁵ sont, indépendamment, choisis parmi -OH, -O-alkyle en C₁₋₄, -OC(O)R₁₃, - OC(O)NHR¹³, -OC(O)N(R¹³)₂ ; ou R¹ et R² forment ensemble -O-CH₂-O- ;
R³, R⁴, R⁶, R⁷, R⁸ et R⁹ sont, indépendamment, choisis parmi H ; un halogéno ; -CN ; - NO₂ ; et -NH₂ ;
chaque -R^{β} est indépendamment choisi parmi un alkyle en C₁-C₆, un alcényle en C₂-C₆, un alcynyle en C₂-C₆, -O(alkyle en C₁₋₂) ou un groupe cyclique en C₃-C₁₄, et tout -R^{β} pouvant éventuellement être substitué par un ou plusieurs groupes alkyle en C₁-C₄, halogénoalkyle en C₁-C₄, cycloalkyle en C₃-C₇, -O(alkyle en C₁-C₄), -O(halogénoalkyle en C₁-C₄), -O(cycloalkyle en C₃-C₇), halogéno, -OH, -NH₂, -CN, -NO₂, -C=CH, -CHO, -CON(CH₃)₂ ou oxo (=O) ;
chaque R¹⁰ est indépendamment choisi parmi H, un alkyle en C₁₋₆, un alcényle en C₂-C₆, un alcynyle en C₂₋₆, un cycloalkyle en C₃₋₁₀ et un benzyle, chaque R¹⁰, lorsqu'il n'est pas H, étant indépendamment éventuellement substitué par 1 ou 2 -R^{β} ;
R¹¹ et R¹² sont indépendamment choisis parmi H, un alkyle en C₁₋₆, un alcényle en C₂-C₆, un alcynyle en C₂₋₆, un cycloalkyle en C₃₋₁₀ et un benzyle, chaque R¹¹ et R¹², lorsqu'ils ne sont pas H, sont indépendamment éventuellement substitués par 1 ou 2 -R^{β} ; ou R¹¹ et R¹² forment ensemble un hétérocycle à 5 ou 6 chaînons ayant éventuellement un hétéroatome supplémentaire choisi parmi N et O ; ledit hétérocycle à 5 ou 6 chaînons étant éventuellement substitué par 1 ou 2 alkyle en C₁₋₄ ou benzyle ;
chaque -R¹³ est indépendamment choisi parmi H, un alkyle en C₁-C₆, un alcényle en C₂-C₆, un alcynyle en C₂-C₆, un groupe cyclique en C₃₋₁₄, un halogéno, -NO₂, -CN, -OH, -NH₂, un mercapto, un formyle, un carboxy, un carbamoyle, un alcoxy en C₁₋₆, un alkylthio en C₁₋₆, - NH(alkyle en C₁₋₆), -N(alkyle en C₁₋₆)₂, un alkylsulfinyle en C₁₋₆, un alkylsulfonyle en C₁₋₆ ou un arylsulfonyle, tout -R¹³ pouvant éventuellement être substitué par un ou plusieurs -R¹⁴ ;
chaque R¹⁴ est indépendamment choisi parmi un alkyle en C₁-C₆, un alcényle en C₂-C₆, un alcynyle en C₂-C₆, un groupe cyclique en C₃₋₁₄, un halogéno, -NO₂, -CN, -OH, -NH₂, un mercapto, un formyle, un carboxy, un carbamoyle, un alcoxy en C₁₋₆, un alkylthio en C₁₋₆, -NH(alkyle en C₁₋₆), -N(alkyle en C₁₋₆)₂, un alkylsulfinyle en C₁₋₆, un alkylsulfonyle en C₁₋₆ ou un arylsulfonyle, tout -R₁₄ pouvant éventuellement être substitué par un ou plusieurs -R₁₅ ;
chaque -R¹⁵ est indépendamment choisi parmi un halogène, un nitro, un cyano, un hydroxy, un trifluorométhoxy, un trifluorométhyle, un amino, un formyle, un carboxy, un carbamoyle, un mercapto, un sulfamoyle, un méthyle, un éthyle, un méthoxy, un éthoxy, un acétyle, un acétoxy, un méthylamino, un éthylamino, un diméthylamino, un diéthylamino, un N-méthyl-N-éthylamino, un acétylamino, un N-méthylcarbamoyle, un N-éthylcarbamoyle, un N,N-diméthylcarbamoyle, un N,N-diéthylcarbamoyle, un N-méthyl-N-éthylcarbamoyle, un méthylthio, un éthylthio, un méthylsulfinyle, un éthylsulfinyle, un mésyle, un éthylsulfonyle, un méthoxycarbonyle, un éthoxycarbonyle, un N-méthylsulfamoyle, un N-éthylsulfamoyle, un N,N-diméthylsulfamoyle, un N,N-diéthylsulfamoyle, un N-méthyl-N-éthylsulfamoyle, un carbocyclyle, un aryle, ou un hétérocyclyle ;
chaque p est indépendamment un nombre entier choisi de 1 à 4 ;
chaque q est indépendamment un nombre entier choisi de 1 à 4 ; et
chaque r est indépendamment un nombre entier choisi de 1 à 4.

2. Composé selon la revendication 1, dans lequel R¹, R² et R⁵ sont, indépendamment, choisis parmi -OH et -O-alkyle en C₁₋₄.

3. Composé selon la revendication 2, dans lequel R¹, R² et R⁵ sont indépendamment choisis parmi -OH et -OCH₃.

4. Composé selon l'une quelconque ou plusieurs des revendications précédentes, dans lequel R³, R⁴, R⁶, R⁷, R⁸ et R⁹ sont H.

5. Composé selon l'une quelconque ou plusieurs des revendications précédentes, dans lequel Z est -N(R¹⁰)-(CH₂)ₚ-NR¹¹R¹².

6. Composé selon la revendication 5, dans lequel p est choisi parmi 3 et 4.

7. Composé selon l'une quelconque ou plusieurs des revendications précédentes ; dans lequel R¹¹ et R¹² forment ensemble un hétérocycle à 5 ou 6 chaînons éventuellement substitué par 1 ou 2 alkyle en C₁₋₄ ou benzyle.

8. Composé selon la revendication 7 ; dans lequel l'hétérocycle à 5 ou 6 chaînons est une morpholine, une pipéridine, une pipérazine ou une pyrrolidine éventuellement substituée par 1 ou 2 alkyle en C₁₋₄ ou benzyle.

9. Composé selon l'une quelconque ou plusieurs des revendications 1 à 6, dans lequel R¹¹ et R¹² sont indépendamment choisis parmi H et un alkyle en C₁₋₆ (par exemple, un méthyle ou un éthyle), un cycloalkyle en C₃₋₁₀ (par exemple, un adamantyle) et un benzyle ; dans lequel chaque R¹¹ et R¹², lorsqu'ils ne sont pas H, sont indépendamment éventuellement substitués par 1 ou 2 - R^{β}.

10. Composé selon l'une quelconque ou plusieurs des revendications précédentes, dans lequel R¹⁰ est H ou -CH₃.

11. Composé selon l'une ou plusieurs d'une quelconque revendication précédente, dans lequel le composé est un composé de Formule (1A) : 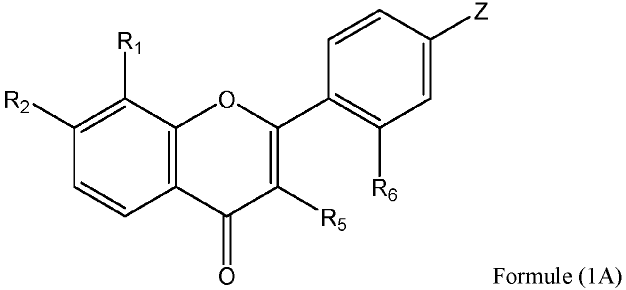 dans lequel R¹, R², R⁵, R⁶ et Z sont tels que définis dans les revendications 1 à 10.

12. Composé selon la revendication 1, ledit composé étant choisi parmi :

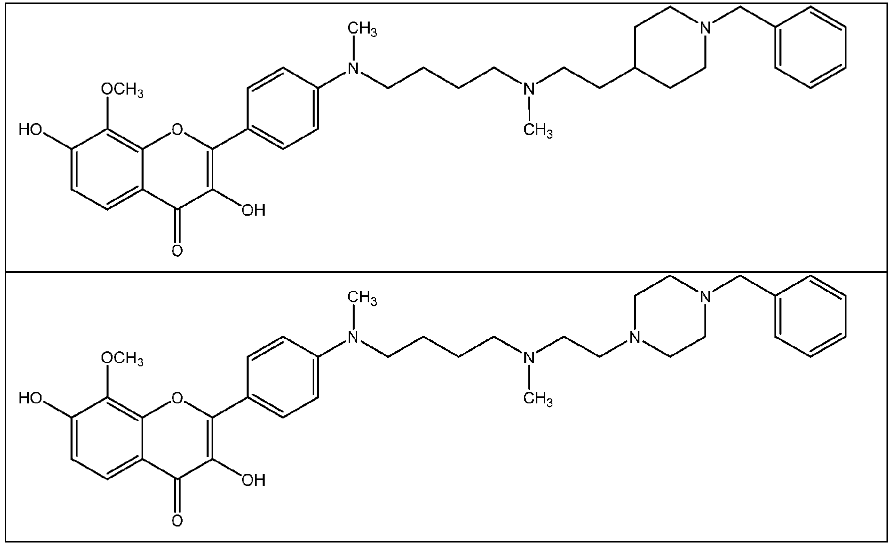

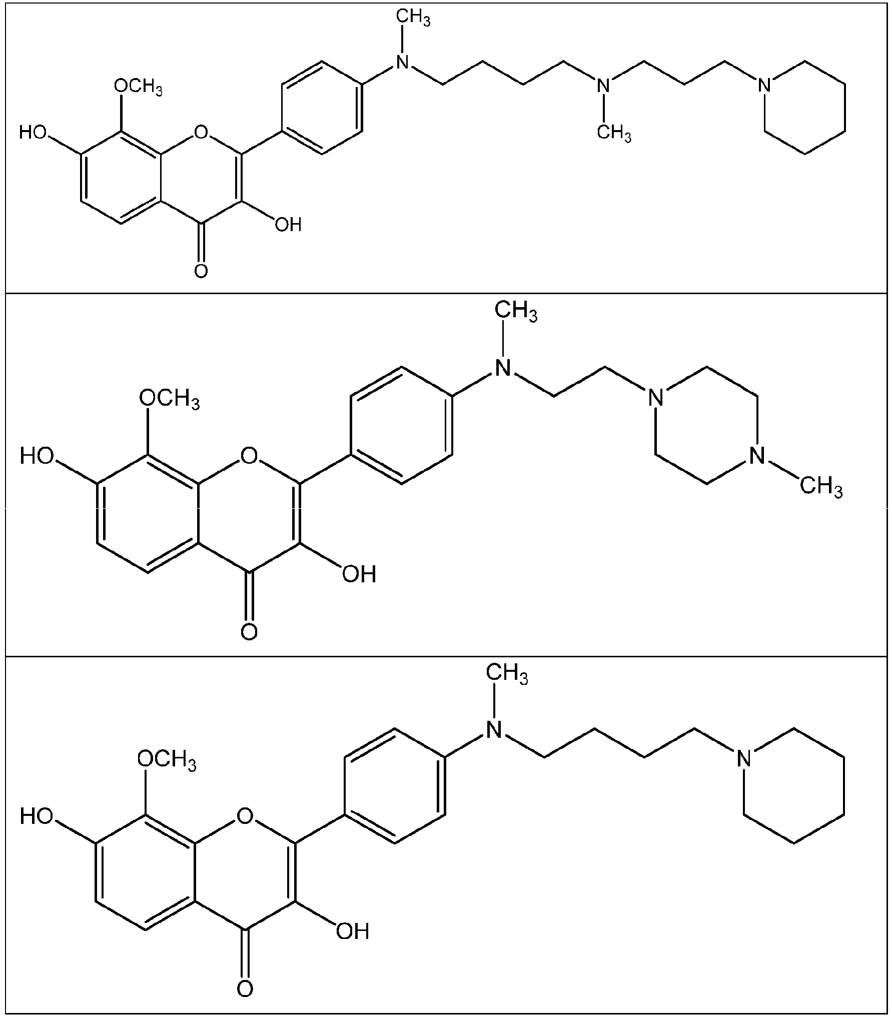

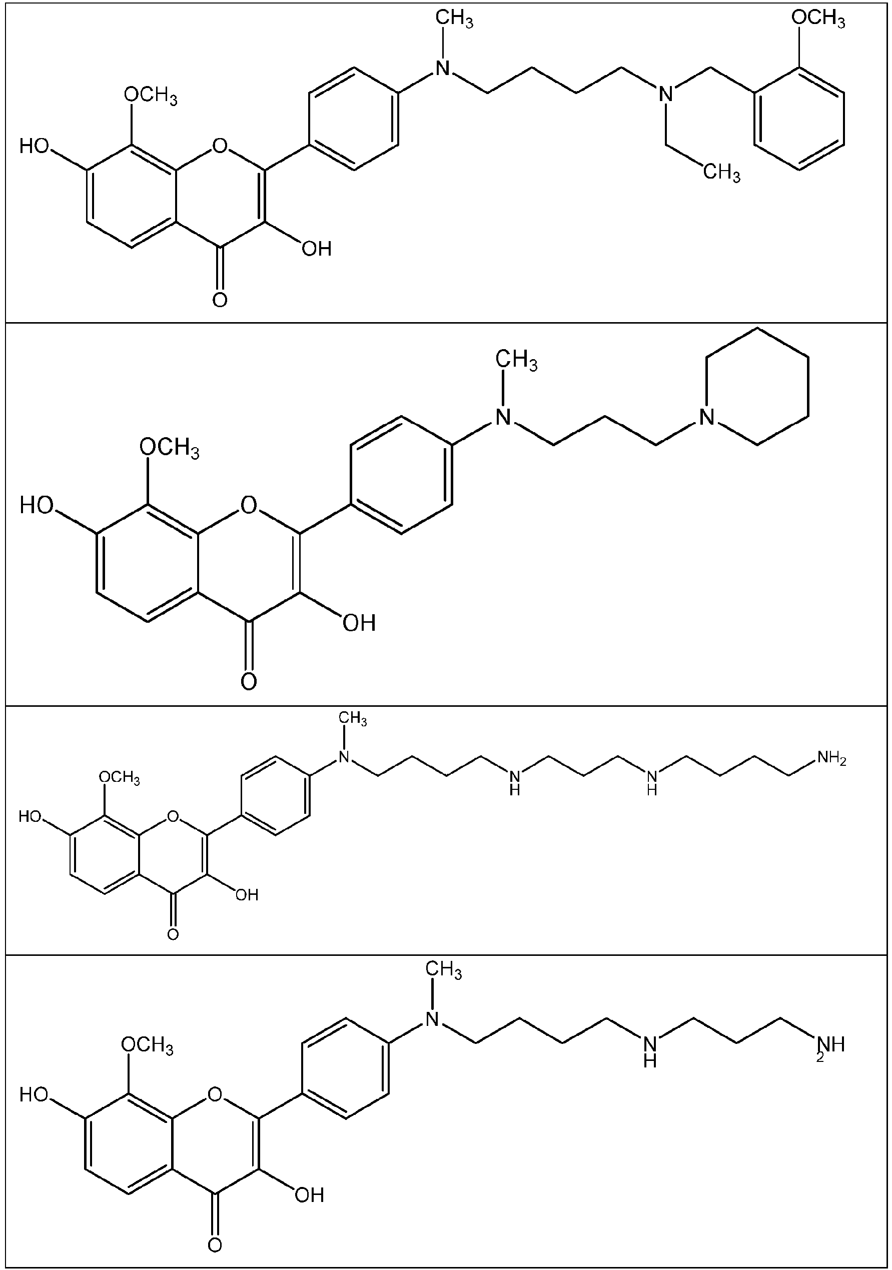

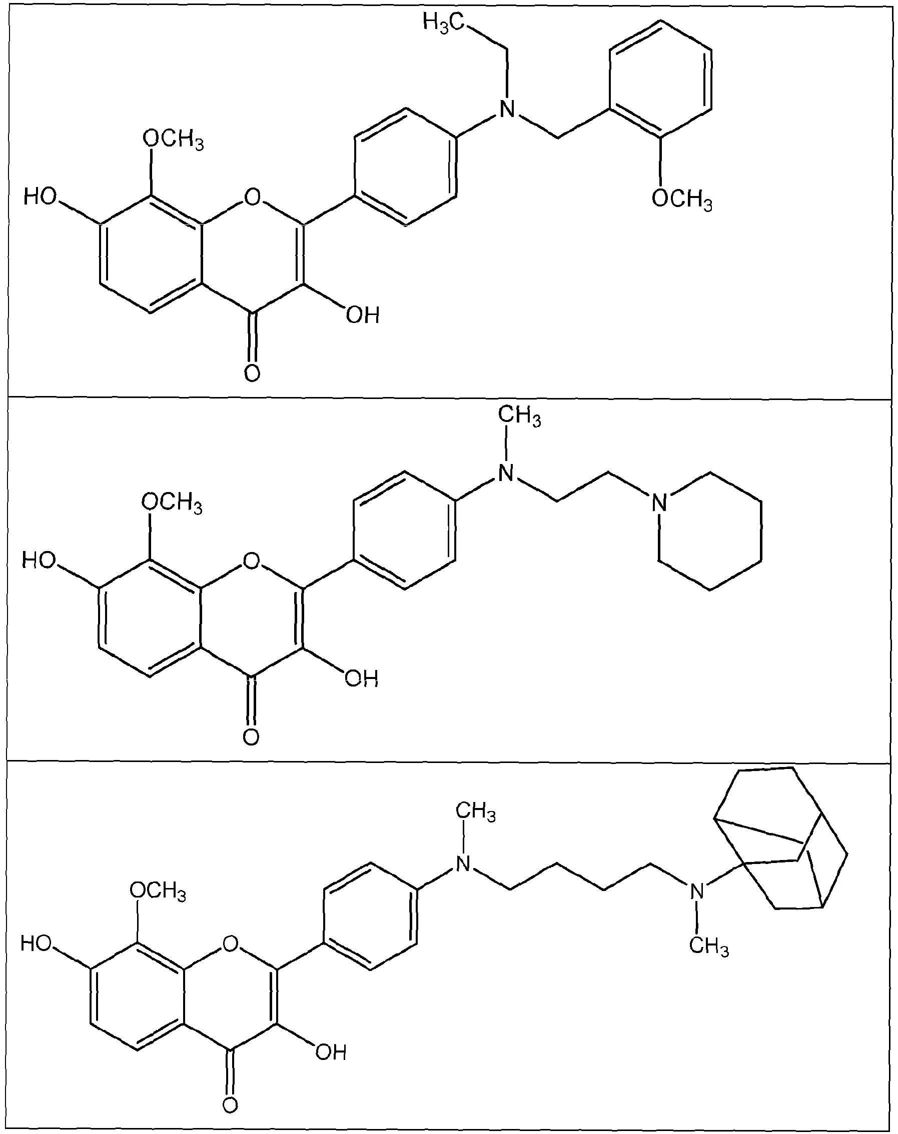

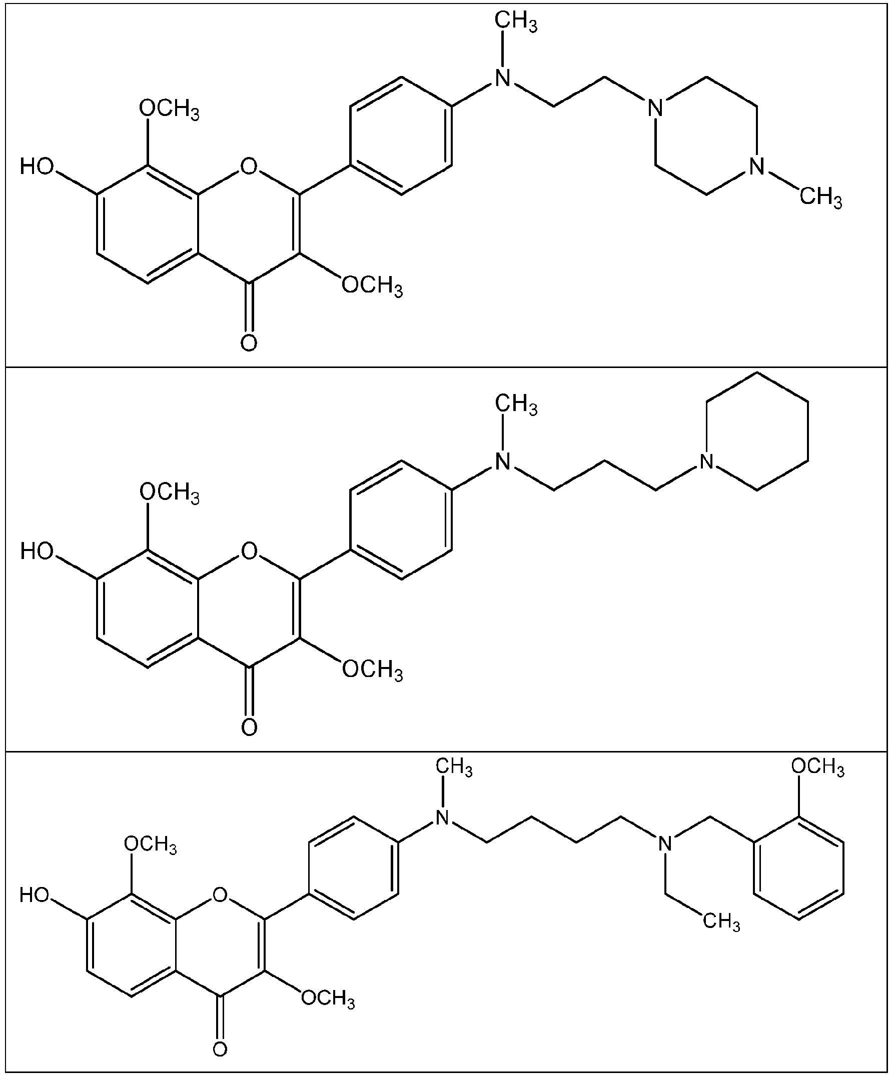

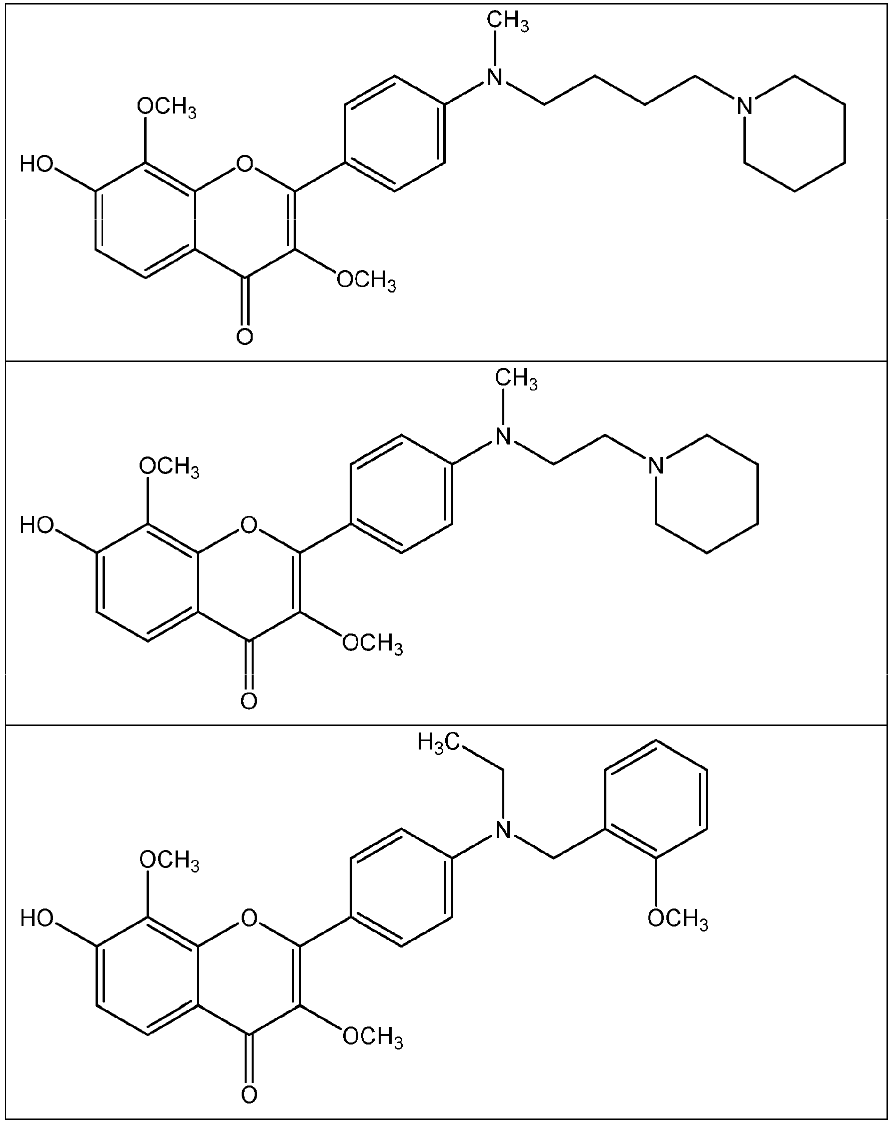

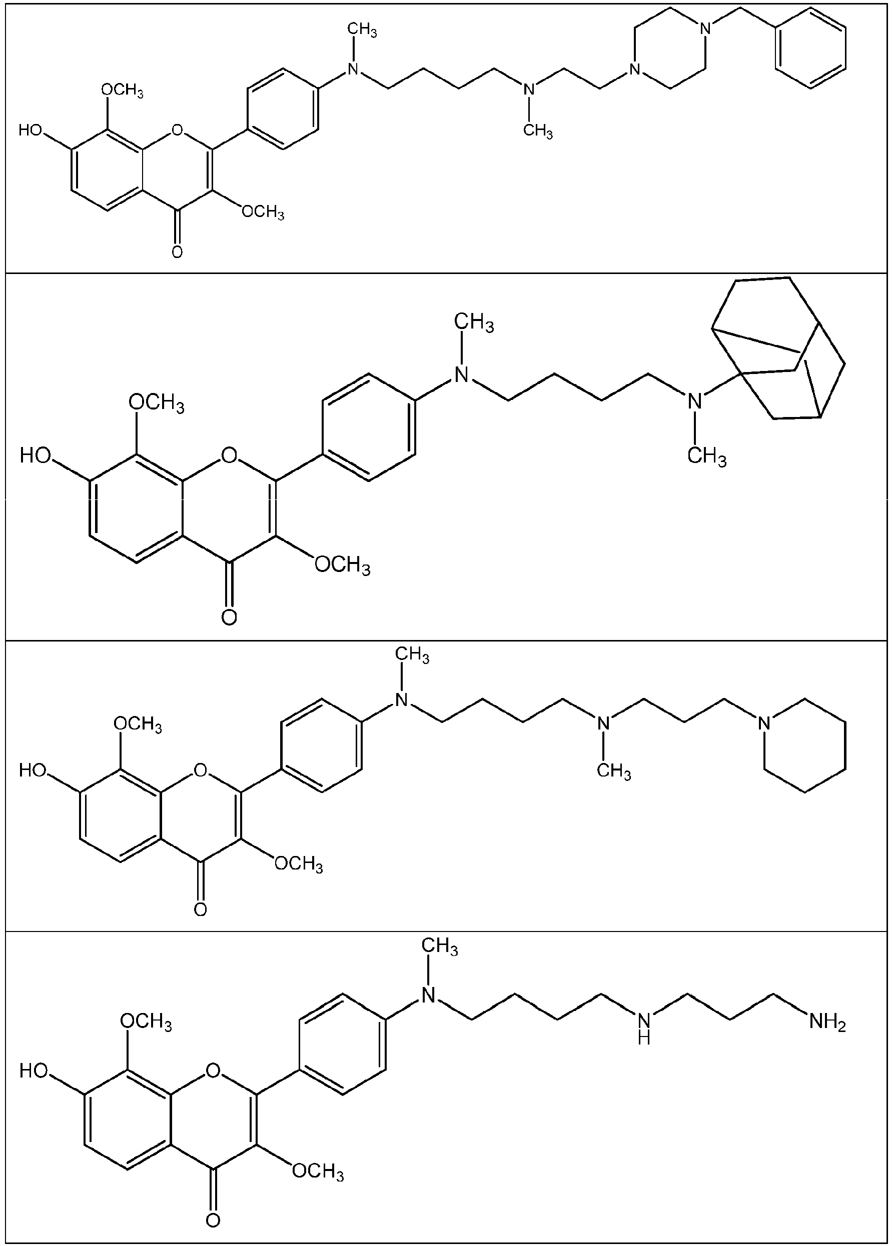

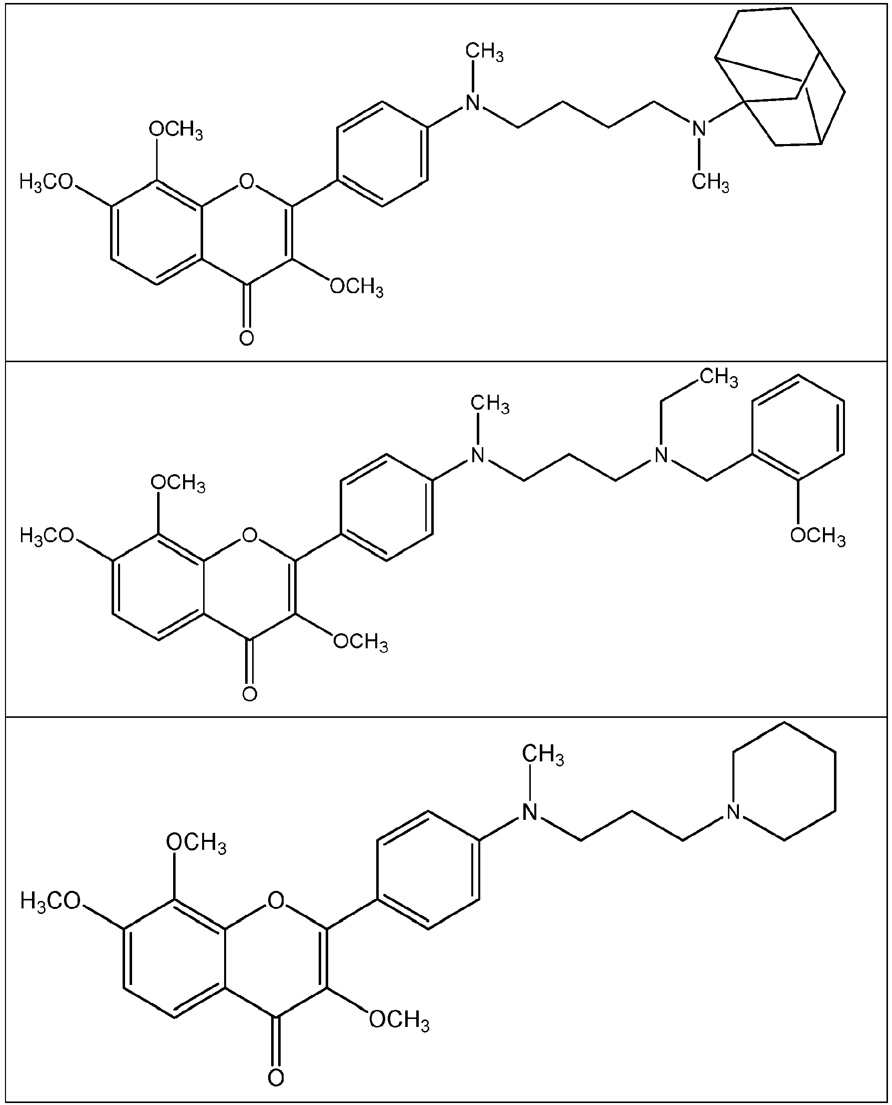

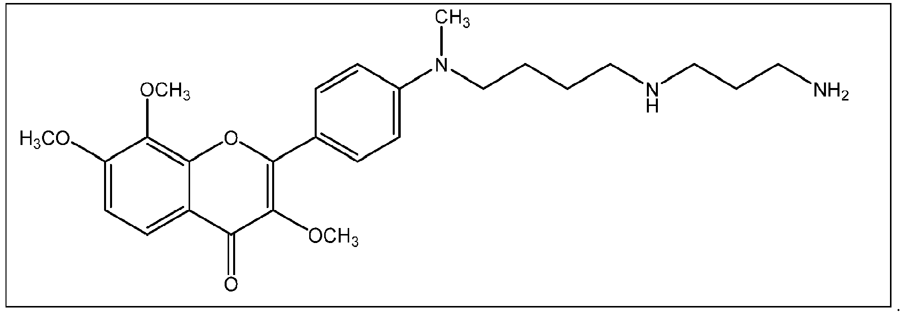

13. Composition pharmaceutique comprenant un composé, ou un sel, des sels multiples ou un solvate acceptable(s) pharmaceutiquement tel(s) que défini(s) dans l'une des revendications précédentes, et un excipient acceptable pharmaceutiquement.

14. Composé, ou sel, sels multiples ou solvate acceptable(s) pharmaceutiquement tel(s) que défini(s) dans une ou plusieurs des revendications 1 à 12, ou composition pharmaceutique telle que définie dans la revendication 13, destiné(es) à être utilisé(es) en médecine.

15. Composé, ou sel, sels multiples ou solvate acceptable(s) pharmaceutiquement tel(s) que défini(s) dans une ou plusieurs des revendications 1 à 12, ou composition pharmaceutique telle que définie dans la revendication 13, destiné(es) à être utilisé(es) pour traiter ou prévenir un cancer.
